# EUROPEAN PATENT APPLICATION

(11) **EP 1 676 574 A2**
(43) Date of publication of application: **05.07.2006**
(21) Application number: 05258093.3
(22) Date of filing: 29.12.2005
(51) Int. Cl.: A61K 31/415, A61K 31/44, A61P 37/06

(54) **Methods for promoting survival of transplanted tissues and cells**

(30) Priority: 30.12.2004 US 640462 P
(71) Applicant: Johnson & Johnson Vision Care, Inc., Jacksonville, FL 32256 (US)
(72) Inventor: Davis, Janet E., Branchburg, NJ 08876 (US); Leperi, Dominic A., Bethlehem, PA (US); Protter, Andrew A., Palo Alto, CA 94301 (US); Higgings, Linda S., Palo Alto, CA 94303 (US)
(74) Representative: Mercer, Christopher Paul

(57) **Abstract**

Methods for enhancing the survival of a cell, tissue, or organ transplanted into a recipient comprising contacting the cell, tissue, or organ graft with an agent that decreases the rate of coagulation on the transplanted cell, tissue or organ are disclosed. The agent is one that acts by reducing the amount or activity of tissue factor (TF) on the donor tissue, such as a p38 MAPK inhibitor, a JNK inhibitor, a p38 MAPK/JNK dual inhibitor and a TF antagonist. Cells, tissues and organs treated with those agents are also disclosed.

## Description

### FIELD OF THE INVENTION

The present invention relates to the field of cell, tissue and organ transplantation. In particular, the invention provides a method in which donor tissue is treated with agents that reduce the amount or activity of tissue factor (TF) on the donor tissue, thereby inhibiting or reducing the risk or severity of an adverse vascular side effect and improving graft survival.

### BACKGROUND OF THE INVENTION

All scientific and scholarly articles, patents and patent publications cited or otherwise referred to in this specification are incorporated by reference in their entireties.

Transplantation of autologous, allogenic or xenogeneic tissue into a recipient often evokes adverse vascular events that include hemorrhage, thrombosis, intravascular coagulation, disseminated intravascular coagulation, platelet aggregation, a reduced platelet count, reduced levels of the clotting factors V, VIII, and IX, an increased level of thrombin-antithrombin (TAT), an increased level of fibrin monomers, and an increased level of D-dimers. These adverse vascular events can result in rejection of the transplanted tissue.

The aforementioned adverse vascular effects have been associated with the presence of tissue factor (TF) on the donor tissue. These effects are amplified by the processes employed to isolate and prepare tissues for transplantation that frequently result in an increase in TF synthesis or exposure (Liu *et al*., 2004, *J Biol. Chem*. 279, 36142-36147). A reduction in the amount or biological activity of TF can therefore result in a therapeutic benefit for the transplant recipient.

**Tissue Factor**: The coagulation of blood involves a cascading series of reactions leading to the formation of fibrin. The coagulation cascade consists of two overlapping pathways. The intrinsic pathway comprises protein factors present in circulating blood while the extrinsic pathway requires TF that is expressed on the surface of cells in a variety of tissues in response to vascular injury (Davie *et al*., 1991, *Biochemistry* 30:10363). When exposed to blood, TF sets in motion a rapid cascade of activation steps that result in the formation of an insoluble fibrin clot.

TF (also known as thromboplastin, CD 142 and coagulation factor III) has been investigated as a target for anticoagulant therapy. TF functions as a receptor for factor VII and VIIa and thereby initiates the extrinsic pathway of the coagulation cascade in response to vascular injury. TF is an integral membrane glycoprotein normally present on the cell surface of non-vascular cells. TF is not present on healthy endothelial cells lining normal blood vessels; however, it is always present in the adventitia of blood vessels.

TF serves as both a cofactor for factor VIIa, forming a proteolytically active TF:VIIa complex on cell surfaces, and as a VIIa receptor, inducing downstream intracellular changes (Bazan, JF, 1990, *Proc. Natl. Acad. Sci USA* 87:6934-8; Reviewed by Konigsberg, *et al*., 2001 *Thromb. Haemost.* 86:757-71). In addition to its role in the maintenance of hemostasis by initiation of blood clotting, TF has been implicated in pathogenic conditions. Specifically, the synthesis and cell surface expression of TF has been implicated in vascular disease (Wilcox *et al.,* 1989, *Proc. Natl. Acad. Sci.* 86:2839) and gram-negative septic shock (Warr *et al.,* 1990, *Blood* 75:1481). Furthermore, in a number of pathological states involving an acute inflammatory response and progression to a thrombotic state, such as sepsis, increased TF expression on the vascular endothelium results from the release of inflammatory mediators such as tumor necrosis factor (TNF) and/or interleukin-1 (IL-1).

An additional relationship between TF and inflammation has been noted in the context of glycemic control agents and insulin. TF antagonists and blood glucose lowering agents have been used for the treatment of thrombotic and coagulopathic diseases, respiratory and inflammatory diseases (Intl. Application Pub. No. WO04/041302).

**p38 Mitogen-Activated Protein Kinase:** Mitogen-activated protein kinases (MAPKs) are a family of serine/threonine protein kinases widely conserved among eukaryotes and involved in multiple cellular programs with a role in cellular proliferation, differentiation, cell movement, and cell death. The MAPK family includes the extracellular signal-regulated kinases (ERK), the c-Jun N-terminal kinases (JNK), and the p38 kinases (p38), which form three parallel signaling cascades activated by distinct and sometimes overlapping sets of stimuli. In general, ERK is activated by mitogenic factors while JNK and p38 are activated by stress-inducing agents or pro-inflammatory cytokines (reviewed in Pearson, G *et al.,* 2001, *Endocrine Reviews* 22: 153-183 and Kyriakis, JM & Avruch, J., 2001, *Physiol. Reviews* 81: 807-886).

The p38 kinases were originally identified as activated by bacterial lipopolysaccharide (LPS) stimulation of monocytes (Han, J *et al*., 1993, *J. Biol. Chem* 268:25009) and later shown to regulate LPS-induced IL-1β and TNF-α release from these cells (Lee, J.C. *et al*., 1994, *Nature* 372: 739). Members of the p38 family include p38α, CSBP1, p38-β, p38-β2/p38-2, p38-γ/ERK6/SAPK3, p38-δ/SAPK4, and Mxi2 (New, L. & J. Han, 1998, *Trends Cardiovasc Med 8:* 220-8). In human tissues, p38-α mRNA is widely expressed, p38-β and p38-δ expression is slightly more restricted (highest in brain and glandular tissues, respectively), and p38-γ is largely restricted to skeletal muscle (Wang, X.S. *et al*., 1997, *J. Biol. Chem.* 272:23668-74). In addition to regulating IL-1 and TNF-α synthesis by monocytes, p38 activity is essential for the production of IL-10 and prostaglandin H synthase-2 (PGHS-2) by monocytes and for the production of PGHS-2, metalloproteinases, and IL-6 from fibroblasts and endothelial cells. Functional roles in lymphocytes, both T and B cells, have also been reported.

Specific small molecule inhibitors of p38 kinase activity have been useful in determining additional roles of the kinase. The pyridinyl imidazole SB203580 specifically inhibits p38, but not other MAPKs, by occupying the ATP-binding site (Cuenda, A. et al., 1995, *FEBS Lett*. 364:229; Wilson, K.P. *et al*, 1996, *J. Biol Chem*. 271:27696). SB203580 and other pyridinyl imidazoles have been shown to inhibit IL-1 and TNF-α biosynthesis in human monocytes (Lee, J.C. *et al*., 1994, *Nature* 372:739; Lee, J.C. *et al*, 1988, *Int. J Immunopharmacol.* 10:835; Gallagher, T.F., *et al*., 1995, *Bioor. Med. Chem. Lett*. 5:1171) by a mechanism involving inhibition of mRNA translation (Young, P. *et al*., 1993, *Agents Actions* 39:C67; Prichett, W. *et al*., 1995, *J. Inflamm.* 45:97; Lee, J.C. and P.R. Young., 1996, *J Leukocyte Biol.* 59:152), and have also been shown to have therapeutic activity in inflammatory disease models (Badger, A.M. *et al*., 1996, *J Pharm & Exp. Ther.* 279:1453). RWJ 67657 (JNJ 3026582) is a novel imidazole inhibitor of p38 previously reported to have potent enzymatic inhibitory activity on p38-α and β but not -γ or -δ (Wadsworth, S.A. *et al.,* 1999, *J. Pharm & Exp. Ther.* 291:680-687).

**p38 Mitogen-Activated Protein Kinase and Coagulation:** A role for p38 kinases in coagulation involving multiple cell types has been demonstrated. Human umbilical vein endothelial cells (HUVEC) stimulated with thrombin show time-dependent phosphorylation of p38 MAPK and its substrate ATF-2. Thrombin-induced secretion of interleukin 8 (IL-8) and monocyte chemoattractant protein-1 (MCP-1) by HUVEC can be inhibited by the selective p38 inhibitor SB203580 (Marin, V *et al.,* 2001, *Blood* 98: 667-673). Late-stage platelet activation results in coagulation-promoting activity, including membrane-blebbing and shedding. Phosphorylation of p38 MAPK is increased in platelets during fibrinogen-adherent coagulation compared to platelets in suspension. The p38 inhibitor SB203580 has a direct effect on inhibiting both bleb formation and calpain activity (Siljander, P. *et al.,* 2001, *Arterioscler. Thromb. Vasc. Biol.* 21:618-627). Thrombin-induced migration of vascular smooth muscle cells is associated with p38 activation and can also be inhibited by SB203580 (Wang *et al.,* 2004, *J. Mol Cell Cardiol.* 36:49-56).

TF initiates the extrinsic coagulation cascade leading to thrombin formation. Thrombin in turn induces upregulated expression of TF mRNA in vascular smooth muscle cells (VSMCs), thereby contributing to prolonged procoagulant activity and enhanced thrombogenicity at sites of vascular injury. Vascular injury also induces the generation of multiple reactive oxygen species (ROS) with NAPDH oxidase being a source of ROS in vascular smooth muscle cells. Inhibitors of p38 MAPK prevent thrombin-stimulated mRNA expression of TF (Herkert, O *et al*., 2002, *Circulation* 105:2030-2036). Downstream signaling *via* reactive oxygen species, as initiated by a redox-sensitive cascade, leads to phosphorylation and activation of p38 MAPK and subsequent upregulation of target genes such as hypoxia-inducible factor-I (HIF1), vascular endothelial growth factor (VEGF), and plasminogen activator inhibitor-1 (PAI-1). Inhibition of p38 MAPK decreases expression of these target genes (Gorlach, A. *et al*., 2001, Circ. Res. 89:47-54).

A correlation between environmental stress, p38 activity, and induction of TF expression has been established for various cell types. Primary monocytes treated with various protease and proteosome inhibitors show increased TF activity in a dose- and time-dependent manner at the level of TF gene transcription, mediated by phosphorylation of JNK and p38 MAPK (Ohsawa, M. *et al*., 2004, *Thromobosis Res.* 112:313-320). Quiescent human dermal microvascular endothelial cells do not constitutively express TF but can be induced to express TF protein after treatment with TNFα. Increased TF expression correlates with increases in TF hnRNA without changes in mRNA stability, suggesting increased gene transcription. Inhibitors of p38 MAPK almost completely block TNFα induced TF expression (O'Reilly, *F.M. et al.,* J 2003, *Invest. Dermatol.* 120:489-494; Lim, M. *et al,* 2004, *J Mol & Cell. Cardiol.* 37: 111-114; Liu, Y. *et al,* 2004, *J Biol. Chem.* 279: 36142-36147).

Procoagulant responses have been measured in healthy human subjects exposed to an *intra venous* dose of lipopolysaccharide (LPS), with or without an orally administered p38 MAPK inhibitor. Subjects treated with a p38 inhibitor show strong inhibition of coagulation activation, as measured by plasma concentrations of the prothrombin fragment F1+2, along with inhibition of the fibrinolytic system (plasma tissue-type plasminogen activator, plasmin-a2-antiplsmin complexes, and plaminogen activator inhibitor type 1) and inhibition of endothelial cell activation (plasma soluble E-selectin and von Willebrand factor) (Branger, J. *et al*, 2003, *Blood* 101:4446-4448).

**c-Jun N-Terminal Kinase and its Role in Coagulation:** The c-Jun N-Terminal kinase (JNK) is a stress-activated protein kinase that can be induced by inflammatory cytokines, bacterial endotoxin, osmotic shock, UV radiation and hypoxia. Targets of the JNK pathway include the transcription factors c-jun and ATF2 (Whitmarsh A. J., and Davis R. J., 1996, *J. Mol. Med*. 74:589-607). These transcription factors are members of the basic leucine zipper (bZIP) group that bind as homo- and hetero-dimeric complexes to AP-1 and AP-1-like sites in the promoters of a number of genes (Karin M. *et al*., 1997, *Curr. Opin. Cell Biol*. 9:240-246), many of which encode pro-inflammatory molecules, including cytokines, growth factors, cell surface receptors, cell adhesion molecules and degradative enzymes. Activation of the JNK pathway has been documented in a number of diseases and pathological conditions, particularly related to inflammation and autoimmune responses.

Similar to p38 MAPK, a correlation between JNK activation and coagulation has also been reported. TF activation and binding to factor VIIa induces a Ca²⁺ signal and subsequent phosphorylation of JNK in addition to other MAP kinases. It has been reported that both p38 and JNK are important for signaling increased expression of TF in endothelial cells; inhibition of both p38 and JNK essentially ablates TF expression in that system (Liu, Y., *et al.,* 2004, *J.Biol. Chem*. 279: 36142-36147). A cell-permeable peptidic inhibitor of JNK suppresses coagulation necrosis in a rat model of ischemic brain injury (Asami, A.T. *et al*., 2004, *Neuroscience Letters* 359: 57-60). Interstitial activation of the coagulation cascade, mediated in part by thrombin, activates the JNK-AP-1 signaling pathway and can be modulated by a small molecule inhibitor of JNK (Pontrelli, P. *et al*., 2004, *Kidney International* 65:2249-2261).

**The Role of Tissue Factor in Transplantation:** A number of reports implicate the role of active TF in the pathogenesis of transplant failure. US Patent No. 6,387,366 notes that bone marrow stem cell (BMSC) transplantation causes blood clotting and /or hemorrhage due to the expression of TF on the infused cells and suggests several methods to reduce the biological activity of TF or FVII in infusions employing BMSC transplantation, gene therapies employing BMSC, and other types of cell transplantation.

Veno-occlusive disease (VOD) is the most common regimen-related toxicity accompanying stem cell transplantation (SCT). Severe VOD complicated by multi-system organ failure (MOF) remains almost uniformly fatal. Defibrotide, a single-stranded polydeoxy-ribonucleotide that has specific aptameric binding sites on vascular endothelium, has shown promise in the treatment of VOD. Among other actions, Defibrotide modulates TF expression by microvascular endothelial cells. (Falanga A, et al. Blood 1999; 94:146a). Cyclosporine, a common agent used to prevent graft rejection in patients receiving all types of organ transplants, has been shown to inhibit TF expression in monocytes and macrophages (Hoelshermann, H. et al. Blood 1996; 10: 3837-3845).

Cardiac allograft vasculopathy (CAV) is the cause of death for 20% of heart transplant patients who have survived more than 3 to 5 years and is associated with early deposition of fibrin. A study undertaken to determine the role of TF in CAV demonstrated increased TF staining on the luminal surface and beyond the medial layers of small intramyocardial arteries in biopsy samples taken from transplanted hearts. The TF score was associated with a greatly increased incidence of CAV. (Yen, M.H. et al., Circulation 2002, 106: 1379-1383).

TF is also present on the surface of pancreatic cells used in the preparation of islets for portal vein transplantation. The presence of TF has been implicated in mediating the thrombotic and necrotic events that ultimately lead to graft rejection. Blockade of cell surface TF using anti-TF antibodies can reduce the coagulation time of these preparations when exposed to serum.

**Diabetes and Islet Transplantation:** Presently there are approximately 17 million diabetic patients in the United States alone. Most cases of diabetes fall into two clinical types: type 1 (insulin -dependent diabetes mellitus or "IDDM") and type 2 (non-insulin dependent diabetes mellitus or "NIDDM"). Approximately 10 percent of the diabetic patients are type 1 diabetics with the remaining being type 2 diabetics.

IDDM is characterized by a partial or complete inability to produce insulin, usually due to destruction of the insulin-producing cells of the pancreatic islets of Langerhans. Absent regular insulin injections, IDDM patients can experience a wide range of debilitating symptoms that can progress to coma and ultimately death. Additionally, a fraction of type 2 NIDDM diabetics are insulin dependent and require insulin injections to improve their insulin resistance. Thus, both type 1 and insulin-dependent type 2 diabetics can benefit from improvements in insulin administration.

An alternative method of treating diabetes that does not require repeated administration of insulin involves transplantation of pancreatic cells or tissue. However, a major problem with pancreatic cell or tissue transplantation has been the shortage of donor tissue. Islet cell transplant has come to be used to treat severe cases of pancreatitis and diabetes. Transplantation of islets rather than the whole pancreas is attractive because of its technical ease and the ability to transplant early in the course of the disease. Islets are more than single cells and less than a complete organ in that they are comprised of several cell types in somewhat organized structures. However, the transplant of islet tissue is often unsuccessful due to the thrombotic events taking place upon introduction of the graft material into to the patient *via* the portal vein.

Some patients undergoing this therapeutic option require two or three sequential transplants of pancreatic cells to achieve insulin independence. It has been estimated that as much as 40-60% of the transplanted cell mass is lost within the initial 48 hour post-transplant period due to events associated with inflammation and coagulation. Recent studies in a mouse model of portal vein islet cell transplantation have provided a clear picture of the scope of thrombotic events and resulting necrosis surrounding islet emboli (Hara, M. *et al,* 2004, *Transplantation* 78:615-618). Ductal epithelial cells are a major contaminant in human clinical islet isolates and, in particular, have a higher endogenous expression of TF than islets or other cells of the pancreas, which contributes to early cell damage and loss post-transplantation (Beuneu, C. *et* al., 2004, Diabetes 53:1407-1411). It has been noted that TF is expressed on cells in these preparations and that anti-TF antibodies can reduce the coagulation time of these preparations when introduced into plasma (Moberg et al. Lancet 2002, 360: 2039-2045). Alternative insulin-producing cell sources, as may be generated from stem cell or progenitor or genetically manipulated sources, would also be expected to suffer post-transplant loss of viability from inflammatory and coagulation events.

A medical need exists for peri-transplant treatments that diminish or eliminate the risk of thrombosis. There is also a need for methods to treat or ameliorate the effects seen in grafts that survive longer term, but that are at risk due to increased TF activation. The therapy must be both efficacious, preventing thrombosis at the graft-blood interface, and safe, having minimal risk of hemorrhagic complications.

### SUMMARY OF THE INVENTION

According to one aspect of the invention, a method is provided for enhancing survival of a cell, tissue, or organ transplanted into a recipient. The method comprises contacting the cell, tissue, or organ with at least one agent that decreases the rate of coagulation on the transplanted cell, tissue or organ. In a preferred embodiment, the agent reduces the amount or activity of tissue factor on or within the cell, tissue or organ.

In certain embodiments, the agent is a p38 MAPK inhibitor, a JNK inhibitor, a p38 MAPK/JNK dual inhibitor, or any combination thereof. In specific embodiments, the agent is a benzyl piperidine, a piperidine, a piperazine, a pyrrolobenzimidazole, an indol or a pyridinyl-imidazol, such as 4-[4-(4-fluorophenyl)-1-(3-phenylpropyl)-5-(4-pyridinyl)-1H-imidazole-2-yl]-3-butyn-1-ol or small molecule indol (referred to herein as compound 2) as described in co-pending U.S. Application No. [not available] (based on U.S. Provisional Application No. 60/507768, filed September 30, 2003).

In additional embodiments, the method further comprises contacting the cell, tissue or organ with a tissue factor antagonist, including but not limited to an anti-tissue factor antibody.

In various embodiments, the cell, tissue or organ is contacted with the agent prior to or during transplantation into the recipient. In one embodiment, the cell, tissue or organ is contacted with the agent *in vitro,* prior to transplantation into the recipient.

The cell, tissue or organ may be any cell, tissue or organ amenable to transplantation. In certain embodiments, a pancreatic-derived cell population or a multipotent or pluripotent progenitor cell population is utilized. These cell populations may be injected or implanted directly into a recipient patient, or they may be implanted as part of a biocompatible scaffold, matrix, artificial tissue or organ, or the like.

In accordance with another aspect of the invention, the method may additionally comprise administering the agent to the recipient before, during, and/or after the transplantation. In embodiments of this method, a TF antagonist and/or an immunosuppressive agent may also be administered to the recipient patient.

In another aspect, the present invention features a cell, tissue, or organ exhibiting enhanced survival upon transplatation into a recipient, wherein the cell tissue or organ has been contacted with, and thereby comprises within or upon it, at least one agent that decreases the rate of coagulation on the cell, tissue or organ. Preferably, the agent reduces the amount or activity of tissue factor on or within the cell, tissue or organ.

In certain embodiments, the agent is a p38 MAPK inhibitor, a JNK inhibitor, a p38 MAPK/JNK dual inhibitor, or any combination thereof. In specific embodiments, the agent is a benzyl piperidine, a piperidine, a piperazine, a pyrrolobenzimidazole, an indol or a pyridinyl-imidazol, such as 4-[4-(4-fluorophenyl)-1-(3-phenylpropyl)-5-(4-pyridinyl)-1H-imidazole-2-yl]-3-butyn-1-ol or the indol compound as referenced above (compound 2).

In additional embodiments, the cells, tissues or organs have been contacted with a tissue factor antagonist, including but not limited to an anti-tissue factor antibody.

The cell, tissue or organ may be any cell, tissue or organ amenable to transplantation. In certain embodiments, a pancreatic-derived cell population or a multipotent or pluripotent progenitor cell population is utilized. These cell populations may be injected or implanted directly into a recipient patient, or they may be implanted as part of a biocompatible scaffold, matrix, artificial tissue or organ, or the like.

Other features and advantages of the present invention will be understood by reference to the drawings, detailed description and examples that follow.

### BRIEF DESCRIPTION OF THE DRAWINGS

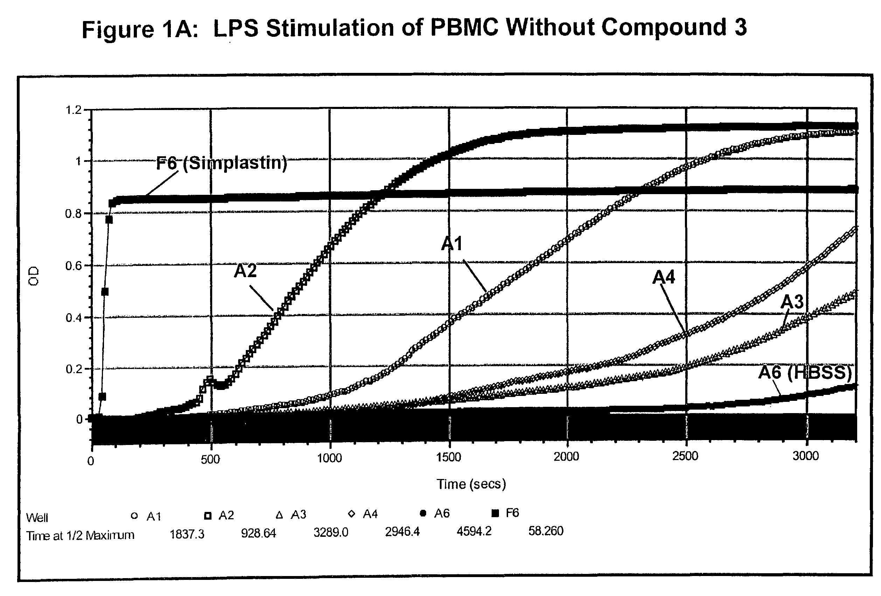

Figure 1A shows a coagulation curve of human PBMCs as a measure of turbidity (OD₄₀₅) versus time (sec). A1 is (+) p38 inhibitor compound 1, (+) LPS; A2 is (-) p38 inhibitor compound 1, (+) LPS; A3 is (+) p38 inhibitor compound 1, (-) LPS; A4 is (-) p38 inhibitor compound 1, (-) LPS; A6 is HBSS medium without cells (negative control); and F6 is Simplastin (liquid thromboplastin, positive control). In all cases, cells were pre-incubated with p38 inhibitor and/or LPS stimulus for 18 to 24 hours prior to assaying for coagulation. The times to half the maximum OD were calculated from the curve of OD vs. time as: 928.6 seconds for (-) p38 inhibitor and (+) LPS (A2); 1837.3 seconds for (+) p38 inhibitor and (+) LPS (A1); 2946.4 seconds for (-) p38 inhibitor and (-) LPS (A4); 3289.0 seconds for (+) p38 inhibitor and (-) LPS (A3); 4594.2 seconds for HBSS without cells (A6); and 58.3 seconds for Simplastin (F6). Cell viability prior to initiating coagulation exceeded 95 % in all cases.

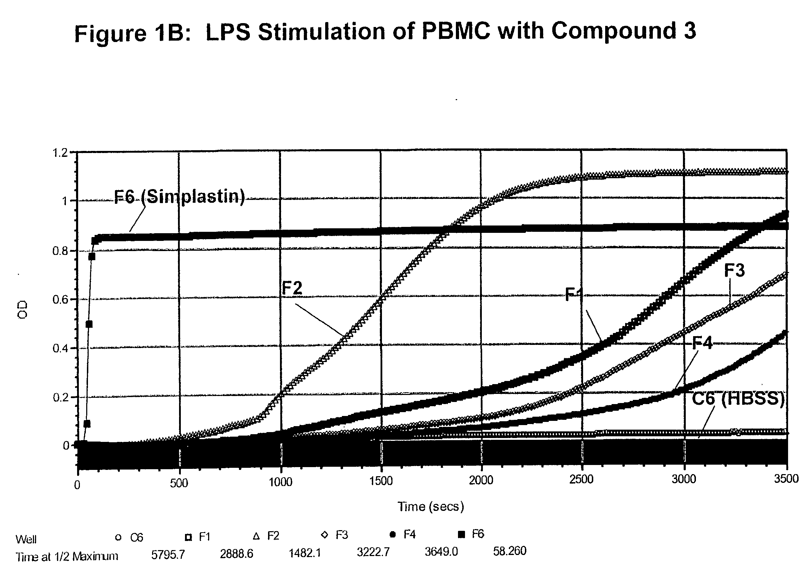

Figure 1B shows a coagulation curve of human PBMC's as a measure of turbidity (OD₄₀₅) versus time (sec). C6 is HBSS medium without cells (negative control); F1 is (+) p38 inhibitor compound 1, (+) LPS, and (+) anti-TF antibody compound 3; F2 is (-) p38 inhibitor compound 1, (+) LPS, and (+) anti-TF antibody compound 3; F3 is (+) p38 inhibitor compound 1, (-) LPS, and (+) anti-TF antibody compound 3; F4 is (-) p38 inhibitor compound 1, (-) LPS, and (+) anti-TF antibody compound 3; and F6 is Simplastin (liquid thromboplastin, positive control). In all cases, cells were pre-incubated with p38 inhibitor and/or LPS stimulus for 18 to 24 hours prior to assaying for coagulation. The anti-TF antibody was added 30 minutes prior to initiation of the clotting reaction. The time to half the maximum OD was calculated as 1482.1 seconds for (-) p38 inhibitor, (+) LPS, and (+) anti-TF antibody compound 3 (F2); 2888.6 seconds for (+) p38 inhibitor, (+) LPS, and (+) anti-TF antibody compound 3 (F1); 3222.7 seconds for (+) p38 inhibitor, (-) LPS, and (+) anti-TF antibody compound 3 (F3); 3649.0 seconds for (-) p38 inhibitor, (-) LPS, and (+) anti-TF antibody compound 3 (F4); and 5795.7 seconds for HBSS without cells (C6). Cell viability prior to initiating coagulation exceeded 95 % in all cases.

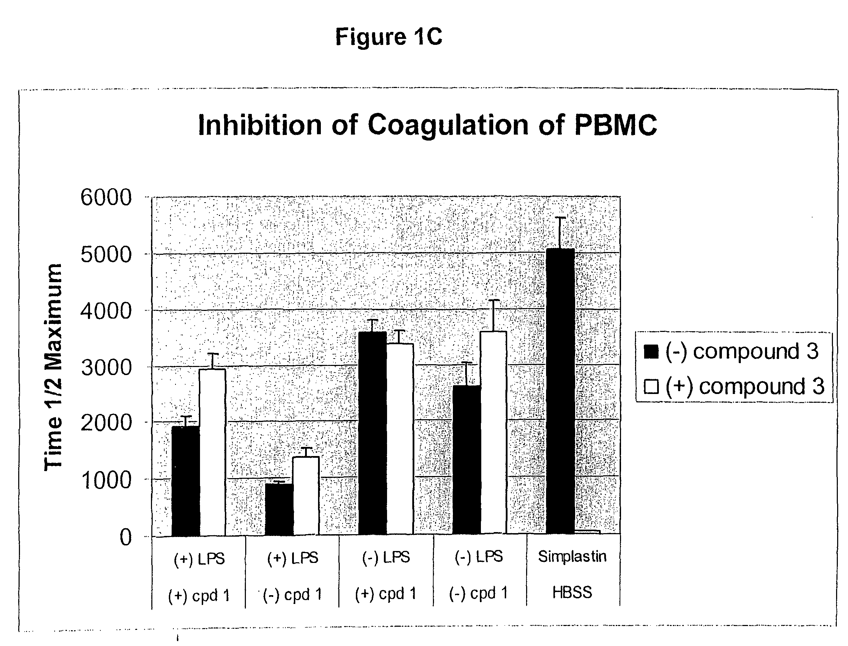

Figure 1 C is a chart using coagulation data from replicate sets in Figures 1A and 1B to express the time to half the maximum OD in cells treated with compound 1 or untreated cells.

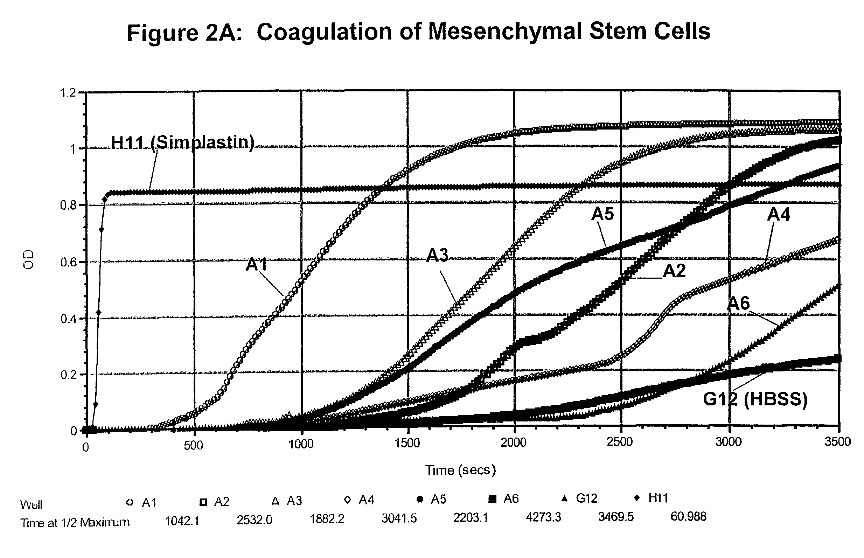

Figure 2A shows a coagulation curve of human mesenchymal stem cells (MSC) as a measure of turbidity (OD₄₀₅) versus time (sec). A1 is cells with DMSO diluent (vehicle control); A3 is cells pre-treated with p38 inhibitor compound 1; A5 is cells pre-treated with p38 inhibitor compound 2; A2 is (+) cells with anti-TF antibody compound 3; A4 is (+) p38 inhibitor compound 1 and anti-TF antibody compound 3; A6 is p38 inhibitor compound 2 and anti-TF antibody compound 3; H11 is Simplastin (liquid thromboplastin, positive control); and G12 is HBSS medium without cells (negative control). In all cases, cells were pre-treated in culture with p38 mitogen-activated protein kinase inhibitor for 10 days prior to assay for coagulation. Anti-TF antibody was added 30 minutes prior to initiation of the clotting reaction.

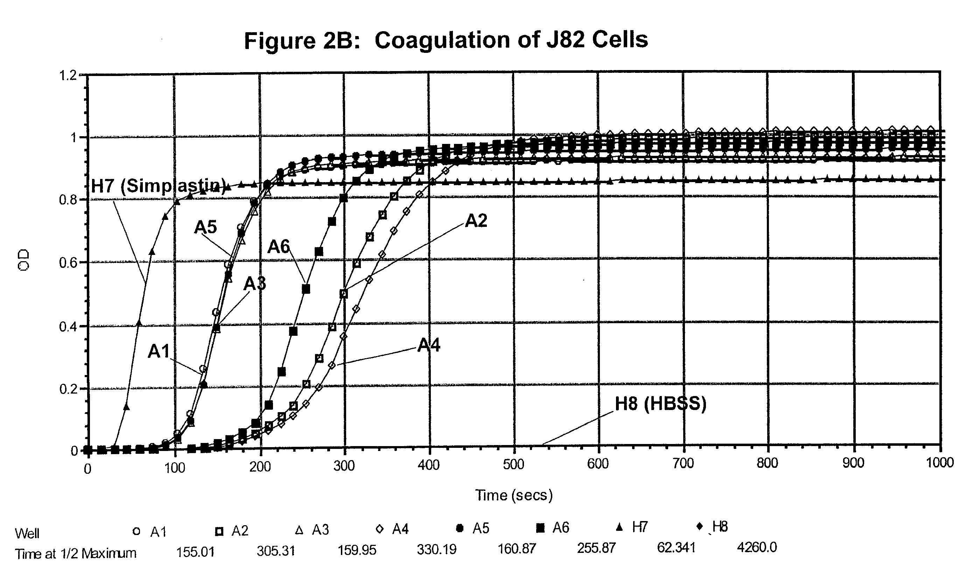

Figure 2B shows coagulation of human J82 bladder carcinoma cells used as a positive control for TF coagulation. A1 is cells with DMSO diluent (vehicle control); A2 is (+) anti-TF antibody compound 3; A3 is (+) p38 inhibitor compound 2 pre-treatment; A4 is (+) p38 inhibitor compound 2 and anti-TF antibody compound 3; A5 is (+) p38 inhibitor compound 1 pre-treatment; A6 is (+) p38 inhibitor compound 1 and anti-TF antibody compound 3; H7 is Simplastin (liquid thromboplastin positive control); and H8 is HBSS medium without cells (negative control). In all cases, cells were pre-treated with p38 mitogen-activated protein kinase inhibitors for 10 days prior to assay for coagulation. Anti-TF antibody was added 30 minutes prior to initiation of the clotting reaction.

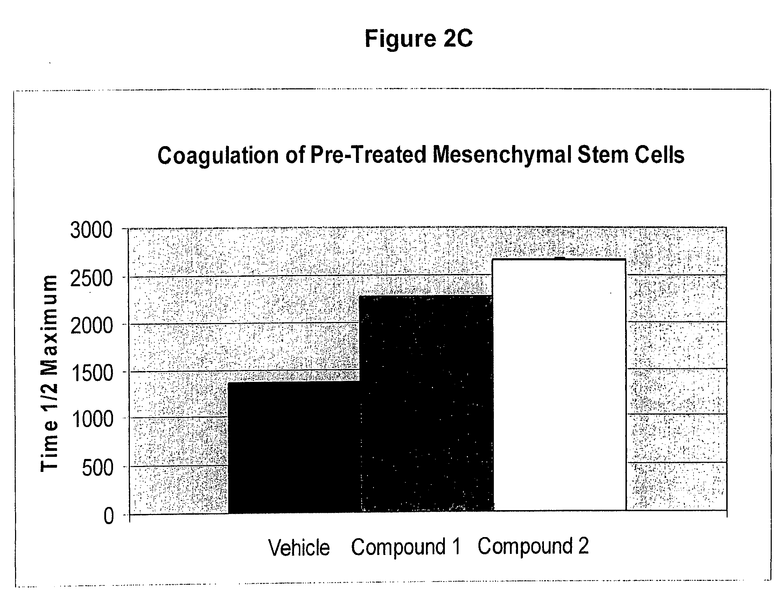

Figure 2C is a graph depicting coagulation data averaged from replicate sets expressing the time to half the maximum OD for human MSC pre-treated with p38 mitogen-activated kinase inhibitors compound 1 or compound 2.

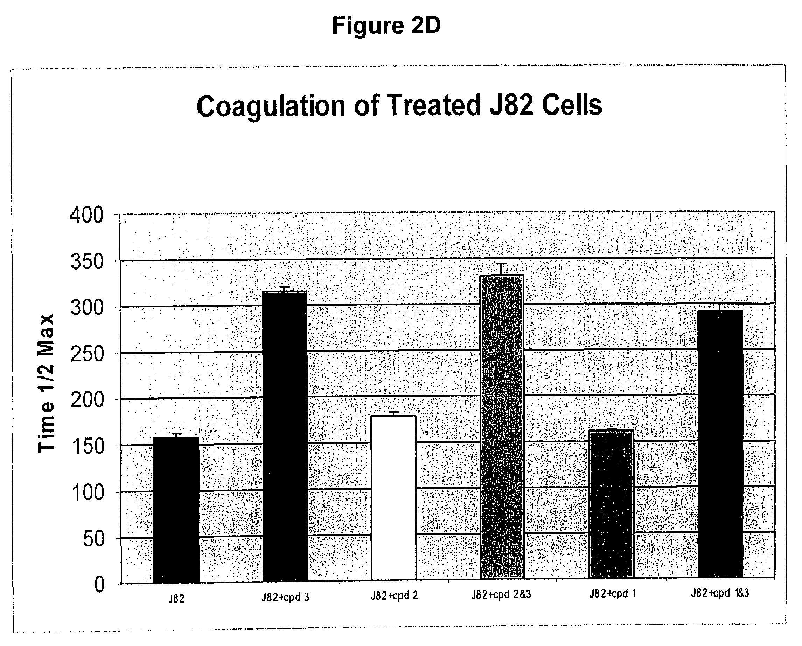

Figure 2D is a graph depicting coagulation data averaged from replicate sets expressing the time to half the maximum OD for J82 cells pre-treated with p38 mitogen-activated kinase inhibitors compound 1 or compound 2.

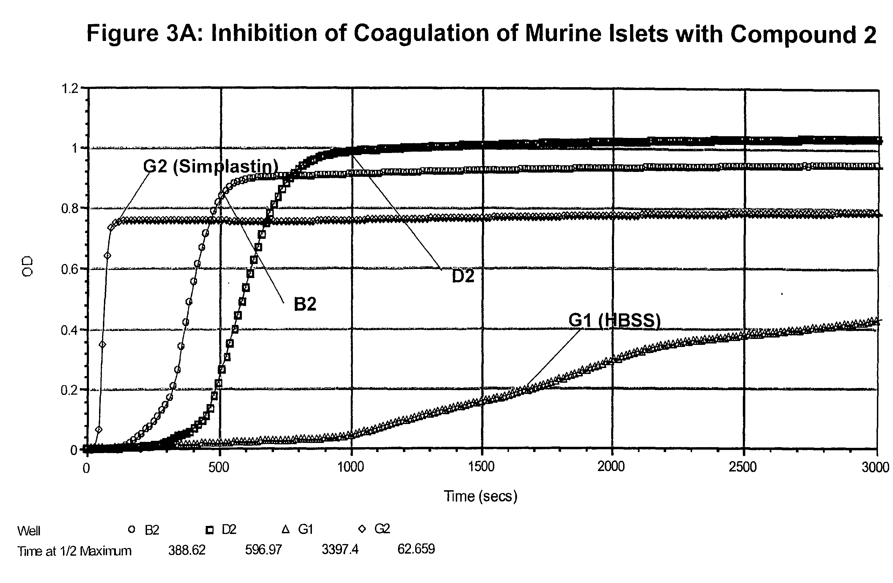

Figure 3A shows inhibition of coagulation for isolated mouse islets using p38 inhibitor compound 2 pretreatment as a measure of turbidity (OD₄₀₅) versus time (sec). B2 is islets with DMSO diluent (vehicle control); D2 is (+) p38 inhibitor compound 2 pretreatment; G1 is (+) HBSS medium without islets (negative control); and G2 is Simplastin (liquid thromboplastin positive control). In all cases, islets were pre-cultured with p38 mitogen-activated protein kinase inhibitor for three days prior to assay for coagulation.

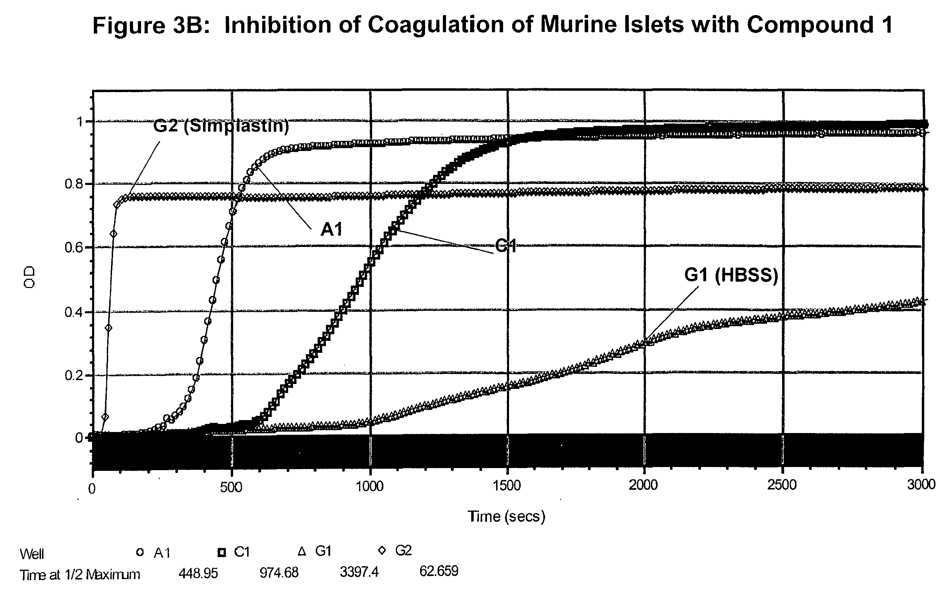

Figure 3B shows inhibition of coagulation for isolated mouse islets using p38 inhibitor compound 1 pretreatment as a measure of turbidity (OD₄₀₅) versus time (sec). A1 is islets with DMSO diluent (vehicle control); C1 is (+) p38 inhibitor compound 1 pretreatment; G1 is HBSS medium without islets (negative control); and G2 is Simplastin (liquid thromboplastin, positive control). In all cases, cells were pre-cultured with p38 mitogen-activated protein kinase inhibitor for three days prior to assay for coagulation.

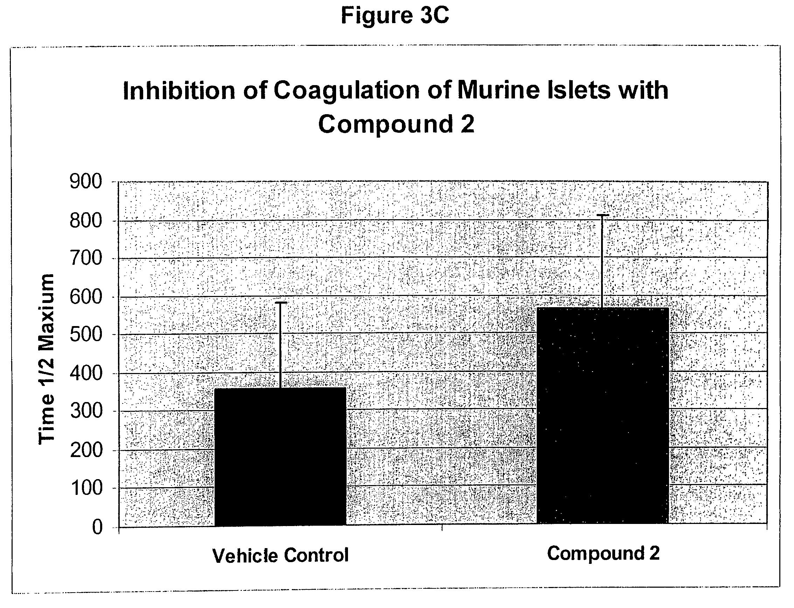

Figure 3C depicts coagulation data averaged from replicate sets expressing the time to half the maximum OD for isolated mouse islets pre-cultured with p38 mitogen-activated kinase inhibitor compound 2.

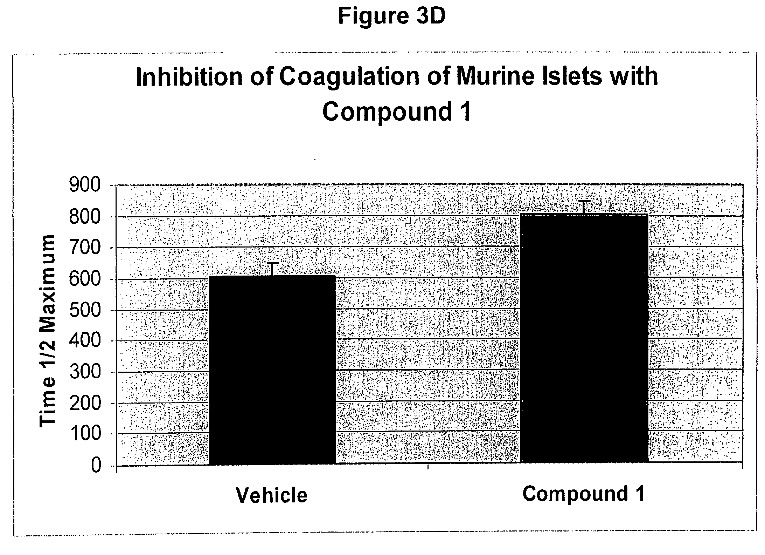

Figure 3D depicts coagulation data averaged from replicate sets expressing the the time to half the maximum OD for isolated mouse islets pre-cultured with p38 mitogen-activated kinase inhibitor compound 1.

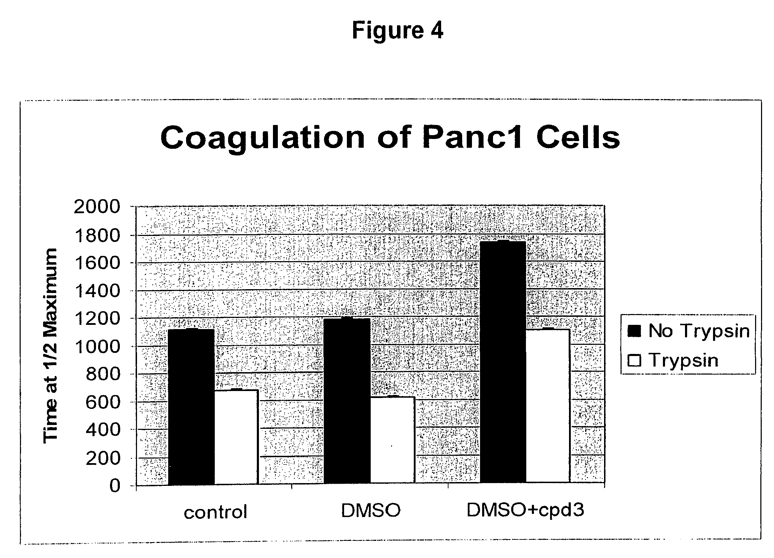

Figure 4 depicts inhibition of coagulation of Panc1 insulinoma cells shown as a measure of turbidity (OD₄₀₅) versus time (sec.). Cells were trypsinized or not trypsinized from culture and pre-incubated with anti-TF antibody 30 minutes prior to initiation of the clotting reaction.

### DETAILED DESCRIPTION OF ILLUSTRATIVE EMBODIMENTS

The methods described herein are directed to reducing the amount of tissue factor in donor tissue prior to or during transplantation into a recipient, thereby minimizing the risk or severity of an adverse vascular event. Preferably, but not necessarily, these adverse effects are prevented entirely or inhibited sufficiently to prevent adverse symptoms. The methods comprise treatment of donor tissue *in vitro* with at least one pharmaceutical agent that is capable of decreasing the rate of coagulation of the donor tissue prior to transplantation. Accordingly, certain embodiments of the invention involve culturing or pre-treating a cell population, tissue or organ with agents capable of down-regulating the expression or function of TF, thereby preventing thrombosis and improving graft survival. In various embodiments, the agent is a p38 MAPK inhibitor, a JNK inhibitor, a dual inhibitor of p3 8 MAPK and JNK, an anti-TF antibody, or any combination thereof.

As used herein, the phrase "enhancing (or promoting) the survival of a cell, tissue, or organ graft" refers to increasing, by any measurable extent, the length of time that a cell, tissue, or organ graft that is treated in accordance with the methods of the invention persists in a patient, and is not rejected by the patient, after implantation into the patient, relative to the length of time that a cell, tissue, or organ graft that is not treated in accordance with the methods of the invention persists in a patient, and is not rejected by the patient, after implantation.

As used herein, the term "implant," "transplant," and all variations thereof, refers to any means by which a cell, tissue, or organ graft is introduced into a patient.

As used herein, the term "administer," and all variations thereof, refers to and includes any means by which a substance is introduced into a patient. When administration is for the purpose of treatment, administration may be for either prophylactic or therapeutic purposes.

As used herein, the term "contact," and all variations thereof, refers to any means that directly or indirectly causes placement together of two or more substances, such that the substances come into physical contact with each other. Contacting thus includes physical acts such as placing the substances together in a container, combining the substances, or mixing the substances. Contacting also includes the administration or implantation of each of the substances into the same patient, such that the substances come into physical contact within the patient.

The terms "p38" and "p38 MAPK" are used interchangeably herein to refer to p38 mitogen-activated protein kinase. If a specific isoform is intended, it is designated as such.

The term "JNK" refers to c-Jun N-terminal kinase. If a specific isoform is intended, it is designated as such.

As used herein, the phrases "inhibitor of p38 mitogen-activated protein kinase," "p38 inhibitor" and "p38 MAPK inhibitor" are used interchangeably to refer to any substance that decreases, by any measurable extent, the amount (e.g., through transcription, translation or post-translational means) or activity of p38 MAPK. p38 MAPK inhibitors include, for example, antibodies and antibody fragments, proteins, protein fragments, polypeptides, nucleic acids, and small organic molecules, as described in detail below. Such molecules may be capable of binding to p38 MAPK. In addition, inhibitors of p38 MAPK can affect the activity of a single p38 MAPK isoform or more than one isoform of p38 MAPK. In a certain embodiment of the invention, the inhibitor affects the activity of the α isoform of p38 MAPK.

As used herein, the phrases "inhibitor of JNK" and "JNK inhibitor" are used interchangeably to refer to any substance that decreases, by any measurable extent, the amount (e.g., through transcription, translation or post-translational means) or activity of JNK. JNK inhibitors include, for example, antibodies and antibody fragments, proteins, protein fragments, polypeptides, nucleic acids, and small organic molecules, as described in detail below. Such molecules may be capable of binding to JNK. In addition, inhibitors of JNK can affect the activity of a single JNK isoform or more than one isoform of JNK.

Agents that inhibit both p38 MAPK and JNK are known in the art. Such inhibitors are referred to herein as "p38 MAPK/JNK dual inhibitors."

Inhibitors of p38 MAPK, inhibitors of JNK and p38 MAPK/JNK dual inhibitors are referred to *collectively* as "p38 MAPK and/or JNK inhibitors."

As used herein, the term "patient" refers to an animal, mammal, or human being.

As used herein, the term "tissue factor antagonist" (TF antagonist) refers to a substance that decreases, by any measurable extent, the activity of TF, factor VIIa, or the TF:factor VIIa complex. TF antagonists include, but are not limited to, anti-TF antibodies. As used herein, the phrase "anti-TF antibody" refers to an immunoglobulin molecule or an immunologically active portion of an immunoglobulin molecule that is directed against, and binds to, TF, factor VIIa, or the TF:factor VIIa complex. Anti-TF antibodies can be monoclonal or polyclonal antibodies.

As used herein, the phrase "immunosuppressive agent" refers to any substance that reduces, to any measurable extent, any type of immune response that is raised against a cell, tissue, or organ graft that has been implanted in a patient. Examples include glucocorticosteroids, cyclosporine, anti-CD3 antibodies, sirolimus, tacrolimus, and daclizumab.

The methods of the invention are applicable to the transplantation of a variety of living materials, including cells, such as stem cells of various lineages; tissues, such as pancreatic islet preparations; or organs, such as hearts or kidneys. Generally, any type of autologous, allogeneic, or xenogeneic transplant in which the transplanted materials are in direct contact with the circulating blood is amenable to the methods of the present invention. The methods of the invention may be applied to any cell or tissue or solid organ that may express or be induced to express TF on the surface of all or some portion of cells comprising the transplanted graft.

Organs such as, for example, skin, kidney, liver, heart, pancreas, bone marrow, small intestine, and lung, can be taken from donors and prepared for transplantation. In some cases, partial hepatectomy tissue can be transplanted from a living donor to a recipient. Digits or limbs can be autografted or re-attached. While the technical difficulties of allografting or xenografting organs and limbs have been overcome, the risk of rejection continues to be a major obstacle in the success of these procedures. As new immunosuppressive agents and regimens are developed, the success of grafting will continue to improve. The appropriate preparation of the patient and graft to avoid unwanted coagulation provides further assurance against ischaemic complications.

Particular embodiments of the invention relate to pre-treating therapeutic cell preparations prior to or during transplantation, thereby enhancing the survival of therapeutic cell preparations once they have been introduced into a patient. The implantation of therapeutic cells is used as a therapy for a disease resulting from a loss or impairment of organ function. These diseases can include, for example diabetes, neurodegenerative diseases such as Parkinson's or Alzheimer's disease and the like. Examples of therapeutic cells suitable for the treatment of diabetes include, for example, islets or any insulin-secreting cell, pancreatic stem cells, precursor or progenitor cells, pancreatic ductal cells, or genetically engineered insulin producing cells. Other disease states utilizing a variety of cell therapies could also benefit from the current invention including but are not limited to hepatocytes for the treatment of liver failure, chromaffin cells for chronic pain, cells that produce clotting factors for hemophilia, and cells that produce nerve growth factors for the treatment of neurodegenerative disease such as Parkinson's or Alzheimer's disease. The term "therapeutic cell" as used herein refers to any cell, organoid or tissue that is employed as an effective treatment of disease or injury.

Other cells that can be therapeutically effective for different applications include bone marrow cells, umbilical cord blood cells, angioblasts, endothelial cells, osteoblasts, smooth muscle cells, kidney cells, stem cells, cardiovascular cells, myofibroblasts, fibroblasts, neural cells, neural precursors, and post partum-derived cells.

The methods of the invention are particularly applicable to pancreatic islet cell transplantation. For allografts of islet cells, islets can be prepared from pancreatic tissue taken from multiorgan donors. The pancreatic duct of *in situ* washed organs is cannulated and liberase enzyme (Boehringer Mannheim, Indianapolis, IN) is perfused. The enzymatically digested pancreas is further mechanically dissociated and the islets are separated in a refrigerated Cobe-2991 centrifuge (Cobe BCT, Lakewood, CO). The pooled islets can be either stored cold or frozen, or cultured for one or two days prior to infusion into the patient.

Stem cells of pancreatic islets have recently been used to prepare insulin responsive tissue or "pseudo" islet-like aggregates by induction and differentiation of stem cells in culture (WO 0326584). Alternatively, the appropriate stem cells can be infused directly into the patient.

Certain embodiments of the invention relate to methods for enhancing the survival of a cell, tissue, or organ graft that has been implanted into a patient comprising contacting the cell, tissue, or organ graft with at least one p38 MAPK and/or JNK inhibitor. The p38 MAPK and/or JNK inhibitor may be an inhibitor of p38 MAPK, an inhibitor of JNK, a p38 MAPK/JNK dual inhibitor, or any combination thereof. In other embodiments, the cell, tissue, or organ graft is contacted with a p38 MAPK and/or JNK inhibitor and a TF antagonist. Treatment of the cell, tissue or organ with the p38 MAPK and/or JNK inhibitor and the TF antagonist may be performed prior to or during transplantation. In particular embodiments, it is performed prior to implantation into the patient.

In addition to treatment of the cell, tissue or organ with the p38 MAPK and/or JNK inhibitor and, optionally, the TF antagonist, certain embodiments of the invention feature concomitant treatment of the recipient patient. For instance, a TF antagonist, a p38 MAPK and/or JNK inhibitor, an immunosuppressive agent, or any combination thereof, may be administered to the patient prior to, and/or during, and/or following implantation of the cell, tissue, or organ graft into the patient.

In particular embodiments of the invention, the cell, tissue, or organ grafts are contacted with a concentration of p38 MAPK and/or JNK inhibitor sufficient to reduce TF expression and/or activity. In addition, the patient is optionally administered a concentration of p38 MAPK and/or JNK inhibitor sufficient to reduce TF expression and/or activity.

p38 MAPK and/or JNK inhibitors may exert their regulatory effects either upstream or downstream of p38 MAPK or JNK, or may exert their effect directly on p38 MAPK, or JNK, or both enzymes. Examples of inhibitor-regulated p38 MAPK or JNK activity include those where the inhibitor decreases transcription and/or translation of p38 MAPK and/or JNK, decreases or inhibits post-translational modification and/or cellular trafficking of p38 MAPK and/or JNK, or shortens the half-life of p38 MAPK and/or JNK. The inhibitor may also reversibly or irreversibly bind p38 MAPK and or/JNK, inhibit their activation, inactivate their enzymatic activities, or otherwise interfere with its interaction with downstream substrates.
In certain embodiments of the invention, if acting on p3 8 MAPK and/or JNK directly, the inhibitor preferably should exhibit an IC₅₀ value on isolated enzyme of about 10 µM or less, preferably 500 nm or less, more preferably 100 nm or less. In an embodiment of the invention related to a selected isoform of JNK or p38 (e.g., p38 α), the inhibitor should exhibit an IC₅₀ value relative to the selected isoform that is several fold, e.g., at least ten fold, less than that observed when the same inhibitor is tested against other isoforms using the same or a comparable assay.

To determine whether a candidate is a p38 MAPK and/or JNK inhibitor, an evaluation of its enzymatic activity and its relative IC₅₀ value can be performed using a variety of convential in *vitro* assays, including those that assess inhibition of kinase or ATPase activity of activated p38 MAPK or JNK. The assays can also evaluate the ability of the candidate to bind to p38 MAPK or JNK, or to reduce or block an identified downstream effect of activated p38 MAPK or JNK, such as, for example, cytokine secretion. For example, a binding assay can be performed in solution or on a solid phase using p38 MAPK or JNK, or a fragment thereof, as a target. By using a binding assay as an initial screen, libraries of compounds can be evaluated for potential regulatory activity. The target can be free in solution, fixed to a support, or expressed in or on the surface of a cell. A label (*ie*. radioactive, fluorescent, quenching, *et cetera*.) can be placed on the target, compound, or both, to determine the presence or absence of binding. Competitive binding assays can also be performed to evaluate the inhibition of binding of a target to a natural or artificial substrate or binding partner. In both binding assays and competitive binding assays, the amount of free label versus bound label can be measured to evaluate binding. Many known variations and adaptations of such approaches exist that minimize interference with binding activity and optimize signal.

For purposes of *in vitro* cellular assays, candidates that represent potential inhibitors of p38 MAPK and/or JNK activity can be added to a cell in any number of ways. Preferably, the candidate is added to the medium in which the cell is growing, such as tissue culture medium for cells grown in culture. The candidate is provided in standard serial dilutions or in an amount determined by analogy to known modulators. Alternatively, the candidate can be encoded by a nucleic acid that is introduced into the cell and the cell in turn produces the candidate itself.

Alternative assays involving *in vitro* analysis of candidate inhibitors include those where cells (HeLa) transfected with DNA encoding relevant kinases can be activated with substances such as sorbitol, IL-1, TNF, or PMA (phorbol myristate acetate). After immunoprecipitation of cell lysates, equal aliquots of immune complexes of the kinases are pre-incubated for an adequate time with a specific concentration of the potential inhibitor followed by addition of kinase substrate buffer mix containing labeled ATP and GST-ATF2 or MBP. After incubation, kinase reactions are stopped by the addition of SDS loading buffer. Phosphorylated substrate is resolved through SDS-PAGE and visualized and quantitated in a phosphorimager. Both p38 MAPK or JNK phosphorylation and IC₅₀ values can be determined by quantitation. *See, for example* Kumar,S., McDonnell, P., Gum, R., Hand, A., Lee, J., and Young, P. (1997) *Biochem. Biophys. Res. Commun.* 235, 533-538, incorporated herein by reference in its entirety.

Other *in vitro* assays, such as human whole blood assays, can also assess the production of TNF-α as a correlate to p38 MAPK activity, for example. In human whole blood assays, venous blood is collected from healthy male volunteers into a heparinized syringe and is used within 2 hours of collection. Test candidates are dissolved in 100 % DMSO and 1 µl aliquots of the candidates ranging from 0 to 1 mM are dispensed into quadruplicate wells of a 24-well microtiter plate (Nunclon Delta SI, Applied Scientific, So. San Francisco, CA). Whole blood is added at a volume of 1 ml/well and the mixture is incubated for 15 minutes with constant shaking (Titer Plate Shaker, Lab-Line Instruments, Inc., Melrose Park, IL) at a humidified atmosphere of 5 % CO₂ at 37° C. Whole blood is cultured either undiluted or at a final dilution of 1:10 with RPMI 1640 (Gibco 31800 + NaHCO₃, Life Technologies, Rockville, MD and Scios, Inc., Sunnyvale, CA). At the end of the incubation period, 10 µl of LPS (E. *coli* 0111:B4, Sigma Chemical Co., St. Louis, MO) is added to each well to a final concentration of 1 or 0.1 µg/ml for undiluted or 1:10 diluted whole blood, respectively. The incubation is continued for an additional 2 hours. The reaction is stopped by placing the microtiter plates in an ice bath and plasma or cell-free supernates are collected by centrifugation at 3000 rpm for 10 minutes at 4° C. The plasma samples are stored at -80° C until they are assayed for TNF-α levels by ELISA, following the directions supplied by Quantikine Human TNF-α assay kit (R&D Systems, Minneapolis, MN). IC₅₀ values are calculated using the concentration of candidate that causes a 50% decrease as compared to a control.

Similar assays include enriched mononuclear cell assays, which begin with freshly isolated or cryopreserved Peripheral Blood Mononuclear Cells (PBMCs) (Clonetics Corp.) that are rinsed and resuspended in a warm mixture of cell growth media. The resuspended cells are then counted and seeded at 1x10⁶ cells/well in a 24-well microtitre plate. The plates are placed in an incubator for an hour to allow the cells to settle in each well. After the cells have settled, the media is aspirated and new media containing 100 ng/ml of the cytokine stimulatory factor lipopolysaccharide (LPS) and a test candidate is added to each well of the microtiter plate. Thus, each well contains PBMCs, LPS, and a test candidate. The cells are then incubated for 2 hours, and the amount of the cytokine Tumor Necrosis Factor Alpha (TNF-α) is measured using an enzyme linked immunosorbent assay (ELISA). One such ELISA for detecting the levels of TNF-α is commercially available from R&D Systems. The amount of TNF-α produced by the PBMCs in each well is then compared to a control well to determine whether the candidate acts as an inhibitor of cytokine production. IC₅₀ values are calculated using the concentration of inhibitor that causes a 50 % decrease as compared to a control.

Assays preferred for use in the present invention include various *in vitro* coagulation assays, as described in detail in the example. These include, but are not limited to, the various plasma clotting assays described in Examples 1-3. As used herein, the term "agent that inhibits coagulation" refers to chemical agents, growth factors, proteins, peptide fragments and the like, which decrease the rate at which cells form aggregates *in vitro,* as determined by the clotting assays described in the examples.

Agents suitable for use in the present invention include protein kinase inhibitors such as, for example, benzyl piperidines, as disclosed in U.S. Patent No. 6,541,477 B2; pyrrolopyridines as disclosed in Olini, G.C. *et al*., 1998, *J. Med. Chem*. 41, 4196; Henry J. R., 1998, *J. Bioorg. Med. Chem. Lett*. 8, 3335; Henry J. R., 1998, *Tetrahedron Lett.* 39, 8763 and Dodd, J. H. *et al*., WO 98/47899; piperidines; piperazines; pyrrolobenzimidazoles , as disclosed in Dodd, J. H., U.S. Patent No. 6,147,096) and pyridinyl imidazoles, as disclosed in Kumar, *et. al*., 2003, *Nat Rev Drug Discov.* 2(9):717-26.

In an exemplary embodiment, the pyridinyl imidazole compound 4-[4-(4-fluorophenyl)-1-(3-phenylpropyl)-5-(4-pyridinyl)-1H-imidazol-2-yl]-3-butyn-1-ol (Compound 1) is used to *decrease in vitro* coagulation rates of tissues and cells. The chemical structure of this compound is disclosed in Wadsworth *et al*., 1999, J. Pharm. Exp. Ther., 291:680-687. Methods for synthesizing this compound have been described in WO 98/478922. In a further embodiment, an indol compound is used to decrease *in vitro* coagulation rates of tissues and cells (Compound 2). The chemical structure of this compound is disclosed in co-pending U.S. Application No. [not available] (based on U.S. Provisional Application No. 60/507768, filed September 30, 2003). All compounds provided are intended for guidance and exemplary purposes only. It should be understood that any inhibitor of p38 MAPK and/or JNK is useful for the present invention provided that it exerts activity relative to p38 MAPK and/or JNK to the extent adequate to exhibit a therapeutic effect in a reasonable dosage and concentration.

Other suitable p38 MAPK inhibitors include, but are not limited to, compounds of formula: 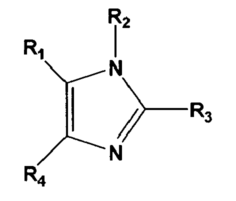 wherein
R₁ is a heteroaryl ring selected from 4-pyridyl, pyrimidinyl, quinolyl, isoquinolinyl, quinazolin-4-yl, 1-imidazolyl, 1-benzimidazolyl, 4-pyridazinyl, and a 1,2,4-triazin-5-yl ring, which heteroaryl ring is substituted one to three times with Y, N(R₁₀)C(O)R_{b}, a halo-substituted mono- or di-C₁₋₆ alkyl-substituted amino, or NHRₐ and which ring is further optionally substituted with C₁₋₄ alkyl, halogen, hydroxyl, optionally-substituted C₁₋₄ alkoxy, optionally-substituted C₁₋₄ alkylthio, optionally-substituted C₁₋₄ alkylsulfinyl, CH₂OR₁₂, amino, mono- and di-C₁₋₆ alkyl-substituted amino, NHRₐ, N(R₁₀)C(O)R_{b}, N(R₁₀)S(O)₂R_{d}, or an N-heterocyclyl ring which has from 5 to 7 members and optionally contains an additional heteroatom selected from oxygen, sulfur or NR₁₅;
Y is X₁-Rₐ;
X₁ is oxygen or sulfur;
Rₐ is C₁₋₆ alkyl, aryl, arylC₁₋₆ alkyl, heterocyclic, heterocyclylC₁₋₆ alkyl, heteroaryl, or heteroarylC₁₋₆ alkyl, wherein each of these moieties can be optionally substituted;
R_{b} is hydrogen, C₁₋₆ alkyl, C₃₋₇ cycloalkyl, aryl, arylC₁₋₄ alkyl, heteroaryl, heteroarylC₁₋₄ alkyl, heterocyclyl, or heterocyclylC₁₋₄ alkyl;
R_{d} is C₁₋₆ alkyl, C₃₋₇ cycloalkyl, aryl, arylC₁₋₄ alkyl, heteroaryl, heteroarylC₁₋₄ alkyl, heterocyclyl, or heterocyclylC₁₋₄ alkyl;
R₃ is hydrogen;
R₄ is phenyl, naphth-1-yl, naphth-2-yl, or a heteroaryl, which is optionally substituted by one or two substituents, each of which is independently selected, and which, for a 4-phenyl, 4-naphth-1-yl, 5-naphth-2-yl or 6-naphth-2-yl substituent, is halogen, cyano, nitro, -C(Z)NR₇R₁₇, -C(Z)OR₁₆, -(CR₁₀R₂₀)ᵥCOR₁₂, -SR₅, -SOR₅, -OR₁₂, halo-substituted-C₁₋₄ alkyl, C₁₋₄ alkyl, - ZC(Z)R₁₂, -NR₁₀C(Z)R₁₆, or -(CR₁₀R₂₀)ᵥNR₁₀R₂₀ and which, for other positions of substitution, is halogen, cyano, -C(Z)NR₁₃R₁₄, -C(Z)OR_{f}, -(CR₁₀R₂₀)_{m"}COR_{f}, -S(O)ₘR_{f}, -OR_{f}, -OR₁₂, halo-substituted C₁₋₄ alkyl, C₁₋₄ alkyl, -(CR₁₀R₂₀)_{m"}NR₁₀C(Z)R_{f}, -NR₁₀S(O)_{m'}R₈, -NR₁₀S(O)_{m'}NR₇R₁₇, -ZC(Z)R_{f}, -ZC(Z)R₁₂, or -(CR₁₀R₂₀)_{m"}NR₁₃R₁₄;
R_{f} is heterocyclyl, heterocyclylC₁₋₁₀ alkyl or R₈;
Z is oxygen or sulfur;
v is 0, 1, or 2;
m is 0, 1, or 2;
m' is 1 or 2;
m" is 0, 1, 2, 3, 4, or 5;
R₂ is C₁₋₁₀ alkyl N₃, -(CR₁₀R₂₀)_{n'}OR₉, heterocylyl, heterocycylC₁₋₁₀ alkyl, C₁₋₁₀ alkyl, halo-substituted C₁₋₁₀ alkyl, C₂₋₁₀ alkenyl, C₂₋₁₀ alkynyl, C₃₋₇ cycloalkyl, C₃₋₇ cycloalkylC₁₋₁₀ alkyl, C₅₋₇ cycloalkenyl, C₅₋₇cycloalkenylC₁₋₁₀ alkyl, aryl, arylC₁₋₁₀ alkyl, heteroaryl, heteroarylC₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄, (CR₁₀R₂₀)ₙNO₂, (CR₁₀R₂₀)ₙCN, (CR₁₀R₂₀)_{n'}SO₂R₁₈, (CR₁₀R₂₀)ₙS(O)_{m'}NR₁₃R₁₄, (CR₁₀R₂₀)ₙC(Z)R₁₁, (CR₁₀R₂₀)ₙOC(Z)R₁₁, (CR₁₀R₂₀)ₙC(Z)OR₁₁, (CR₁₀R₂₀)ₙC(Z)NR₁₃R₁₄, (CR₁₀R₂₀)ₙC(Z)NR₁₁OR₉, (CR₁₀R₂₀)ₙNR₁₀C(Z)R₁₁, (CR₁₀R₂₀)ₙNR₁₀C(Z)NR₁₃R₁₄, (CR₁₀R₂₀)ₙN(OR₆)C(Z)NR₁₃R₁₄, (CR₁₀R₂₀)ₙN(OR₆)C(Z)R₁₁, (CR₁₀R₂₀)ₙC(=NOR₆)R₁₁, (CR₁₀R₂₀)ₙNR₁₀C(=NR₁₉)NR₁₃R₁₄, (CR₁₀R₂₀)ₙOC(Z)NR₁₃R₁₄, (CR₁₀R₂₀)ₙNR₁₀C(Z)NR₁₃R₁₄, (CR₁₀R₂₀)ₙNR₁₀C(Z)OR₁₀, 5-(R₁₈)-1,2,4-oxadiazol-3-yl or 4-(R₁₂)-5-(R₁₈R₁₉)-4,5-dihydro-1,2,4-oxadiazol-3-yl; wherein the aryl, arylalkyl, heteroaryl, heteroaryl alkyl, cycloalkyl, cycloalkyl alkyl, heterocyclic and heterocyclic alkyl groups can be optionally substituted;
n is an integer having a value of 1 to 10;
n' is 0, or an integer having a value of 1 to 10;
R₅ is hydrogen, C₁₋₄ alkyl, C₂₋₄ alkenyl, C₂₋₄ alkynyl or NR₇R₁₇, excluding the moieties - SR₅ being -SNR₇R₁₇ and -S(O)R₅ being -SOH;
R₆ is hydrogen, a pharmaceutically-acceptable cation, C₁₋₁₀ alkyl, C₃₋₇ cycloalkyl, aryl, arylC₁₋₄ alkyl, heteroaryl, heteroarylC₁₋₁₀ alkyl, heterocyclyl, aroyl, or C₁₋₁₀ alkanoyl;
R₇ and R₁₇ are each independently selected from hydrogen or C₁₋₄ alkyl, or R₇ and R₁₇ together with the nitrogen to which they are attached form a heterocyclic ring of 5 to 7 members which ring optionally contains an additional heteroatom selected from oxygen, sulfur or NR₁₅;
R₈ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, arylC₁₋₁₀ alkyl, heteroaryl, heteroarylC₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, or (CR₁₀R₂₀)ₙNR₁₃R₁₄, wherein the aryl, arylalkyl, heteroaryl, and heteroaryl alkyl can be optionally substituted;
R₉ is hydrogen, -C(Z)R₁₁, optionally-substituted C₁₋₁₀ alkyl, S(O)₂R₁₈, optionally-substituted aryl or optionally-substituted arylC₁₋₄ alkyl;
R₁₀ and R₂₀ are each independently selected from hydrogen or C₁₋₄ alkyl;
R₁₁ is hydrogen, C₁₋₁₀ alkyl, C₃₋₇ cycloalkyl, heterocyclyl, heterocyclylC₁₋₁₀ alkyl, aryl, arylC₁₋₁₀ alkyl, heteroaryl or heteroarylC₁₋₁₀ alkyl;
R₁₂ is hydrogen or R₁₆;
R₁₃ and R₁₄ are each independently selected from hydrogen or optionally-substituted C₁₋₄ alkyl, optionally-substituted aryl or optionally-substituted arylC₁₋₄ alkyl, or together with the nitrogen to which they are attached form a heterocyclic ring of 5 to 7 members which ring optionally contains an additional heteroatom selected from oxygen, sulfur or NR₉;
R₁₅ is R₁₀ or C(Z)C₁₋₄ alkyl;
R₁₆ is C₁₋₄ alkyl, halo-substituted C₁₋₄ alkyl, or C₃₋₇ cycloalkyl;
R₁₈ is C₁₋₁₀ alkyl, C₃₋₇ cycloalkyl, heterocyclyl, aryl, arylC₁₋₁₀ alkyl, heterocyclyl, heterocyclylC₁₋₁₀ alkyl, heteroaryl or heteroarylC₁₋₁₀ alkyl; and
R₁₉ is hydrogen, cyano, C₁₋₄ alkyl, C₃₋₇ cycloalkyl or aryl; or a pharmaceutically-acceptable salt thereof,
or wherein
R₁, Y, X₁, Rₐ, R_{b}, R_{d}, v, m, m', m", Z, n, n', and R₅ are defined as above, and
R₂ is hydrogen, C₁₋₁₀ alkyl, halo-substituted C₁₋₁₀ alkyl, C₂₋₁₀ alkenyl, C₂₋₁₀ alkynyl, C₃₋₇ cycloalkyl, C₃₋₇ cycloalkylC₁₋₁₀ alkyl, C₅₋₇ cycloalkenyl, aryl, arylC₁₋₁₀ alkyl, heteroaryl, heteroarylC₁₋₁₀ alkyl, heterocyclyl, heterocyclylC₁₋₁₀ alkyl, (CR₁₀R₂₈)ₙOR₁₂, (CR₁₀R₂₈)_{n'}OR₁₃, (CR₁₀R₂₈)_{n'}S(O)ₘR₂₅, (CR₁₀R₂₈)ₙS(O)₂R₂₅, (CR₁₀R₂₈)_{n'}NHS(O)₂R₂₅, (CR₁₀R₂₈)_{n'}NR₈R₉, (CR₁₀R₂₈)_{n'}NO₂, (CR₁₀R₂₈)_{n'}CN, (CR₁₀R₂₈)_{n'}S(O)ₘNR₈R₉, (CR₁₀R₂₈)_{n'}C(Z)R₁₃, (CR₁₀R₂₈)_{n'}C(Z)OR₁₃, (CR₁₀R₂₈)_{n'}C(Z)NR₈R₉, (CR₁₀R₂₈)_{n'}C(Z)NR₁₃OR₁₂, (CR₁₀R₂₈)_{n'}NR₁₀C(Z)R₁₃, (CR₁₀R₂₈)_{n'}NR₁₀C(Z)NR₈R₉, (CR₁₀R₂₈)_{n'}N(OR₂₁)C(Z)NR₈R₉, (CR₁₀R₂₈)_{n'}N(OR₂₁)C(Z)R₁₃, (CR₁₀R₂₈)_{n'}C(=NOR₂₁)R₁₃, (CR₁₀R₂₈)_{n'}NR₁₀C(=NR₂₇)NR₈R₉, (CR₁₀R₂₈)_{n'}OC(Z)NR₈R₉, (CR₁₀R₂₈)_{n'}NR₁₀C(Z)OR₁₀, (CR₁₀R₂₈)_{n'}NR₁₀C(Z)OR₁₀, 5-(R₂₅)-1,2,4-oxadiazol-3-yl or 4-(R₁₂)-5-(R₁₈R₁₉)-4,5-dihydro-1,2,4-oxadiazol-3-yl; wherein the cycloalkyl, cycloalkyl alkyl, aryl, arylalkyl, heteroaryl, heteroarylalkyl, heterocyclyl, or heterocyclylalkyl moieties can be optionally substituted;
R₃ is hydrogen or Q-(Y₁)ₜ;
Q is an aryl or heteroaryl group;
t is 1, 2, or 3;
Y₁ is independently selected from hydrogen, C₁₋₅ alkyl, halo-substituted C₁₋₅ alkyl, halogen, or -(CR₁₀R₂₀)ₙY₂;
Y₂ is OR₈, NO₂, S(O)_{m"}R₁₁, SR₈, S(O)_{m"}OR₈, S(O)ₘNR₈R₉, NR₈R₉, O(CR₁₀R₂₀)_{n'}NR₈R₉, C(O)R₈, CO₂R₈, CO₂(CR₁₀R₂₀)_{n'}CONR₈R₉, ZC(O)R₈, CN, C(Z)NR₈R₉, NR₁₀C(Z)R₈, C(Z)NR₈OR₉, NR₁₀C(Z)NR₈R₉, NR₁₀S(O)_{m"}R₁₁, N(OR₂₁)C(Z)NR₈R₉, N(OR₂₁)C(Z)R₈, C(=NOR₂₁)R₈, NR₁₀C(=NR₁₅)SR₁₁, NR₁₀C(=NR₁₅)NR₈R₉, NR₁₀C(=CR₁₄R₂₄)SR₁₁, NR₁₀C(=CR₁₄R₂₄)NR₈R₉, NR₁₀C(O)C(O)NR₈R₉, NR₁₀C(O)C(O)OR₁₀, C(=NR₁₃)NR₈R₉, C(=NOR₁₃)NR₈R₉, C(=NR₁₃)ZR₁₁, OC(Z)NR₈R₉, NR₁₀S(O)_{m"}CF₃,NR₁₀C(Z)OR₁₀, 5-(R₁₈)-1,2,4-oxadiazol-3-yl or 4-(R₁₂)-5-(R₁₈R₁₉)-4,5-dihydro-1,2,4-oxadiazol-3-yl;
R₄ is phenyl, naphth-1-yl or naphth-2-yl which is optionally substituted by one or two substituents, each of which is independently selected, and which, for a 4-phenyl, 4-naphth-1-yl or 5-naphth-2-yl substituent, is halo, nitro, cyano, C(Z)NR₇R₁₇, C(Z)OR₂₃, (CR₁₀R₂₀)ᵥCOR₃₆, SR₅, SOR₅, OR₃₆, halo-substituted-C₁₋₄ alkyl, C₁₋₄ alkyl, ZC(Z)R₃₆, NR₁₀C(Z)R₂₃, or (CR₁₀R₂₀)ᵥNR₁₀R₂₀ and which, for other positions of substitution, is halo, nitro, cyano, C(Z)NR₁₆R₂₆, C(Z)OR₈, (CR₁₀R₂₀)_{m"}COR₈, S(O)ₘR₈, OR₈, halo-substituted-C₁₋₄ alkyl, C₁₋₄ alkyl, (CR₁₀R₂₀)_{m"}NR₁₀C(Z)R₈, NR₁₀S(O)_{m'}R₁₁, NR₁₀S(O)_{m'}NR₇R₁₇, ZC(Z)R₈ or (CR₁₀R₂₀)_{m"}NR₁₆R₂₆;
R₇ and R₁₇ are each independently selected from hydrogen or C₁₋₄ alkyl, or R₇ and R₁₇ together with the nitrogen to which they are attached form a heterocyclic ring of 5 to 7 members, which ring optionally contains an additional heteroatom selected from oxygen, sulfur or NR₂₂;
R₈ is hydrogen, heterocyclyl, heterocyclylalkyl or R₁₁;
R₉ is hydrogen, C₁₋₁₀ alkyl, C₂₋₁₀ alkenyl, C₂₋₁₀ alkynyl, C₃₋₇ cycloalkyl, C₅₋₇ cycloalkenyl, aryl, arylalkyl, heteroaryl or heteroarylalkyl, or R₈ and R₉ can together with the nitrogen to which they are attached form a heterocyclic ring of 5 to 7 members, which ring optionally contains an additional heteroatom selected from oxygen, sulfur or NR₁₂;
R₁₀ and R₂₀ are each independently selected from hydrogen or C₁₋₄ alkyl;
R₁₁ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, arylalkyl, heteroaryl or heteroarylalkyl;
R₁₂ is hydrogen, -C(Z)R₁₃ or optionally-substituted C₁₋₄ alkyl, optionally-substituted aryl, optionally-substituted arylC₁₋₄ alkyl, or S(O)₂R₂₅;
R₁₃ is hydrogen, C₁₋₁₀ alkyl, C₃₋₇ cycloalkyl, heterocyclyl, heterocyclylC₁₋₁₀ alkyl, aryl, arylC₁₋₁₀ alkyl, heteroaryl or heteroaryl C₁₋₁₀ alkyl, wherein all of these moieties can be optionally substituted;
R₁₄ and R₂₄ are each independently selected from hydrogen, alkyl, nitro or cyano;
R₁₅ is hydrogen, cyano, C₁₋₄ alkyl, C₃₋₇ cycloalkyl or aryl;
R₁₆ and R₂₆ are each independently selected from hydrogen or optionally-substituted C₁₋₄ alkyl, optionally-substituted aryl or optionally-substituted arylC₁₋₄ alkyl, or together with the nitrogen to which they are attached form a heterocyclic ring of 5 to 7 members, which ring optionally contains an additional heteroatom selected from oxygen, sulfur or NR₁₂ ;
R₁₈ and R₁₉ are each independently selected from hydrogen, C₁₋₄ alkyl, substituted alkyl, optionally-substituted aryl, optionally-substituted arylalkyl, or together denote an oxygen or sulfur;
R₂₁ is hydrogen, a pharmaceutically-acceptable cation, C₁₋₁₀ alkyl, C₃₋₇ cycloalkyl, aryl, arylC₁₋₄ alkyl, heteroaryl, heteroarylalkyl, heterocyclyl, aroyl, or C₁₋₁₀ alkanoyl;
R₂₂ is R₁₀ or C(Z)-C₁₋₄ alkyl;
R₂₃ is C₁₋₄ alkyl, halo-substituted-C₁₋₄ alkyl, or C₃₋₅ cycloalkyl;
R₂₅ is C₁₋₁₀ alkyl, C₃₋₇ cycloalkyl, heterocyclyl, aryl, arylalkyl, heterocyclyl, heterocyclylC₁₋₁₀ alkyl, heteroaryl or heteroarylalkyl;
R₂₇ is hydrogen, cyano, C₁₋₄ alkyl, C₃₋₇ cycloalkyl, or aryl;
R₂₈ is hydrogen, C₁₋₆ alkyl, C₃₋₇ cycloalkyl, aryl, arylC₁₋₄ alkyl, heteroaryl, heteroarylC₁₋₄alkyl, heterocyclyl, or heterocyclylC₁₋₄ alkyl moiety, all of which can be optionally substituted; and
R₃₆ is hydrogen or R₂₃;
or a pharmaceutically acceptable salt thereof.

Exemplary compounds of the above formula include, but are not limited to:
1-[3-(4-morpholinyl)propyl]-4-(4-fluorophenyl)-5-(4-pyridyl)imidazole;
1-(3-chloropropyl)-4-(4-fluorophenyl)-5-(4-pyridyl)imidazole;
1-(3-azidopropyl)-4-(4-fluorophenyl)-5-(4-pyridyl)imidazole;
1-(3-aminopropyl)-4-(4-fluorophenyl)-5-(4-pyridyl)imidazole;
1-(3-methylsulfonamidopropyl)-4-(4-fluorophenyl)-5-(4-pyridyl)imidazole;
1-[3-(N-phenylmethyl)aminopropyl]-4-(4-fluorophenyl)-5-(4-pyridyl)imidazole;
1-[3-(N-phenylmethyl-N-methyl)aminopropyl]-4-(4-fluorophenyl)-5-(4-pyridyl)imidazole;
1-[3-(1-pyrrolidinyl)propyl]-4-(4-fluorophenyl)-5-(4-pyridyl)imidazole;
1-(3-diethylaminopropyl)-4-(4-fluorophenyl)-5-(4-pyridyl)imidazole;
1-[3-(1-piperidinyl)propyl]-4-(4-fluorophenyl)-5-(4-pyridyl)imidazole;
1-[3-(methylthio)propyl]-4-(4-fluorophenyl)-5-(4-pyridyl)imidazole;
1-[2-(4-morpholinyl)ethyl]-4-(4-fluorophenyl)-5-(4-pyridyl)imidazole;
1-[3-(4-morpholinyl)propyl]-4-(3-methylthiophenyl)-5-(4-pyridyl)imidazole;
(+/-)-1-[3-(4-morpholinyl)propyl]-4-(3-methylsulfinylphenyl)-5-(4-pyridyl)imidazole;
1-[3-(N-methyl-N-benzyl)aminopropyl]-4-(3-methylthiophenyl)-5-(4-pyridyl)imidazole;
1-[3-(N-methyl-N-benzyl)aminopropyl]-4-(3-methylsulfinylphenyl)-5-(4-pyridyl)imidazole;
1-[4-(methylthio)phenyl]-4-(4-fluorophenyl)-5-(4-pyridyl)imidazole;
1-[4-(methylsulfinyl)phenyl]-4-(4-fluorophenyl)-5-(4-pyridyl)imidazole;
1-[3-(methylthio)phenyl]-4-(4-fluorophenyl)-5-(4-pyridyl)imidazole;
(+/-)-1-[3-(methylsulfinyl)phenyl]-4-(4-fluorophenyl)-5-(4-pyridyl)imidazole;
1-[2-(methylthio)phenyl]-4-(4-fluorophenyl)-5-(4-pyridyl)imidazole;
1-[2-(methylsulfinyl)phenyl]-4-(4-fluorophenyl)-5-(4-pyridyl)imidazole;
1-[4-(4-morpholinyl)butyl]-4-(4-fluorophenyl)-5-(4-pyridyl)imidazole;
1-cyclopropyl-4-(4-fluorophenyl)-5-(4-pyridyl)imidazole;
1-isopropyl-4-(4-fluorophenyl)-5-(4-pyridyl)imidazole;
1-cyclopropylmethyl-4-(4-fluorophenyl)-5-(4-pyridyl)imidazole;
1-*tert*-butyl-4-(4-fluorophenyl)-5-(4-pyridyl)imidazole;
1-(2,2-diethoxyethyl)-4-(4-fluorophenyl)-5-(4-pyridyl)imidazole;
1-formylmethyl-4-(4-fluorophenyl)-5-(4-pyridyl)imidazole;
1-hydroxyiminylmethyl-4-(4-fluorophenyl)-5-(4-pyridyl)imidazole;
1-cyanomethyl-4-(4-fluorophenyl)-5-(4-pyridyl)imidazole;
1-[3-(4-morpholinyl)propyl)-4-(4-fluorophenyl)-5-(2-methylpyrid-4- yl)imidazole;
4-(4-fluorophenyl)-1-[3-(4-morpholinyl)propyl]-5-(2-chloropyridin-4-yl)imidazole;
4-(4-fluorophenyl)-1-[3-(4-morpholinyl)propyl]-5-(2-amino-4-pyridinyl)imidazole;
1-(4-carboxymethyl)propyl-4-(4-fluorophenyl)-5-(4-pyridyl)imidazole;
1-(4-carboxypropyl)-4-(4-fluorophenyl)-5-(4-pyridyl)imidazole;
1-(3-carboxymethyl)ethyl-4-(4-fluorophenyl)-5-(4-pyridyl)imidazole;
1-(3-carboxy)ethyl-4-(4-fluorophenyl)-5-(4-pyridyl)imidazole;
1-(1-benzylpiperidin-4-yl)-4-(4-fluorophenyl)-5-(4-pyridyl)imidazole;
5-(2-aminopyrimidin-4-yl)-4-(4-fluorophenyl)-1-[3-(4-morpholinyl)propyl]imidazole;
5-(2-aminopyrimidin-4-yl)-4-(4-fluorophenyl)-1-(1-benzylpiperidin-4-yl)imidazole;
5-(2-aminopyrimidin-4-yl)-4-(4-fluorophenyl)-1-(2-propyl)imidazole;
5-(2-aminopyrimidin-4-yl)-4-(4-fluorophenyl)-1-(cyclopropylmethyl)imidazole;
5-(2-aminopyrimidin-4-yl)-4-(4-fluorophenyl)-1-(1-carboxyethyl-4-piperidinyl)imidazole;
5-(2-aminopyrimidin-4-yl)-4-(4-fluorophenyl)-1-(4-piperidinyl)imidazole;
1-methyl-4-phenyl-5-(4-pyridyl)imidazole;
1-methyl-4-[3-(chlorophenyl)]-5-(4-pyridinyl)imidazole;
1-methyl-4-(3-methylthiophenyl)-5-(4-pyridyl)imidazole;
(+/-)-1-methyl-4-(3-methylsulfinylphenyl)-5-(4-pyridyl)imidazole;
(+/-)-4-(4-fluorophenyl)-1-[3-(methylsulfinyl)propyl]-5-(4-pyridinyl)imidazole;
4-(4-fluorophenyl)-1-[(3-methylsulfonyl)propyl]-5-(4-pyridinyl)imidazole;
1-(3-phenoxypropyl)-4-(4-fluorophenyl)-5-(4-pyridinyl)imidazole;
1-[3-(phenylthio)propyl]-4-(4-fluorophenyl)-5-(4-pyridinyl)imidazole;
1-[3-(4-morpholinyl)propyl]-4-(4-fluorophenyl)-5-(4-quinolyl)imidazole;
(+/-)-1-(3-phenylsulfinylpropyl)-4-(4-fluorophenyl)-5-(4-pyridinyl)imidazole;
1-(3-ethoxypropyl)-4-(4-fluorophenyl)-5-(4-pyridinyl)imidazole;
1-(3-phenylsulfonylpropyl-4-(4-fluorophenyl)-5-(4-pyridinyl)imidazole;
1-[3-(4-morpholinyl)propyl]-4-(3-chlorophenyl)-5-(4-pyridyl)imidazole;
1-[3-(4-morpholinyl)propyl]-4-(3,4-dichlorophenyl)-5-(4-pyridyl)imidazole;
4-[4-(4-fluorophenyl)-1-[3-(4-morpholinyl)propyl]-5-(pyrimid-2-one-4-yl)imidazole;
4-(4-fluorophenyl)-5-[2-(methylthio)-4-pyrimidinyl]-1-[3-(4-morpholinyl)propyl]imidazole;
(+/-)-4-(4-fluorophenyl)-5-[2-(methylsulfinyl)-4-pyrimidinyl]-1-[3-(4-morpholinyl)propyl]imidazole;
1-(1-propenyl)-4-(4-fluorophenyl)-5-(4-pyridinyl)imidazole;
1-(2-propenyl)-4-(4-fluorophenyl)-5-(4-pyridinyl)imidazole;
5-[(2-N,N-dimethylamino)pyrimidin-4-yl]-4-(4-fluorophenyl)-1-[3-(4-morpholinyl)propyl]imidazole;
1-[3-(4-morpholinyl)propyl]-5-(4-pyridinyl)-4-[4-(trifluoromethyl)phenyl]imidazole;
1-[3-(4-morpholinyl)propyl]-5-(4-pyridinyl)-4-[3-(trifluoromethyl)phenyl]imidazole;
1-(cyclopropylmethyl)-4-(3,4-dichlorophenyl)-5-(4-pyridinyl)imidazole;
1-(cyclopropylmethyl)-4-(3-trifluoromethylphenyl)-5-(4-pyridinyl)imidazole;
1-(cyclopropylmethyl)-4-(4-fluorophenyl)-5-(2-methylpyrid-4-yl)imidazole;
1-[3-(4-morpholinyl)propyl]-5-(4-pyridinyl)-4-(3,5-bistrifluoromethylphenyl)imidazole;
5-[4-(2-aminopyrimidinyl)]-4-(4-fluorophenyl)-1-(2-carboxy-2,2-dimethylethyl)imidazole;
1-(1-formyl-4-piperidinyl)-4-(4-fluorophenyl)-5-(4-pyridinyl)imidazole;
5-(2-amino-4-pyrimidinyl)-4-(4-fluorophenyl)-1-(1-methyl-4-piperidinyl)imidazole;
1-(2,2-dimethyl-3-morpholin-4-yl)propyl-4-(4-fluorophenyl)-5-(2-amino-4-pyrimidinyl)imidazole;
4-(4-fluorophenyl)-5-(4-pyridyl)-1-(2-acetoxyethyl)imidazole;
5-(2-aminopyrimidin-4-yl)-4-(4-fluorophenyl)-1-(1-benzylpyrrolin-3-yl)imidazole;
5-(2-aminopyrimidin-4-yl)-4-(4-fluorophenyl)-1-(2,2,6,6-tetramethylpiperidin-4-yl)imidazole;
5-[4-(2-N-methylamino)pyrimidinyl]-4-(4-fluorophenyl)-1-(4-N-methylpiperidine)imidazole;
5-[4-(2-N-methylamino)pyrimidinyl]-4-(4-fluorophenyl)-1-(4-N-morpholino-1-propyl)imidazole;
5-[4-(2-N-methylamino)pyrimidinyl)-4-(4-fluorophenyl)-1-(4-piperidine)imidazole;
5-[(2-ethylamino)pyrimidin-4-yl]-4-(4-fluorophenyl)-1-(1-methylpiperidin-4-yl)imidazole;
4-(4-fluorophenyl)-5-[2-(isopropyl)aminopyrimidin-4-yl]-1-(1-methylpiperidin-4-yl)imidazole;
5 -(2-acetamido-4-pyrimidinyl)-4-(4-fluorophenyl)-1-(4-N-morpholino-1-propyl)imidazole;
5-(2-acetamido-4-pyrimidinyl)-4-(4-fluorophenyl)-1-(1-methyl-4-piperidinyl)imidazole;
5-[4-(2-N-methylthio)pyrimidinyl]-4-(4-fluorophenyl)-1-(4-piperidine)imidazole;
4-(fluorophenyl)-1-(methyl-4-piperidinyl)-5-(2-methylthio-4-pyrimidinyl)imidazole;
4-(fluorophenyl)-1-(methyl-4-piperidinyl)-5-(2-methysulfinyl-4-pyrimidinyl)imidazole;
1-*tert*-butyl-4-(4-fluorophenyl)-5-(2-methysulfinyl-4-pyrimidinyl)imidazole;
5-[4-(2-aminopyrimidinyl)]-4-(4-fluorophenyl)-1-(2,2,6,6-tetramethyl-4-piperidinyl)imidazole;
5-[4-(2-N-methylamino-4-pyrimidinyl)]-4-(4-fluorophenyl)-1-(2,2,6,6-tetramethyl-4-piperidine)imidazole;
5-(2-amino-4-pyrimidinyl)-4-(4-fluorophenyl)-1-(tetrahydro-4-thiopyranyl)imidazole;
5-(2-amino-4-pyrimidinyl)-4-(4-fluorophenyl)-1-(tetrahydro-4-pyranyl)imidazole;
5-(2-methylamino-4-pyrimidinyl)-4-(4-fluorophenyl)-1-(2-cyanoethyl)imidazole;
5-(2-amino-4-pyrimidinyl)-4-(4-fluorophenyl)-1-(tetrahydro-4-sulfinylpyranyl)imidazole;
5-(2-amino-4-pyrimidinyl)-4-(4-fluorophenyl)-1-(tetrahydro-4-sulfonylpyranyl)imidazole;
5-(2-methylamino-4-pyrimidinyl)-4-(4-fluorophenyl)-1-(2,2,2-trifluoroethyl-4-piperidinyl)imidazole;
5-(2-amino-4-pyrimidinyl)-4-(4-fluorophenyl)-1-(trifluoroacetyl-4-piperidinyl)imidazole;
5-(4-pyridyl)-4-(4-fluorophenyl)-1-(4-piperidinyl)imidazole;
5-(4-pyridyl)-4-(4-fluorophenyl)-1-(1-*t*-butoxycarbonyl-4-piperidinyl)imidazole;
5-(2-amino-4-pyrimidinyl)-4-(4-fluorophenyl)-1-(4-(1,3-dioxycyclopentyl)cyclohexyl)imidazole;
5-(2-amino-4-pyrimidinyl)-4-(4-fluorophenyl)-1-(4-ketocyclohexyl)imidazole;
5-(2-amino-4-pyrimidinyl)-4-(4-fluorophenyl)-1-(4-cyclohexyl oxime) imidazole;
5-(2-amino-4-pyrimidinyl)-4-(4-fluorophenyl)-1-(4-cyclohexyl hydroxylamine) imidazole;
5-(2-amino-4-pyrimidinyl)-4-(4-fluorophenyl)-1-(*trans*-4-hydroxyurea) imidazole;
5-(2-amino-4-pyrimidinyl)-4-(4-fluorophenyl)-1-(*cis*-4-hydroxyurea) imidazole;
5-(2-amino-4-pyrimidinyl)-4-(4-fluorophenyl)-1-(4-hydroxycyclohexyl)imidazole;
5-[4-(2-N-methylamino)pyrimidinyl]-4-(4-fluorophenyl)-1-(4-ketocyclohexyl)imidazole;
5-[4-(2-N-methylamino)pyrimidinyl]-4-(4-fluorophenyl)-1-(*trans*-4-hydroxycyclohexyl)imidazole;
5-[4-(2-N-methylamino)pyrimidinyl]-4-(4-fluorophenyl)-1-(*cis*-4-hydroxycyclohexyl)imidazole;
5-[4-(2-N-methylamino)pyrimidinyl]-4-(4-fluorophenyl)-1-[4-(*cis-*pyrrolidinyl)cyclohexyl]imidazole;
5-[4-(2-N-methylamino)pyrimidinyl]-4-(4-fluorophenyl)-1-[4-(*trans*-1-pyrrolidinyl)cyclohexyl]imidazole;
5-[4-(2-N-methylamino)pyrimidinyl]-4-(4-fluorophenyl)-1-(4-ethynyl-4-hydroxycyclohexyl)imidazole;
5-[4-(2-N-methylamino)pyrimidinyl]-4-(4-fluorophenyl)-1-(4-(1-propynyl)-4-hydroxycyclohexyl)imidazole;
5-[4-(2-N-methylamino)pyrimidinyl]-4-(4-fluorophenyl)-1-(4-amino-4-methylcyclohexyl)imidazole;
5-[4-(2-N-methylamino)pyrimidinyl]-4-(4-fluorophenyl)-1-(4-acetamido-4-methylcyclohexyl)imidazole;
5-[4-(2-N-methylamino)pyrimidinyl]-4-(4-fluorophenyl)-1-(4-hydroxy-4-methylcyclohexyl)imidazole;
5-[4-(2-N-methylamino)pyrimidinyl]-4-(4-fluorophenyl)-1-(4-oxiranylcyclohexyl)imidazole;
5-[4-(2-N-methylamino)pyrimidinyl]-4-(4-fluorophenyl)-1-(4-cyanomethyl-4-hydroxycyclohexyl)imidazole;
5-[4-(2-N-methylamino)pyrimidinyl]-4-(4-fluorophenyl)-1-(4-hydroxy-4-hydroxymethylcyclohexyl)imidazole;
5-[4-(2-amino)pyrimidinyl]-4-(4-fluorophenyl)-1-[4-hydroxy-4-(1-propynyl)-cyclohexyl]imidazole;
5-[4-(2-amino)pyrimidinyl]-4-(4-fluorophenyl)-1-(4-hydroxy-4-methylcyclohexyl)imidazole;
5-[4-(2-N-methylamino)pyrimidinyl]-4-(4-fluorophenyl)-1-(4-hydroxy-4-isopropyl-cyclohexyl)imidazole;
5-[4-(2-N-methylamino)pyrimidinyl]-4-(4-fluorophenyl)-1-(4-hydroxy-4-phenylcyclohexyl)imidazole;
5-[4-(2-N-methylamino)pyrimidinyl]-4-(4-fluorophenyl)-1-(4-hydroxy-4-benzyl-cyclohexyl)imidazole;
5-[4-(2-N-methylamino)pyrimidinyl]-4-(4-fluorophenyl)-1-(4-hydroxy-4-cyanomethyl cyclohexyl)imidazole;
5-[4-(2-N-methylamino)pyrimidinyl]-4-(4-fluorophenyl)-1-(4-hydroxy-4-(2-cyanoethyl)cyclohexyl)imidazole;
5-[4-(2-N-methylamino)pyrimidinyl]-4-(4-fluorophenyl)-1-(4-hydroxy-4-(2-aminoethyl)cyclohexyl)imidazole;
5-[4-(2-N-methylamino)pyrimidinyl]-4-(4-fluorophenyl)-1-(4-hydroxy-4-(2-nitroethyl)-cyclohexyl)imidazole;
5-[4-(2-N-methylamino)pyrimidinyl]-4-(4-fluorophenyl)-1-(4-hydroxymethyl-4-aminocyclohexyl)imidazole;
5-[4-(2-N-methylamino)pyrimidinyl]-4-(4-fluorophenyl)-1-(4-hydroxy-4-aminocyclohexyl)imidazole;
5-[4-(2-N-methylamino)pyrimidinyl]-4-(4-fluorophenyl)-1-(4-aminocyclohexyl)imidazole;
5-[4-(2-N-methylamino)pyrimidinyl]-4-(4-fluorophenyl)-1-(4-hydroxy-4-thiomethyl cyclohexyl)imidazole;
5-[4-(2-N-methylamino)pyrimidinyl]-4-(4-fluorophenyl)-1-(4-hydroxy-4-hydroxy methylcyclohexyl)imidazole;
5-[4-(2-N-methylamino)pyrimidinyl]-4-(4-fluorophenyl)-1-(4-hydroxy-4-aminomethylcyclohexyl)imidazole;
5-[4-(2-amino)pyrimidinyl]-4-(4-fluorophenyl)-1-(4-amino-4-methylcyclohexyl)imidazole;
5-[4-(2-amino)pyrimidinyl]-4-(4-fluorophenyl)-1-(4-hydroxy-4-methylcyclohexyl)imidazole;
5-[4-(2-amino)pyrimidinyl]-4-(4-fluorophenyl)-1-(4-oxiranylcyclohexyl)imidazole;
4-(fluorophenyl)-1-(methyl-4-piperidinyl)-5-(2-methysulfinyl-4-pyrimidinyl)imidazole;
4-(fluorophenyl)-1-(methyl-4-piperidinyl)-5-(2-methylthio-4-pyrimidinyl)imidazole;
5-[(2-benzylamino)pyrimidin-4-yl]-4-(4-fluorophenyl)-1-(1-methylpiperidin-4-yl)imidazole;
4-(4-fluorophenyl)-1-(1-methylpiperidin-4-yl)-5-[2-(4-tetrahydrothiopyranyl)aminopyrimidin-4-yl]imidazole;
4-(4-fluorophenyl)-5-[(2-hydroxy)ethylamino]pyrimidin-4-yl-1-(1-methylpiperidin-4-yl)imidazole;
5-[(2-(3-chlorobenzylamino)pyrimidin-4-yl]-4-(4-fluorophenyl)-1-(1-methylpiperidin-4-yl)imidazole;
5-[(2-(1-naphthylmethylamino)pyrimidin-4-yl]-4-(4-fluorophenyl)-1-(1-methylpiperidin-4-yl)imidazole;
5-[(2-(1-benzyl-4-piperidinylamino)pyrimidin-4-yl]-4-(4-fluorophenyl)-1-(1-methylpiperidin-4-yl)imidazole;
4-(4-fluorophenyl)-1-(1-methylpiperidin-4-yl)-5-[2-[3-(morpholino)propyl]aminopyrimidin-4-yl]imidazole;
5-[2-[(3-bromophenyl)amino]pyrimidin-4-yl]-4-(4-fluorophenyl)-1-(1-methylpiperidin-4-yl)imidazole;
5-[(2-(piperonylamino)pyrimidin-4-yl]-4-(4-fluorophenyl)-1-(1-methylpiperidin-4-yl)imidazole;
5-[(2-(4-piperidinylamino)pyrimidin-4-yl]-4-(4-fluorophenyl)-1-(1-methylpiperidin-4-yl)imidazole;
5-[(2-(5-chlorotryptamino)pyrimidin-4-yl]-4-(4-fluorophenyl)-1-(1-methylpiperidin-4-yl)imidazole;
5-[(2-(2,2,6,6-tetramethylpiperidin-4-yl)aminopyrimidin-4-yl]-4-(4- fluorophenyl)-1-(1-methylpiperidin-4-yl)imidazole;
5-[(2-[1-ethoxycarbonyl)piperidin-4-yl]aminopyrimidin-4-yl]-4-(4-fluorophenyl)-1-(1-methylpiperidin-4-yl)imidazole;
1-(4-oxocyclohexyl)-4-(4-fluorophenyl)-5-[(2-methoxy)pyrimidin-4-yl]imidazole;
*cis*-1-(4-hydroxycyclohexyl)-4-(4-fluorophenyl)-5-[(2-methoxy)pyrimidin-4-yl]imidazole;
*trans*-1-(4-hydroxycyclohexyl)-4-(4-fluorophenyl)-5-[(2-methoxy)pyrimidin-4-yl]imidazole;
1-(4-oxocyclohexyl)-4-(4-fluorophenyl)-5-[(2-methylthio)pyrimidin-4-yl]imidazole;
*trans*-1-(4-hydroxycyclohexyl)-4-(4-fluorophenyl)-5-[(2methylthio)pyrimidin-4-yl]imidazole;
1-(4-oxocyclohexyl)-4-(4-fluorophenyl)-5-[(2-hydroxy)pyrimidin-4-yl]imidazole;
1-(4-oxocyclohexyl)-4-(4-fluorophenyl)-5-[(2-isopropoxy)pyrimidin-4-yl]imidazole;
1-(4-hydroxycyclohexyl)-4-(4-fluorophenyl)-5-[(2-isopropoxy)pyrimidin-4-yl]imidazole;
*trans*-1-(4-hydroxy-4-methylcyclohexyl)-4-(4-fluorophenyl)-5-[(2-methoxy)pyrimidin-4-yl]imidazole;
*cis*- 1-(4-hydroxy-4-methylcyclohexyl)-4-(4-fluorophenyl)-5-[(2- methoxy)pyrimidin-4-yl]imidazole;
*trans*-1-(4-hydroxycyclohexyl)-4-(4-fluorophenyl)-5-[(2-ethoxy)pyrimidin-4-yl]imidazole;
1-(4-piperidinyl)-4-(4-fluorophenyl)-5-(2-phenoxypyrimidin-4-yl)imidazole;
1-(4-piperidinyl)-4-(4-fluorophenyl)-5-(2-phenoxy-4-pyridinyl)imidazole;
1-(4-piperidinyl)-4-(4-fluorophenyl)-5-[2-(4-methoxyphenoxy)-4-pyridinyl]imidazole;
1-(4-piperidinyl)-4-(4-fluorophenyl)-5-[2-(4-fluorophenoxy)-4- pyridinyl]imidazole;
1-(piperidin-4-yl)-4-(4-fluorophenyl)-5-[2-(4-methoxyphenoxy)pyrimidin-4-yl]imidazole;
1-(piperidin-4-yl)-4-(4-fluorophenyl)-5-[2-(4-fluorophenoxy)pyrimidin-4-yl]imidazole;
1-(piperidin-4-yl)-4-(4-fluorophenyl)-5-[2-(4-aminocarbonylphenoxy)pyrimidin-4-yl]imidazole;
1-(piperidin-4-yl)-4-(4-fluorophenyl)-5-[2-(4-ethylphenoxy)pyrimidin-4-yl]imidazole;
1-(piperidin-4-yl)-4-(4-fluorophenyl)-5-[2-(4-benzyloxyphenoxy)pyrimidin-4-yl]imidazole;
1-(piperidin-4-yl)-4-(4-fluorophenyl)-5-[2-(4-cyanophenoxy)pyrimidin-4-yl]imidazole;
1-(piperidin-4-yl)-4-(4-fluorophenyl)-5-[2-(4-hydroxyphenoxy)pyrimidin-4-yl]imidazole;
1-(4-hydroxycyclohexyl)-4-(4-fluorophenyl)-5-[2-(phenoxy)pyrimidin-4-yl]imidazole;
1-(piperidin-4-yl)-4-(4-fluorophenyl)-5-[2-(2,6-dimethylphenoxy)pyridin-4-yl]imidazole;
1-(piperidin-4-yl)-4-(4-fluorophenyl)-5-[2-(4-methylphenoxy)pyridin-4-yl]imidazole;
1-(piperidin-4-yl)-4-(4-fluorophenyl)-5-[2-(4-chlorophenoxy)pyridin-4-yl]imidazole;
1-[3-(N-morpholino)propyl]-4-(4-fluorophenyl)-5-[2-(phenoxy)pyrimidin-4-yl]imidazole;
1-(piperidin-4-yl)-4-(4-fluorophenyl)-5-[2-(3-methoxyphenoxy)pyrimidin-4-yl]imidazole;
1-(piperidin-4-yl)-4-(4-fluorophenyl)-5-[2-(4-phenylphenoxy)pyrimidin-4-yl]imidazole;
1-(piperidin-4-yl)-4-(4-fluorophenyl)-5-[2-(4-phenoxyphenoxy)pyrimidin-4-yl]imidazole;
1-(piperidin-4-yl)-4-(4-fluorophenyl)-5-[2-(3-hydroxyphenoxy)pyrimidin-4-yl]imidazole;
1-(3-(N-morpholino)propyl)-4-(4-fluorophenyl)-5-[2-(4-fluorophenoxy)pyrimidin-4-yl]imidazole;
1-(piperidin-4-yl)-4-(4-fluorophenyl)-5-[2-(2-hydroxyphenoxy)pyrimidin-4-yl]imidazole;
1-(piperidin-4-yl)-4-(4-fluorophenyl)-5-[2-((3,4-methylenedioxy)phenoxy)pyrimidin-4-yl]imidazole;
1-(piperidin-4-yl)-4-(4-fluorophenyl)-5-[2-(3-fluorophenoxy)pyrimidin-4-yl]imidazole;
1-(piperidin-4-yl)-4-(4-fluorophenyl)-5-[2-(2-fluorophenoxy)pyrimidin-4-yl]imidazole;
1-(piperidin-4-yl)-4-(4-fluorophenyl)-5-[2-(2-methoxyphenoxy)pyrimidin-4-yl]imidazole;
1-(piperidin-4-yl)-4-(4-fluorophenyl)-5-[2-(3-trifluoromethylphenoxy)pyrimidin-4-yl]imidazole;
1-(piperidin-4-yl)-4-(4-fluorophenyl)-5-[2-(3,4-difluorophenoxy)pyrimidin-4-yl]imidazole;
1-(piperidin-4-yl)-4-(4-fluorophenyl)-5-[2-(4-methylsulfonylphenoxy)pyrimidin-4-yl]imidazole;
1-(4-piperidinyl)-4-(4-fluorophenyl)-5-(2-thiophenoxypyrimidin-4yl)imidazole;
1-(4-piperidinyl)-4-(4-fluorophenyl)-5-[2-(1-methyltetrazol-5-ylthio)pyridin-4-yl]imidazole;
5-[2-(2-hydroxyethoxy)pyrimidin-4-yl]-4-(4-fluorophenyl)-1-(4-oxocyclohexyl)imidazole;
5-[2-(2-hydroxyethoxy)]pyrimidin-4-yl)-4-(4-fluorophenyl)-1-(4-hydroxycyclohexyl)imidazole;
5-[2-(2-*tert*-butylamino)ethoxypyrimidin-4-yl]-4-(4-fluorophenyl)-1-(4-oxocyclohexyl)imidazole;
5-[2-(2-*tert*-butylamino)ethoxypyrimidin-4-yl]-4-(4-fluorophenyl)-1-(4-hydroxycyclohexyl)imidazole;
1-(4-piperidinyl)-4-(4-Fluorophenyl)-5-(2-isopropoxy-4-pyrimidinyl)imidazole;
1-(4-piperidinyl)-4-(4-Fluorophenyl)-5-(2-methoxy-4-pyrimidinyl)imidazole;
5-(2-hydroxy-4-pyrimidinyl)-4-(4-fluorophenyl)-1-(4-piperidinyl)imidazole;
5-(2-methoxy-4-pyridinyl)-4-(4-fluorophenyl)-1-(4-piperidinyl)imidazole;
5-(2-isopropoxy-4-pyridinyl)-4-(4-fluorophenyl)-1-(4-piperidinyl)imidazole;
5-(2-methylthio-4-pyrimidinyl)-4-(4-fluorophenyl)-1-(4-piperidinyl)imidazole;
5-(2-methylthio-4-pyrimidinyl)-4-(4-fluorophenyl)-1-[ 1-methyl-4-piperidinyl]imidazole;
5-(2-ethoxy-4-pyrimidinyl)-4-(4-fluorophenyl)-1-(4-piperidinyl)imidazole;
1-(1-ethylcarboxylpiperidin-4-yl)-3-(4-thiomethylphenyl)-5-[2-(thiomethyl)pyrimidin-4-yl]-imidazole;
1-(1-ethylcarbonylpiperidin-4-yl)-4-(4-methylsulfinylphenyl)-5-[(2-methylsulfinyl)pyrimidin-4-yl]imidazole;
2-(4-methylthiophenyl)-4-(4-fluorophenyl)-5-(2-methoxy-4- pyrimidinyl)imidazole;
2-(4-methylsulfinylphenyl)-4-(4-fluorophenyl)-5-(2-methoxy-4- pyrimidinyl)imidazole;
2-[(4-N,N-dimethyl)aminomethylphenyl]-4-(4-fluorophenyl)-5-(2-methoxy-4-pyrimidinyl)imidazole;
2-[(4-N,N-dimethyl)aminomethylphenyl]-4-(4-fluorophenyl)-5-(2-phenoxy-4-pyrimidinyl)imidazole;
(+/-)-2-(4-methylsulfinylphenyl]-4-(4-fluorophenyl)-5-(2-phenoxy-4-pyrimidinyl)imidazole;
2-(4-methylthiophenyl]-4-(4-fluorophenyl)-5-(2-phenoxy-4- pyrimidinyl)imidazole; and pharmaceutically acceptable salts thereof.

Additional examples of p38 MAPK inhibitors include, but are not limited to, compounds of the following formula: 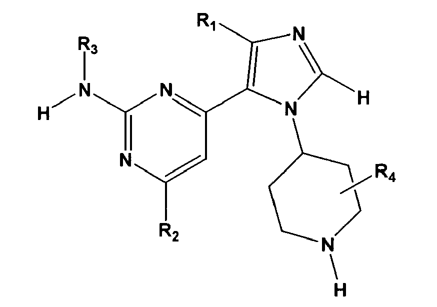 wherein
R₁ is hydrogen, C₁₋₅ alkyl, halogen, C₁₋₅ alkoxy, or arylC₁₋₅ alkyl;
R₂ and R₄ are independently hydrogen, C₁₋₅ alkyl, aryl, arylC₁₋₅ alkyl, heteroaryl, heteroarylC₁₋₅ alkyl, heterocyclic, or heterocyclicC₁₋₅ alkyl; and
R₃ is hydrogen or C₁₋₃ alkyl;
or a pharmaceutically-acceptable salt thereof.

Another class of compounds that are useful as p38 MAPK inhhibitors include, but are not limited to, compounds of the following formula: 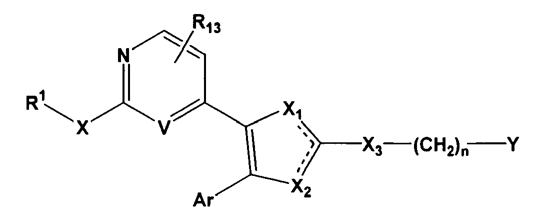 wherein
X is O, CH₂, S or NH, or the moiety X-R¹ is hydrogen;
R¹ is hydrogen, C₁₋₆ alkyl, C₃₋₇ cycloalkyl, aryl, arylC₁₋₆ alkyl, heterocyclyl, heterocyclylC₁₋₆ alkyl, heteroaryl, or heteroarylC₁₋₆ alkyl, any of which, except for hydrogen, can be optionally substituted;
V is CH or N;
Ar is an aryl or heteroaryl ring, either of which can be optionally substituted;
one of X₁ and X₂ is N, and the other is NR¹⁵, wherein R¹⁵ is hydrogen, C₁₋₆ alkyl, or arylC₁₋₆ alkyl;
X₃ is a covalent bond or C(R²)(R³);
R² and R³ independently represent optionally substituted C₁₋₆ alkyl, or R² and R³ together with the carbon atom to which they are attached form an optionally substituted C₃₋₇ cycloalkyl, C₃₋₇ cycloalkenyl, or 5- to 7-membered heterocyclyl ring containing up to three heteroatoms independently selected from N, O, and S;
n is 0, 1, 2, 3, or 4;
Y is NR¹⁰R¹¹, NR¹⁰C(Z)NR¹⁰R¹¹, NR¹⁰COOR¹¹, NR¹⁰SO₂R¹¹, or C(O)NR⁴R⁵;
R⁴ and R⁵ independently represent hydrogen, C₁₋₆ alkyl, C₃₋₇ cycloalkyl, aryl, arylC₁₋₆ alkyl, heteroaryl, heteroarylC₁₋₆ alkyl, heterocyclyl, or heterocyclylC₁₋₆ alkyl, any one of which, except hydrogen, can be optionally substituted, or R⁴ and R⁵ together with the nitrogen atom to which they are attached form a 4- to 10-membered optionally-substituted mono- or bicyclic ring;
R¹³ is hydrogen, X-R¹, halogen, optionally-substituted C₁₋₆ alkylsulfinyl, CH₂OR¹⁴, di-C₁₋₆ alkylamino, N(R⁶)C(O)R⁷, N(R⁶)S(O)₂R⁸, or a 5- to 7-membered N-heterocyclyl ring which optionally contains an additional heteroatom selected from O, S, and NR⁹;
R¹⁴ is hydrogen, -C(Z)R¹² or optionally-substituted C₁₋₆ alkyl, optionally-substituted aryl, optionally-substituted arylC₁₋₆ alkyl or S(O)₂R⁸;
R⁶ is hydrogen or C₁₋₆ alkyl;
R⁷ is hydrogen, C₁₋₆ alkyl, C₃₋₇ cycloalkyl, aryl, arylC₁₋₆ alkyl, heteroaryl, heteroarylC₁₋₆ alkyl, heterocyclyl or heterocyclylC₁₋₆ alkyl;
R⁸ is C₁₋₆ alkyl, C₃₋₇ cycloalkyl, aryl, arylC₁₋₆ alkyl, heteroaryl, heteroarylC₁₋₆ alkyl, heterocyclyl or heterocyclylC₁₋₆ alkyl;
R⁹ is hydrogen, cyano, C₁₋₄ alkyl, C₃₋₇ cycloalkyl or aryl;
R¹⁰, R¹¹ and R¹² are independently selected from hydrogen, C₁₋₆ alkyl, C₃₋₇ cycloalkyl, heterocyclyl, heterocyclylC₁₋₆ alkyl, heterocyclylC₂₋₆ alkenyl, aryl, arylC₁₋₆ alkyl, arylC₂₋₆ alkenyl, heteroaryl, heteroarylC₁₋₆ alkyl and heteroarylC₂₋₆ alkenyl, any of which can be optionally substituted; or NR¹⁰R¹¹ can represent a 5- to 7-membered heterocyclyl ring optionally containing an additional heteroatom selected from O, N and S; and
Z is oxygen or sulfur;
or a pharmaceutically-acceptable salt thereof.

Additional compounds useful as p38 MAPK inhibitors include, but are not limited to, compounds of the following formulas: 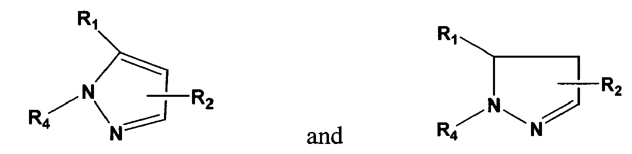 wherein
R₁ is a heteroaryl selected from 4-pyridyl, 4-pyrimidinyl, 4-quinolyl, 6-isoquinolinyl, quinazolin-4-yl, 1-imidazolyl, 1-benzimidazolyl, 4-pyridazinyl, and a 1,2,4-triazin-5-yl ring, which heteroaryl ring is substituted one to three times with Y, NHRₐ, optionally-substituted C₁₋₄ alkyl, halogen, hydroxyl, optionally-substituted C₁₋₄ alkoxy, optionally-substituted C₁₋₄ alkylthio, optionally-substituted C₁₋₄ alkylsulfinyl, CH₂OR₁₂, amino, mono- and di-C₁₋₆ alkyl-substituted amino, N(R₁₀)C(O)R_{b}, N(R₁₀)S(O)₂R_{d}, or an N-heterocyclyl ring which has from 5 to 7 members and optionally contains an additional heteroatom selected from oxygen, sulfur or NR₁₅;
Y is X₁-Rₐ;
X₁ is oxygen or sulfur;
Rₐ is C₁₋₆ alkyl, aryl, arylC₁₋₆ alkyl, heterocyclic, heterocyclylC₁₋₆ alkyl, heteroaryl, or heteroarylC₁₋₆ alkyl, wherein each of these moieties can be optionally substituted;
R_{b} is hydrogen, C₁₋₆ alkyl, C₃₋₇ cycloalkyl, aryl, arylC₁₋₄ alkyl, heteroaryl, heteroarylC₁₋₄ alkyl, heterocyclyl, or heterocyclylC₁₋₄ alkyl;
R_{d} is C₁₋₆ alkyl, C₃₋₇ cycloalkyl, aryl, arylC₁₋₄ alkyl, heteroaryl, heteroarylC₁₋₄ alkyl, heterocyclyl, or heterocyclylC₁₋₄ alkyl;
R₄ is phenyl, naphth-1-yl, naphth-2-yl, a heteroaryl or a fused phenyl-containing ring system, which is optionally substituted by one or two substituents, each of which is independently selected, and which, for a 4-phenyl, 4-naphth-1-yl, 5-naphth-2-yl or 6-naphth-2-yl substituent, is halogen, cyano, nitro, -C(Z)NR₇R₁₇, -C(Z)OR₁₆, -(CR₁₀R₂₀)ᵥCOR₁₂, -SR₅, -SOR₅, -OR₁₂, halo-substituted-C₁₋₄ alkyl, C₁₋₄ alkyl, -ZC(Z)R₁₂, -NR₁₀C(Z)R₁₆, or -(CR₁₀R₂₀)ᵥNR₁₀R₂₀ and which, for other positions of substitution, is halogen, cyano, nitro, phenyl, -C(Z)NR₁₃R₁₄, -C(Z)OR_{f}, -(CR₁₀R₂₀)_{m"}COR_{f}, -S(O)ₘR_{f}, -OR_{f}, halo-substituted C₁₋₄ alkyl, C₁₋₁₀ alkyl, -ZC(Z)R_{f}, optionally-substituted phenyl, -(CR₁₀R₂₀)_{m"}NR₁₀C(Z)R_{f}, -NR₁₀S(O)_{m'}R₈, -NR₁₀S(O)_{m'}NR₇R₁₇, -ZC(Z)R₁₂, or -(CR₁₀R₂₀)_{m"}NR₁₃R₁₄;
R_{f} is heterocyclyl, heterocyclylC₁₋₁₀ alkyl or R₈;
v is 0, 1, or 2;
m is 0, 1, or 2;
m' is 1 or 2;
m" is 0, 1, 2, 3, 4, or 5;
R₂ hydrogen, -(CR₁₀R₂₃)ₙOR₉, heterocylyl, heterocyclylC₁₋₁₀ alkyl, C₁₋₁₀ alkyl, halo-substituted C₁₋₁₀ alkyl, C₂₋₁₀ alkenyl, C₂₋₁₀ alkynyl, C₃₋₇ cycloalkyl, C₃₋₇ cycloalkylC₁₋₁₀ alkyl, C₅₋₇ cycloalkenyl, C₅₋₇cycloalkenylC₁₋₁₀ alkyl, aryl, arylC₁₋₁₀ alkyl, heteroaryl, heteroarylC₁₋₁₀ alkyl, (CR₁₀R₂₃)ₙOR₁₁, (CR₁₀R₂₃)ₙS(O)ₘR₁₈, (CR₁₀R₂₃)ₙNHS(O)₂R₁₈, (CR₁₀R₂₃)ₙNR₁₃R₁₄, (CR₁₀R₂₃)ₙNO₂, (CR₁₀R₂₃)ₙCN, (CR₁₀R₂₃)ₙS(O)_{m'}NR₁₃R₁₄, (CR₁₀R₂₃)ₙC(Z)R₁₁, (CR₁₀R₂₃)ₙOC(Z)R₁₁, (CR₁₀R₂₃)ₙC(Z)OR₁₁, (CR₁₀R₂₃)ₙC(Z)NR₁₃R₁₄, (CR₁₀R₂₃)ₙC(Z)NR₁₁OR₉, (CR₁₀R₂₃)ₙNR₁₀C(Z)R₁₁, (CR₁₀R₂₃)ₙNR₁₀C(Z)NR₁₃R₁₄, (CR₁₀R₂₃)ₙN(OR₆)C(Z)NR₁₃R₁₄, (CR₁₀R₂₃)ₙN(OR₆)C(Z)R₁₁, (CR₁₀R₂₃)ₙC(=NOR₆)R₁₁, (CR₁₀R₂₃)ₙNR₁₀C(=NR₁₉)NR₁₃R₁₄, (CR₁₀R₂₃)ₙOC(Z)NR₁₃R₁₄, (CR₁₀R₂₃)ₙNR₁₀C(Z)NR₁₃R₁₄, (CR₁₀R₂₃)ₙNR₁₀C(Z)OR₁₀, 5-(R₁₈)-1,2,4-oxadiazol-3-yl or 4-(R₁₂)-5-(R₁₈R₁₉)-4,5-dihydro-1,2,4-oxadiazol-3-yl; wherein the aryl, arylalkyl, heteroaryl, heteroaryl alkyl, cycloalkyl, cycloalkyl alkyl, heterocyclic and heterocyclic alkyl groups can be optionally substituted;
n is 0, or an integer having a value of 1 to 10;
Z is oxygen or sulfur;
R₅ is hydrogen, C₁₋₄ alkyl, C₂₋₄ alkenyl, C₂₋₄ alkynyl or NR₇R₁₇, excluding the moieties -SR₅ being -SNR₇R₁₇ and -S(O)R₅ being -SOH;
R₆ is hydrogen, a pharmaceutically-acceptable cation, C₁₋₁₀ alkyl, C₃₋₇ cycloalkyl, aryl, arylC₁₋₄ alkyl, heteroaryl, heteroarylC₁₋₄ alkyl, heterocyclyl, aroyl, or C₁₋₁₀ alkanoyl;
R₇ and R₁₇ are each independently selected from hydrogen or C₁₋₄ alkyl, or R₇ and R₁₇ together with the nitrogen to which they are attached form a heterocyclic ring of 5 to 7 members which ring optionally contains an additional heteroatom selected from oxygen, sulfur or NR₁₅;
R₈ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, arylC₁₋₁₀ alkyl, heteroaryl, heteroarylC₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, or (CR₁₀R₂₀)ₙNR₁₃R₁₄, wherein the aryl, arylalkyl, heteroaryl, and heteroaryl alkyl can be optionally substituted;
R₉ is hydrogen, -C(Z)R₁₁, optionally-substituted C₁₋₁₀ alkyl, S(O)₂R₁₈, optionally-substituted aryl or optionally-substituted arylC₁₋₄ alkyl;
R₁₀ and R₂₀ are each independently selected from hydrogen or C₁₋₄ alkyl;
R₁₁ is hydrogen, C₁₋₁₀ alkyl, C₃₋₇ cycloalkyl, heterocyclyl, heterocyclylC₁₋₁₀ alkyl, aryl, arylC₁₋₁₀ alkyl, heteroaryl or heteroarylC₁₋₁₀ alkyl, wherein the aryl, arylalkyl, heteroaryl, heteroaryl alkyl, heterocyclyl or heterocyclylalkyl can be optionally substituted;
R₁₂ is hydrogen or R₁₆;
R₁₃ and R₁₄ are each independently selected from hydrogen or optionally-substituted C₁₋₄ alkyl, optionally-substituted aryl or optionally-substituted arylC₁₋₄ alkyl, or together with the nitrogen to which they are attached form a heterocyclic ring of 5 to 7 members which ring optionally contains an additional heteroatom selected from oxygen, sulfur or NR₉;
R₁₅ is hydrogen, C₁₋₄ alkyl or C(Z)-C₁₋₄ alkyl;
R₁₆ is C₁₋₄ alkyl, halo-substituted C₁₋₄ alkyl, or C₃₋₇ cycloalkyl;
R₁₈ is C₁₋₁₀ alkyl, C₃₋₇ cycloalkyl, heterocyclyl, aryl, arylC₁₋₁₀ alkyl, heterocyclyl, heterocyclylC₁₋₁₀ alkyl, heteroaryl or heteroarylC₁₋₁₀ alkyl, wherein the aryl, arylalkyl, heteroaryl, heteroaryl alkyl, heterocyclyl or heterocyclylalkyl can be optionally substituted;
R₁₉ is hydrogen, cyano, C₁₋₄ alkyl, C₃₋₇ cycloalkyl or aryl; and
R₂₃ is hydrogen, C₁₋₆ alkyl, C₃₋₇ cycloalkyl, aryl, arylC₁₋₄ alkyl, heteroaryl, heteroarylC₁₋₄ alkyl, heterocyclyl, or heterocyclylC₁₋₄ alkyl, all of which can be optionally substituted; or a pharmaceutically-acceptable salt thereof.

Exemplary compounds of this class include, but are not limited to:
4-[1-(4-fluorophenyl)-3-phenyl-1*H*-pyrazol-5-yl]pyridine
4-[4-bromo-1-(4-fluorophenyl)-3-phenyl-1*H*-pyrazol-5-yl]pyridine
4-[1-(4-fluorophenyl)-3-[4-(methylthio)phenyl]-1*H*-pyrazol-5-yl]pyridine
4-[1-(4-fluorophenyl)-3-[4-(methylsulfonyl)phenyl]-1*H*-pyrazol-5-yl]pyridine 4-[1-(4-fluorophenyl)-3-[4-(methylsulfinyl)phenyl]-1*H*-pyrazol-5-yl]pyridine;
4-[1-(4-fluorophenyl)-4,5-dihydro-3-phenyl-1*H*-pyrazol-5-yl]pyridine
4-[1-(4-fluorophenyl)-4,5-dihydro-3-[4-(methylthio)phenyl]-1*H*-pyrazol-5-yl]pyridine and pharmaceutically acceptable salts thereof.

Similar compounds that are also useful as p38 MAPK inhibitors include, but are not limited to, compounds of the following formulas: 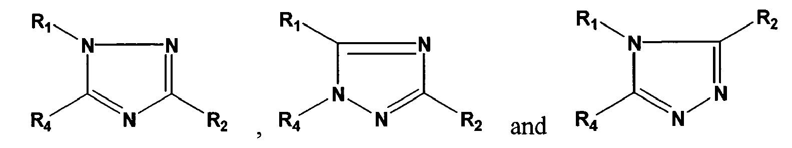 wherein
R₁ is 4-pyridyl or 4-pyrimidinyl ring, which ring is optionally substituted one or more times with Y, C₁₋₄ alkyl, halogen, hydroxyl, C₁₋₄ alkoxy, C₁₋₄ alkylthio, C₁₋₄ alkylsulfinyl, CH₂OR₁₂, amino, mono- and di-C₁₋₆ alkyl-substituted amino, N(R₁₀)C(O)R_{b}, or an N-heterocyclyl ring which has from 5 to 7 members and optionally contains an additional heteroatom selected from oxygen, sulfur or NR₁₅;
Y is X₁-Rₐ;
X₁ is oxygen, sulfur, or NH;
Rₐ is C₁₋₆ alkyl, aryl, arylC₁₋₆ alkyl, heterocyclic, heterocyclylC₁₋₆ alkyl, heteroaryl, or heteroarylC₁₋₆ alkyl, wherein each of these moieties can be optionally substituted;
R_{b} is hydrogen, C₁₋₆ alkyl, C₃₋₇ cycloalkyl, aryl, arylC₁₋₄ alkyl, heteroaryl, heteroarylC₁₋₄ alkyl, heterocyclyl, or heterocyclylC₁₋₄ alkyl, wherein each of these moieties can be optionally substituted;
R₄ is phenyl, naphth-1-yl, naphth-2-yl, or a heteroaryl, which is optionally substituted by one or two substituents, each of which is independently selected, and which, for a 4-phenyl, 4-naphth-1-yl, 5-naphth-2-yl or 6-naphth-2-yl substituent, is halogen, cyano, nitro, -C(Z)NR₇R₁₇, -C(Z)OR₁₆, -(CR₁₀R₂₀)ᵥCOR₁₂, -SR₅, -SOR₅, -OR₁₂, halo-substituted-C₁₋₄ alkyl, C₁₋₄ alkyl, - ZC(Z)R₁₂, -NR₁₀C(Z)R₁₆, or -(CR₁₀R₂₀)ᵥNR₁₀R₂₀ and which, for other positions of substitution, is halogen, cyano, -C(Z)NR₁₃R₁₄, -C(Z)OR_{f}, -(CR₁₀R₂₀)_{m"}COR_{f}, -S(O)ₘR_{f}, -OR_{f}, halo-substituted C₁₋₄ alkyl, C₁₋₄ alkyl, -ZC(Z)R_{f}, -(CR₁₀R₂₀)_{m"}NR₁₀C(Z)R_{f}, -NR₁₀S(O)_{m'}R₈, -NR₁₀S(O)_{m'}NR₇R₁₇, or -(CR₁₀R₂₀)_{m"}NR₁₃R₁₄;
R_{f} is heterocyclyl, heterocyclylC₁₋₁₀ alkyl or R₈;
v is 0, 1, or 2;
m is 0, 1, or 2;
m' is 1 or 2;
m" is 0, 1, 2, 3, 4, or 5;
R₂ hydrogen, C(HOURS)(A)(R₂₂), -(CR₁₀R₂₃)ₙOR₉, heterocylyl, heterocyclylC₁₋₁₀ alkyl, C₁₋₁₀ alkyl, halo-substituted C₁₋₁₀ alkyl, C₂₋₁₀ alkenyl, C₂₋₁₀ alkynyl, C₃₋₇ cycloalkyl, C₃₋₇ cycloalkylC₁₋₁₀ alkyl, C₅₋₇ cycloalkenyl, C₅₋₇cycloalkenylC₁₋₁₀ alkyl, aryl, arylC₁₋₁₀ alkyl, heteroaryl, heteroarylC₁₋₁₀ alkyl, (CR₁₀R₂₃)ₙOR₁₁, (CR₁₀R₂₃)ₙS(O)ₘR₁₈, (CR₁₀R₂₃)ₙNHS(O)₂R₁₈, (CR₁₀R₂₃)ₙNR₁₃R₁₄, (CR₁₀R₂₃)ₙNO₂, (CR₁₀R₂₃)ₙCN, (CR₁₀R₂₃)ₙS(O)_{m'}NR₁₃R₁₄, (CR₁₀R₂₃)ₙC(Z)R₁₁, (CR₁₀R₂₃)ₙOC(Z)R₁₁, (CR₁₀R₂₃)ₙC(Z)OR₁₁, (CR₁₀R₂₃)ₙC(Z)NR₁₃R₁₄, (CR₁₀R₂₃)ₙC(Z)NR₁₁OR₉, (CR₁₀R₂₃)ₙNR₁₀C(Z)R₁₁, (CR₁₀R₂₃)ₙNR₁₀C(Z)NR₁₃R₁₄, (CR₁₀R₂₃)ₙN(OR₆)C(Z)NR₁₃R₁₄, (CR₁₀R₂₃)ₙN(OR₆)C(Z)R₁₁, (CR₁₀R₂₃)ₙC(=NOR₆)R₁₁, (CR₁₀R₂₃)ₙNR₁₀C(=NR₁₉)NR₁₃R₁₄, (CR₁₀R₂₃)ₙOC(Z)NR₁₃R₁₄, (CR₁₀R₂₃)ₙNR₁₀C(Z)NR₁₃R₁₄, (CR₁₀R₂₃)ₙNR₁₀C(Z)OR₁₀, 5-(R₁₈)-1,2,4-oxadiazol-3-yl or 4-(R₁₂)-5-(R₁₈R₁₉)-4,5-dihydro-1,2,4-oxadiazol-3-yl; wherein the aryl, arylalkyl, heteroaryl, heteroaryl alkyl, cycloalkyl, cycloalkyl alkyl, heterocyclic and heterocyclic alkyl groups can be optionally substituted;

A is an optionally-substituted aryl, heterocyclyl or heteroaryl ring, or A is a substituted C₁₋₁₀ alkyl;
n is 0, or an integer having a value of 1 to 10;
Z is oxygen or sulfur;
R₅ is hydrogen, C₁₋₄ alkyl, C₂₋₄ alkenyl, C₂₋₄ alkynyl or NR₇R₁₇, excluding the moieties -SR₅ being -SNR₇R₁₇ and -S(O)R₅ being -SOH;
R₆ is hydrogen, a pharmaceutically-acceptable cation, C₁₋₁₀ alkyl, C₃₋₇ cycloalkyl, aryl, arylC₁₋₄ alkyl, heteroaryl, heteroarylC₁₋₄ alkyl, heterocyclyl, aroyl, or C₁₋₁₀ alkanoyl;
R₇ and R₁₇ are each independently selected from hydrogen or C₁₋₄ alkyl, or R₇ and R₁₇ together with the nitrogen to which they are attached form a heterocyclic ring of 5 to 7 members which ring optionally contains an additional heteroatom selected from oxygen, sulfur or NR₁₅;
R₈ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, arylC₁₋₁₀ alkyl, heteroaryl, heteroarylC₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, or (CR₁₀R₂₀)ₙNR₁₃R₁₄, wherein the aryl, arylalkyl, heteroaryl, and heteroaryl alkyl can be optionally substituted;
R₉ is hydrogen, -C(Z)R₁₁, optionally-substituted C₁₋₁₀ alkyl, S(O)₂R₁₈, optionally-substituted aryl or optionally-substituted arylC₁₋₄ alkyl;
R₁₀ and R₂₀ are each independently selected from hydrogen or C₁₋₄ alkyl;
R₁₁ is hydrogen, C₁₋₁₀ alkyl, C₃₋₇ cycloalkyl, heterocyclyl, heterocyclylC₁₋₁₀ alkyl, aryl, arylC₁₋₁₀ alkyl, heteroaryl or heteroarylC₁₋₁₀ alkyl, wherein the aryl, arylalkyl, heteroaryl, heteroaryl alkyl, heterocyclyl or heterocyclylalkyl can be optionally substituted;
R₁₂ is hydrogen or R₁₆;
R₁₃ and R₁₄ are each independently selected from hydrogen or optionally-substituted C₁₋₄ alkyl, optionally-substituted aryl or optionally-substituted arylC₁₋₄ alkyl, or together with the nitrogen to which they are attached form a heterocyclic ring of 5 to 7 members which ring optionally contains an additional heteroatom selected from oxygen, sulfur or NR₉;
R₁₅ is R₁₀ or C(Z)C₁₋₄ alkyl;
R₁₆ is C₁₋₄ alkyl, halo-substituted C₁₋₄ alkyl, or C₃₋₇ cycloalkyl;
R₁₈ is C₁₋₁₀ alkyl, C₃₋₇ cycloalkyl, heterocyclyl, aryl, arylC₁₋₁₀ alkyl, heterocyclyl, heterocyclylC₁₋₁₀ alkyl, heteroaryl or heteroarylC₁₋₁₀ alkyl;
R₁₉ is hydrogen, cyano, C₁₋₄ alkyl, C₃₋₇ cycloalkyl or aryl; and
R₂₃ is hydrogen, C₁₋₆ alkyl, C₃₋₇ cycloalkyl, aryl, arylC₁₋₄ alkyl, heteroaryl, heteroarylC₁₋₄ alkyl, heterocyclyl, or heterocyclylC₁₋₄ alkyl, all of which can be optionally substituted; or a pharmaceutically-acceptable salt thereof.
Examples of this class of compounds include, but are not limited to:
1-(pyrid-4-yl)-3-phenyl-5-(4-fluorophenyl)-1,2,4-triazole;
1-(6-aminopyrimidin-4-yl)-3-phenyl-5-(4-fluorophenyl)-1,2,4-triazole;
1-[4-(6,7-dimethoxyquinazoline)]-3-phenyl-5-(4-fluorophenyl)-1,2,4- triazole;
1-(4-fluorophenyl)-3-phenyl-5-(2-aminopyrimidin-4-yl)-1,2,4-triazole;
3-(4-fluorophenyl)-4-(2-aminopyrimidin-4-yl)-5-phenyl- 1,2,4-triazole; and pharmaceutically acceptable salts thereof.

Yet another class of compounds that are useful as p38 MAPK inhibitors includes compounds of the following formula: 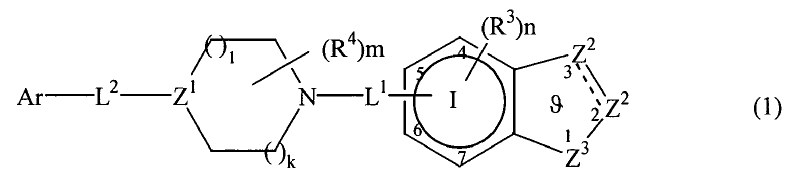 and the pharmaceutically acceptable salts thereof, or a pharmaceutical composition thereof, wherein
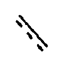 represents a single or double bond;
one Z² is CA or CR⁸A and the other is CR¹, CR¹₂, NR⁶ or N wherein each R¹, R⁶ and R⁸ is independently hydrogen or noninterfering substituent;
A is -CO(X)ⱼY wherein Y is COR² or an isostere thereof and R² is hydrogen or a noninterfering substituent, X is a spacer preferably of 2-6Å, and j is 0 or 1;
Z³ is NR⁷ or O;
each R³ is independently a noninterfering substituent;
n is 0-3;
each of L¹ and L² is a linker;
each R⁴ is independently a noninterfering substituent;
m is 0-4;
Z¹ is CR⁵ or N wherein R⁵ is hydrogen or a noninterfering substituent;
each of 1 and k is an integer from 0-2 wherein the sum of 1 and k is 0-3;
Ar is an aryl group substituted with 0-5 noninterfering substituents, wherein two noninterfering substituents can form a fused ring; and the distance between the atom of Ar linked to L² and the center of the α ring is preferably less than 24Å. As provided above, certain positions of the molecule are described as permitting "noninterfering substituents." This terminology is where there is a wide variety of substituents possible in these positions as determined by those skilled in the art with the proviso however that the substituents must not impart a detrimental effect on the essential activity or application of the molecule when taken as a whole.

Exemplary compounds of this formula include, but are not limited to, those in the following Table A:
TABLE A

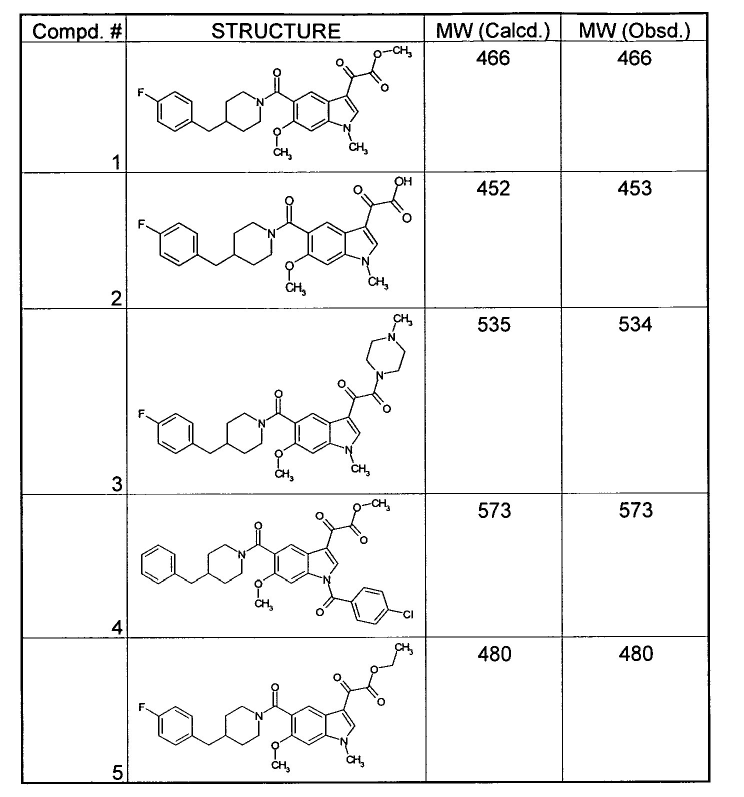

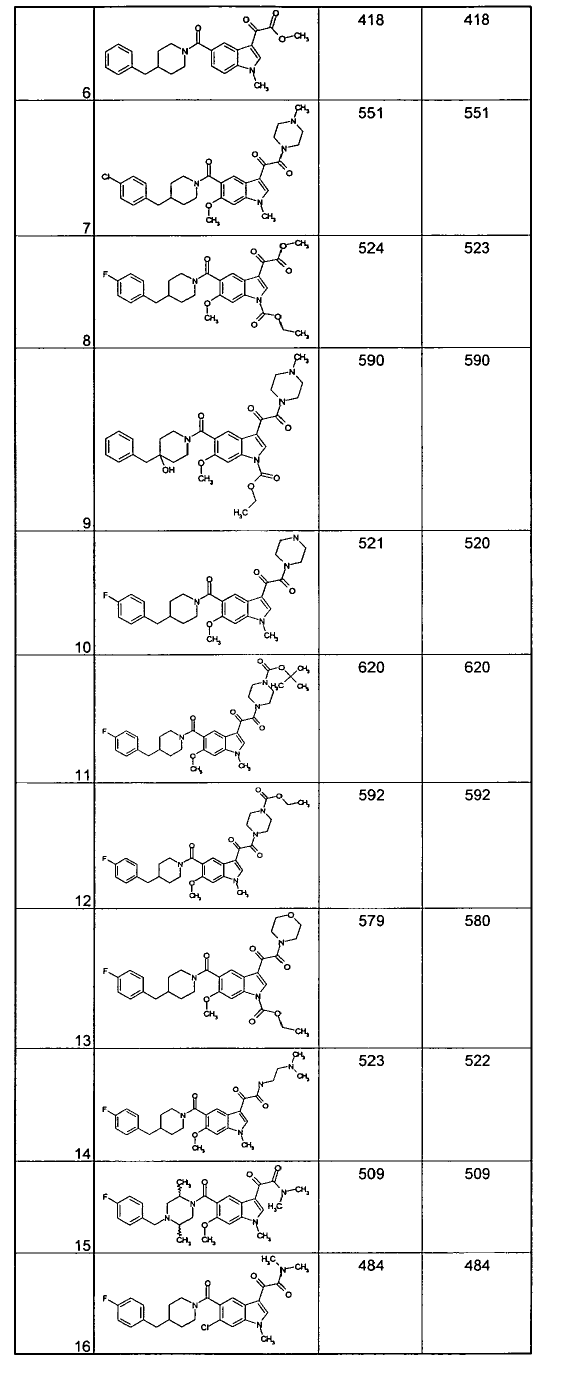

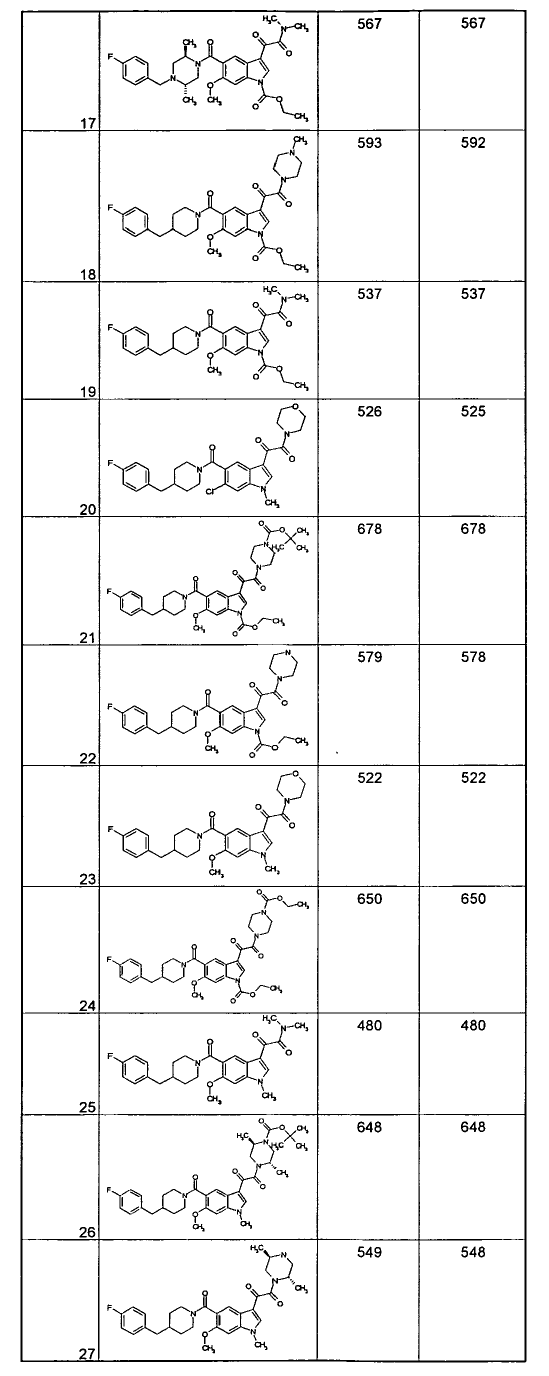

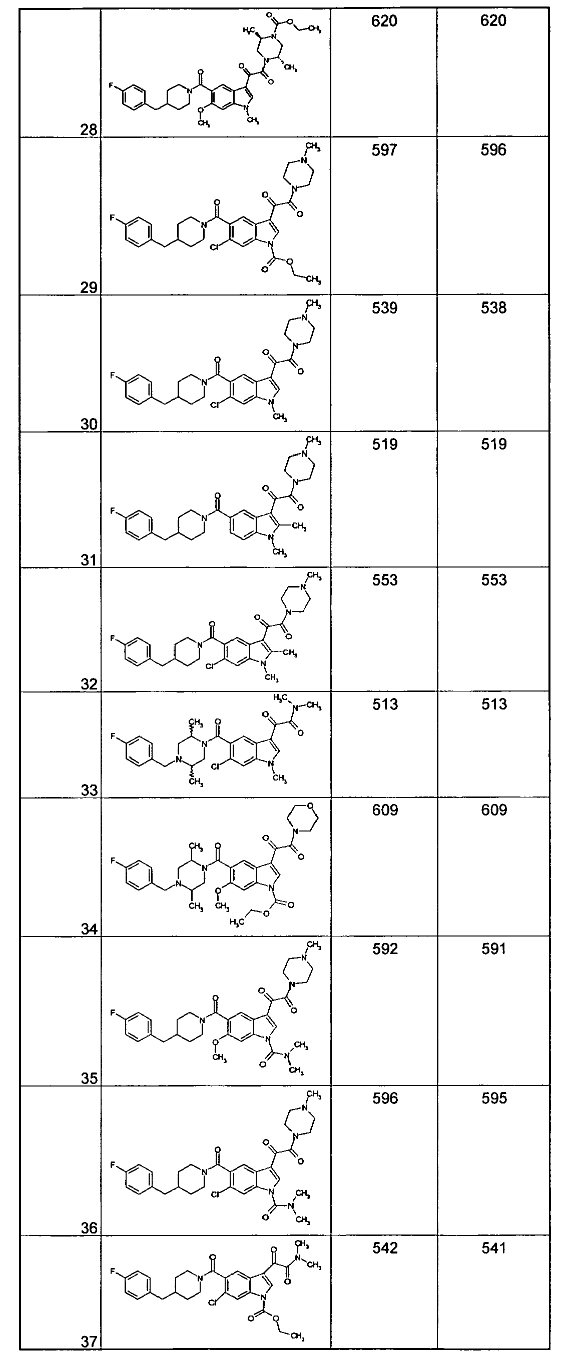

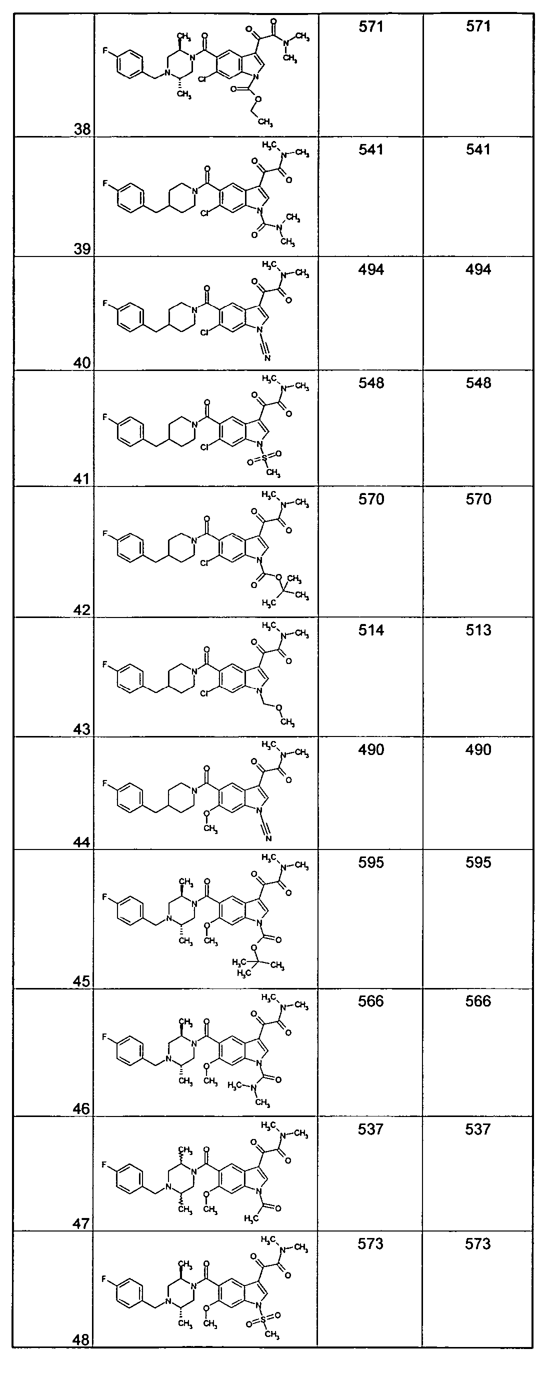

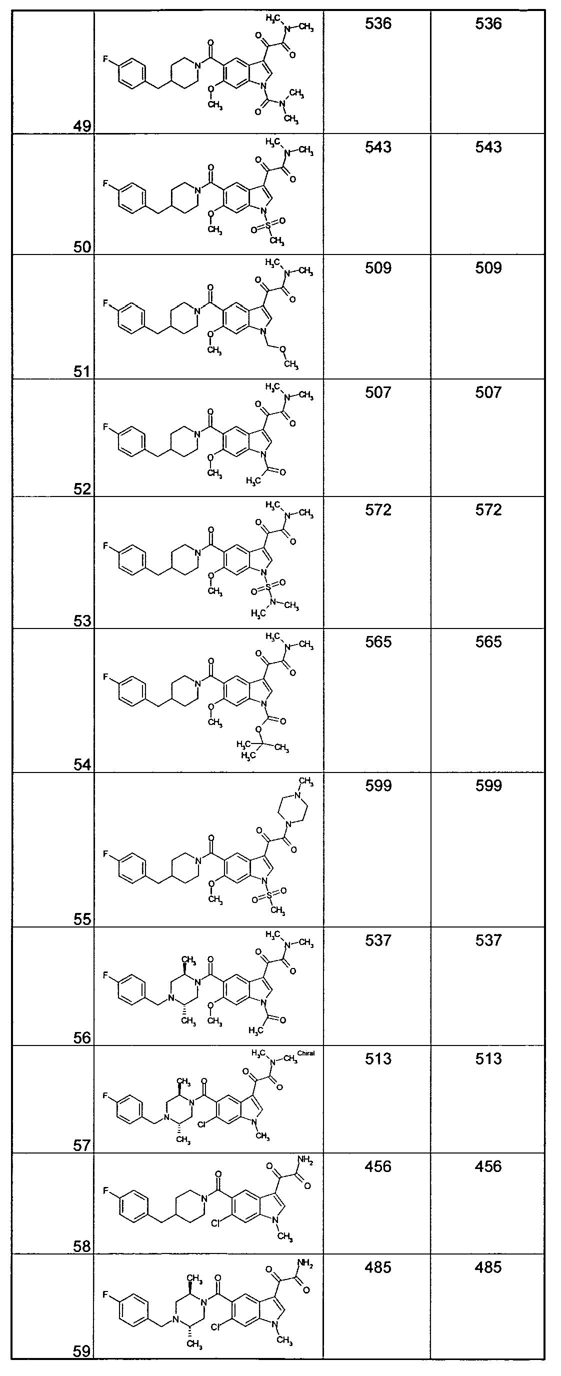

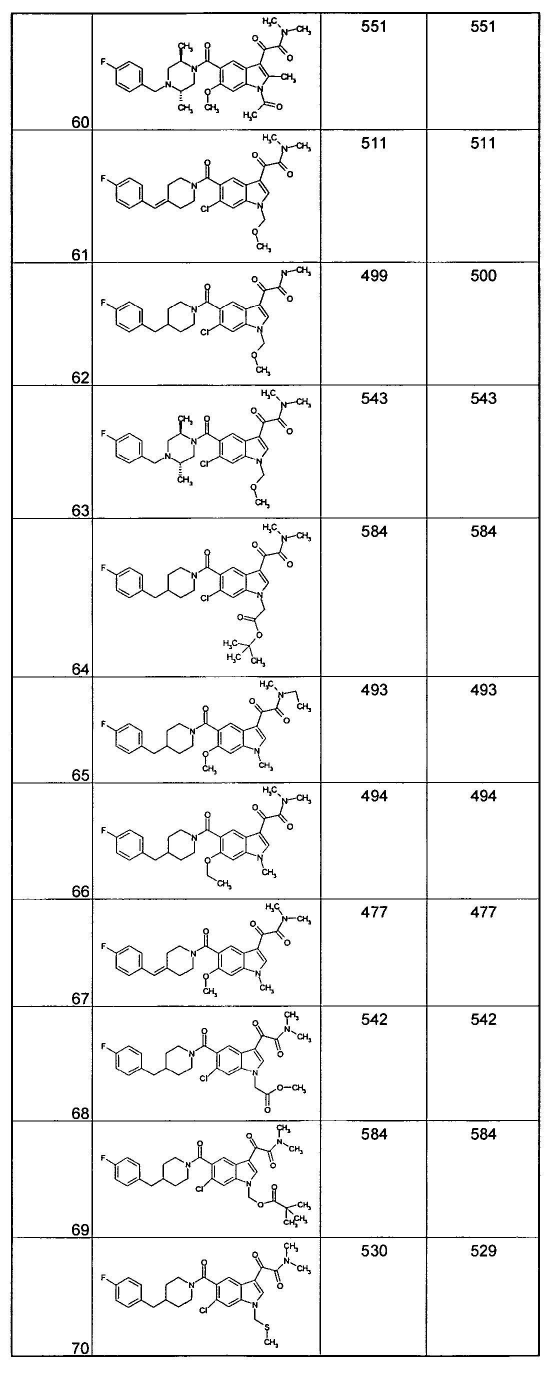

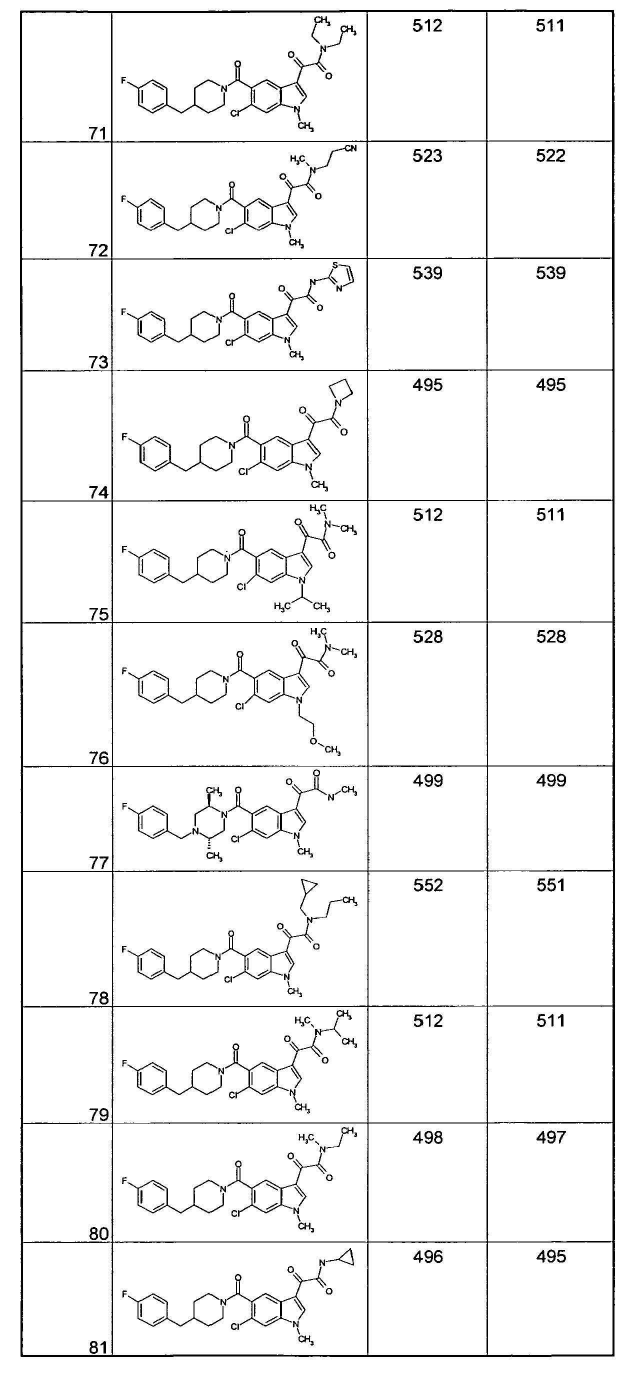

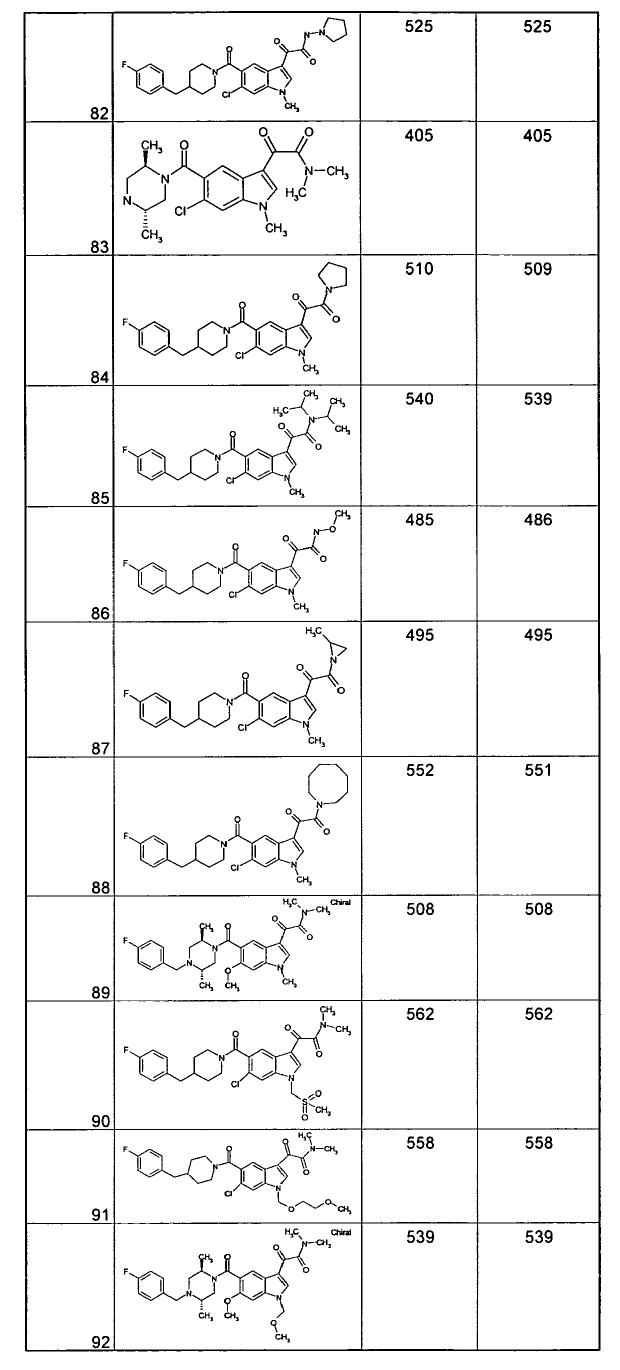

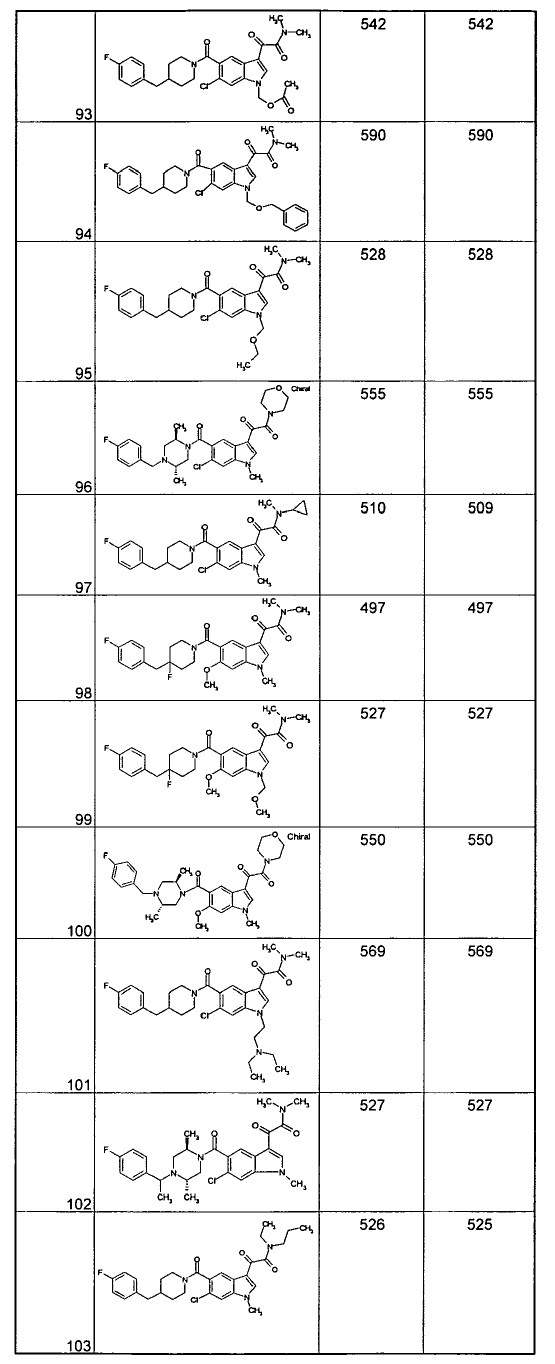

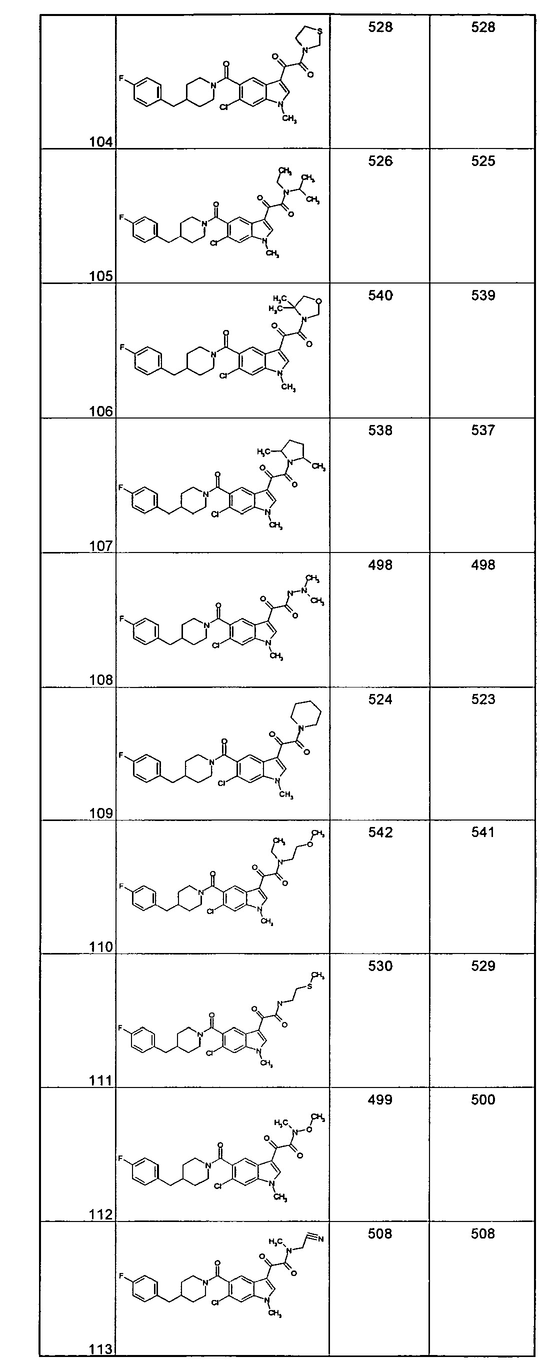

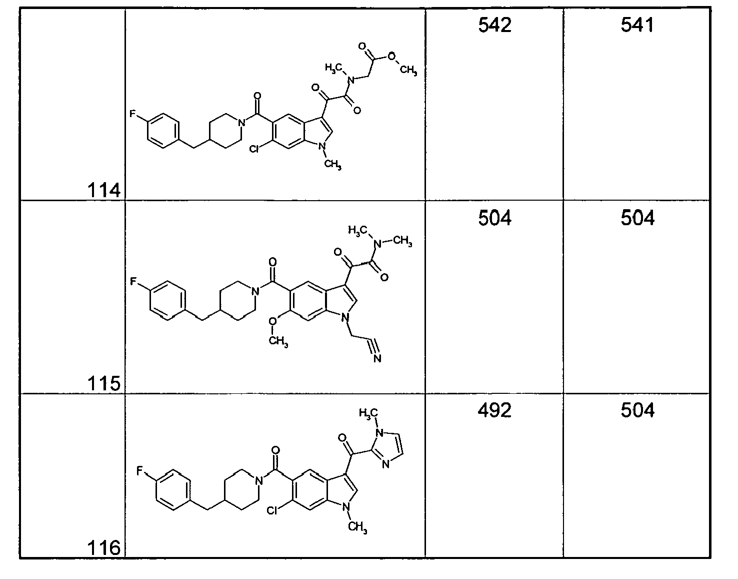

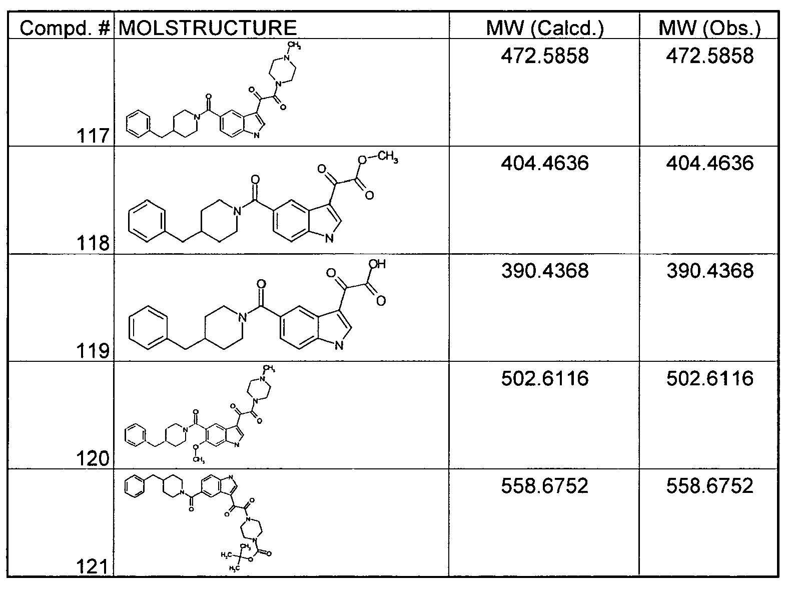

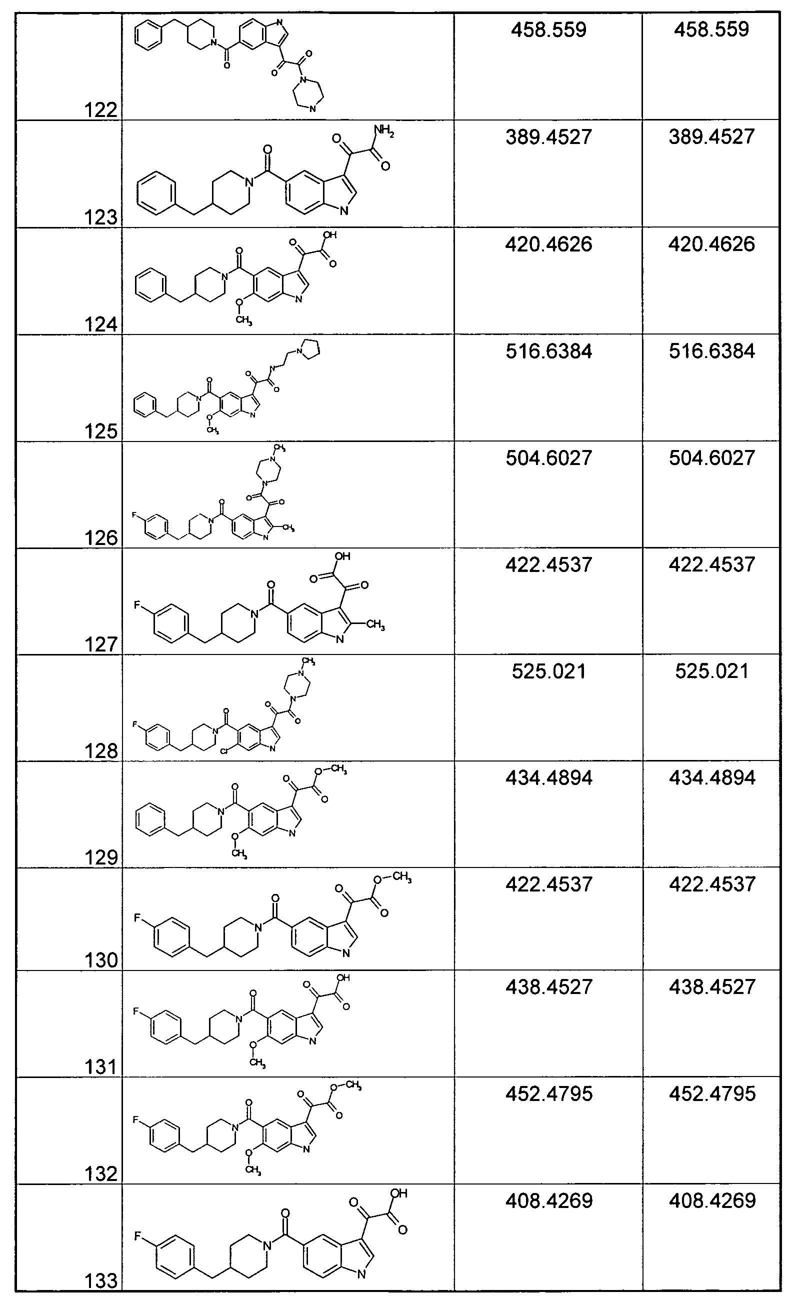

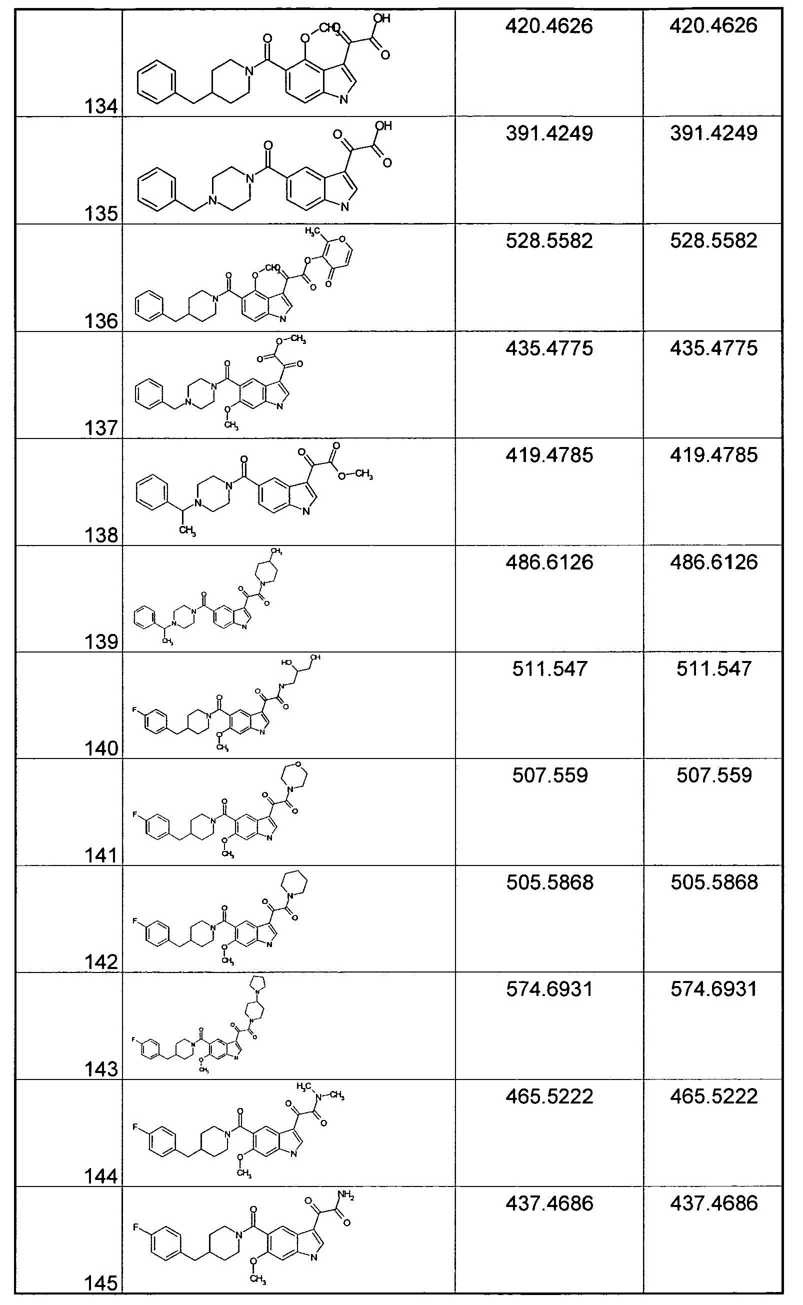

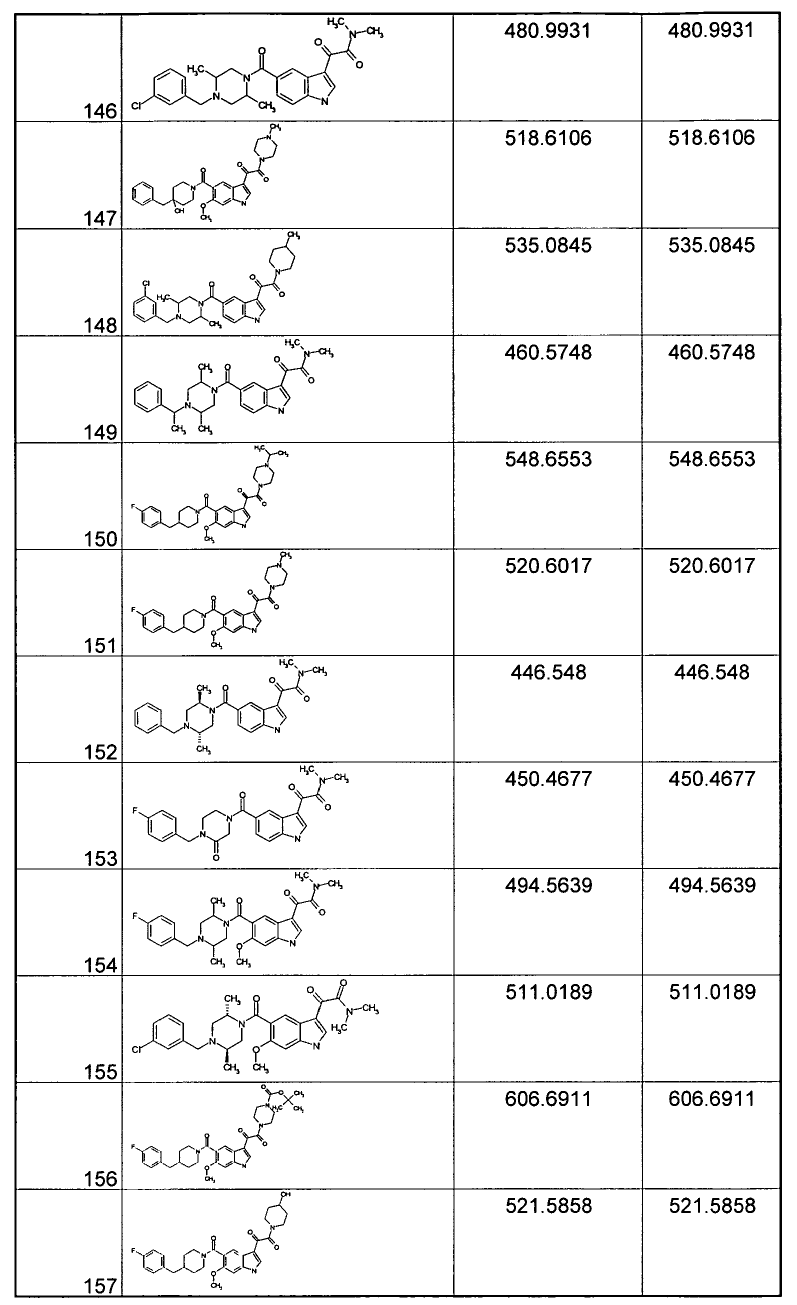

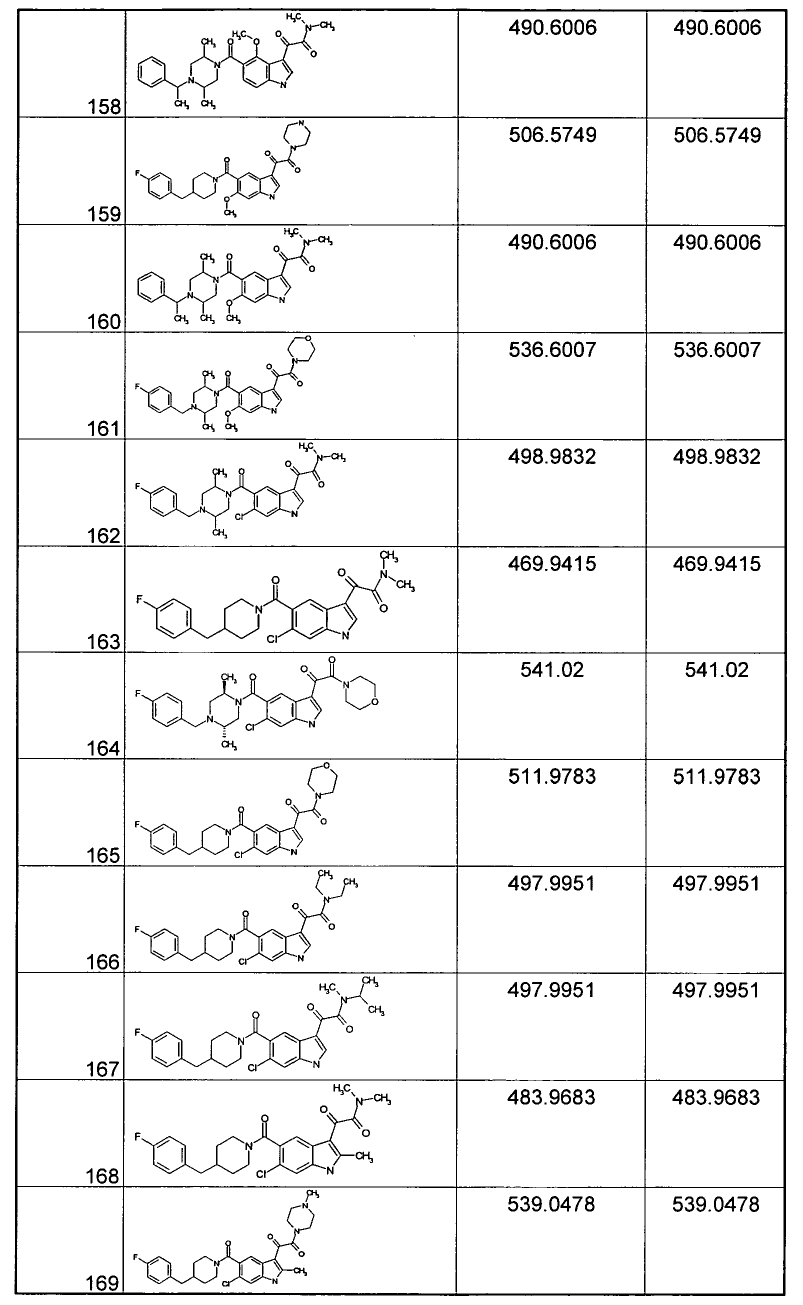

Compounds useful as p38 MAPK inhibitors also include, but are not limited to, compounds of the following formulas: 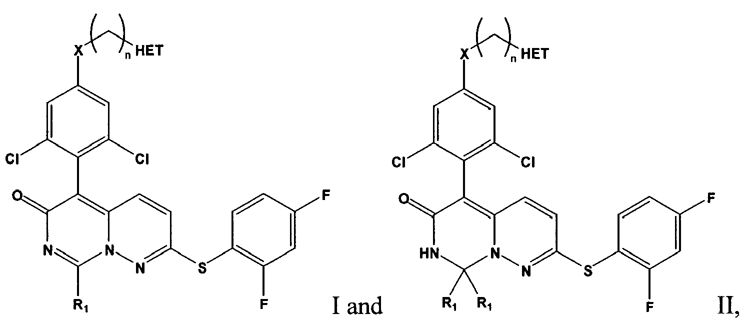 or pharmaceutically acceptable salts thereof, wherein
HET is a 5-7 membered heterocycle with 1 to 4 N, S or O atoms, which heterocycle is substituted with 1 to 3 C₁-C₄ branched or straight chain alkyl groups. HET can optionally be substituted with halo, cyano, N(R')₂, OR', CO₂R', CON(R')₂, and SO₂N(R²)₂;
X is O or NR';
n is 1 to 3;
R' is selected from hydrogen, (C₁-C₃)-alkyl, (C₂-C₃)-alkenyl or alkynyl, phenyl or phenyl substituted with 1 to 3 substituents independently selected from halo, methoxy, cyano, nitro, amino, hydroxy, methyl or ethyl; or a 5-6 membered heterocyclic ring system optionally substituted with 1 to 3 substituents independently selected from halo, methoxy, cyano, nitro, amino, hydroxy, methyl or ethyl;
R₁ is selected from hydrogen, (C₁-C₃)-alkyl, hydroxy, or (C₁-C₃)-alkoxy;
R₂ is selected from hydrogen, (C₁-C₃)-alkyl, or (C₁-C₃)-alkenyloxy; each optionally substituted with -N(R')₂, -OR', -SR', -C(O)-N(R')₂, -S(O₂)-N(R')₂, -C(O)-OR', or R³; and
R³ is selected from 5-6 membered aromatic carbocyclic or heterocyclic ring systems.

Compounds useful as p38 MAPK inhibitors also include, but are not limited to, compounds of the following formulas: 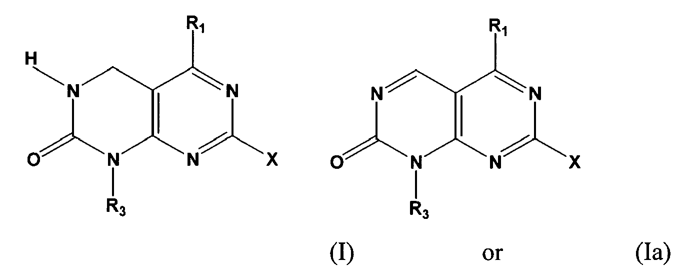 wherein
R₁ is an aryl or heteroaryl ring, which ring is optionally substituted;
R₂ is hydrogen, C₁₋₁₀ alkyl, C₃₋₇ cycloalkyl, C₃₋₇ cycloalkylC₁₋₁₀ alkyl, aryl, arylC₁₋₁₀ alkyl, heteroaryl, heteroarylC₁₋₁₀ alkyl, heterocyclic, or a heterocyclylC₁₋₁₀ alkyl moiety; and
wherein each of these moieties, excluding hydrogen, are optionally substituted;
R₃ is a C₁₋₁₀ alkyl, C₃₋₇cycloalkyl, C₃₋₇cycloalkylC₁₋₁₀alkyl, arylC₁₋₁₀alkyl, heteroaryl C₁₋₁₀alkyl,or heterocyclylC₁₋₁₀ alkyl moiety; and wherein each of these moieties are optionally substituted;
X is R₂, OR₂, S(O)ₘR₂ or (CH₂)ₙNR₄R₁₄, or (CH₂)ₙNR₂R₄;
n is 0 or an integer having a value of 1 to 10;
m is 0 or an integer having a value of 1 or 2;
R₄ and R₁₄ are each independently selected from hydrogen, optionally substituted C₁₋₁₄ alkyl, optionally substituted aryl, or an optionally substituted arylC₁₋₄alkyl, or R₄ and R₁₄ together with the nitrogen to which they are attached form a heterocyclic ring of 5 to 7 members, which ring optionally contains an additional heteroatom selected from oxygen, sulfur or NR₉, and which ring can be optionally substituted;
R₆ is hydrogen, C₁₋₁₀ alkyl, C₃₋₇ cycloalkyl, heterocyclyl, heterocyclylC₁₋₁₀alkyl, aryl, arylC₁₋₁₀ alkyl, heteroaryl or a heteroarylC₁₋₁₀ alkyl moiety; and wherein each of these moieties, excluding hydrogen, can be optionally substituted;
R₉ is hydrogen, C(Z)R₆, optionally substituted C₁₋₁₀ alkyl, optionally substituted aryl or optionally substituted arylC₁₋₄ alkyl;
Z is oxygen or sulfur;
or a pharmaceutically acceptable salt thereof.

Compounds useful as p38 MAPK inhibitors also include, but are not limited to, compounds of the following formulas: 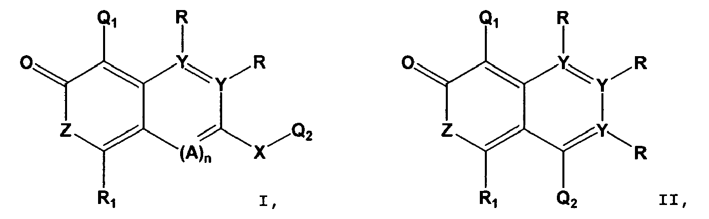 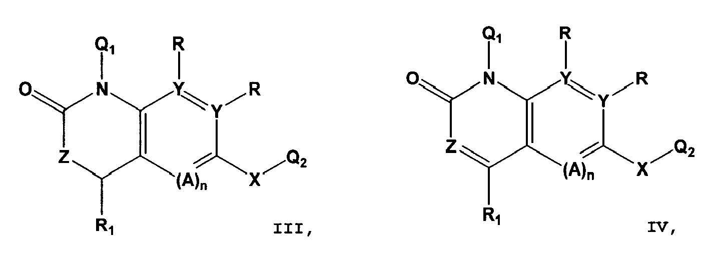 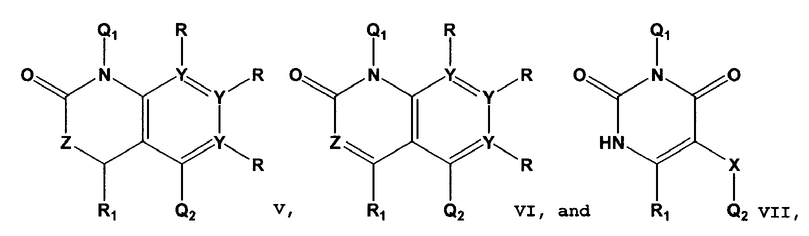 or pharmaceutically acceptable salts thereof, wherein
each of Q₁ and Q₂ are independently selected from 5-6 membered aromatic carbocyclic or heterocyclic ring systems, or 8-10 membered bicyclic ring systems comprising aromatic carbocyclic rings, aromatic heterocyclic rings or a combination of an aromatic carbocyclic ring and an aromatic heterocyclic ring;
the rings that make up Q₁ are substituted with 1 to 4 substituents, each of which is independently selected from halo; C₁-C₃ alkyl optionally substituted with NR'₂, OR', CO₂R' or CONR'₂; (C₁-C₃)-alkoxy optionally substituted with NR'₂, OR', CO₂R' or CONR'₂; NR'₂; OCF₃; CF₃; NO₂; CO₂R'; CONR' ; SR' ; S(O₂)N(R')₂; SCF₃; CN; N(R')C(O)R⁴; N(R')C(O)OR⁴; N(R')C(O)C(O)R⁴; N(R')S(O₂)R⁴; N(R')R⁴; N(R⁴)₂; OR⁴; OC(O)R⁴; OP(O)₃H₂; or N=C-N (R')₂;
the rings that make up Q₂ are optionally substituted with up to 4 substituents, each of which is independently selected from halo; C₁-C₃ straight or branched alkyl optionally substituted with NR'₂, OR', CO₂R', S(O₂)N(R')₂, N=C-N(R')₂, R³, or CONR'₂; (C₁-C₃)-alkoxy optionally substituted with NR'₂, OR', CO₂R', S(O₂)N(R')₂, N=C-N(R')₂, R³, or CONR'₂; NR'₂, OCF₃; CF₃; NO₂; CO₂R'; CONR'; R³; OR³; NR³; SR³; C(O)R³; C(O)N(R')R³; C(O)OR³; SR'; S(O₂)N(R')₂; SCF₃; N=C-N(R')₂; or CN;
R' is selected from hydrogen, (C₁-C₃)-alkyl; (C₂-C₃)-alkenyl; (C₂-C₃) alkynyl; phenyl substituted with 1 to 3 substituents independently selected from halo, methoxy, cyano, nitro, amino, hydroxy, methyl or ethyl;
R³ is selected from 5-6 membered aromatic carbocyclic or heterocyclic ring systems;
R⁴ is (C₁-C₄)-alkyl optionally substituted with N(R')₂, OR', CO₂R', CON(R')₂, or SO₂N(R²)₂; or a 5-6 membered carbocyclic or heterocyclic ring system optionally substituted with N(R')₂, OR', CO₂R', CON(R')₂, or SO₂N(R²)₂;
X, if present, is selected from -S-, -O-, -S(O₂)-, -S(O)-, -S(O₂)-N(R²)-, -N(R²)-S(O₂)-, -N(R²)-C(O)O-, -O-C(O)-N(R²), -C(O)-, -C(O)O-, -O-C(O)-, -C(O)-N(R²)-, -N(R²)-C(O)-, -N(R²)-, -C(R²)₂-, or -C(OR²)₂-;
each R is independently selected from hydrogen, -R², -N(R²)₂, -OR², SR², -C(O)-N(R²)₂, -S(O₂)-N(R²)₂, or -C(O)-OR², wherein two adjacent R are optionally bound to one another and, together with each Y to which they are respectively bound, form a 4-8 membered carbocyclic or heterocyclic ring;
R² is selected from hydrogen, (C₁-C₃)-alkyl, or (C₁-C₃)-alkenyl; each optionally substituted with -N(R')₂, -OR', SR', -C(O)-N(R')₂, -S(O₂)-N(R')₂, -C(O)-OR', or R³;
Y is N or C;
Z, if present, is N, NH, or, if chemically feasible, O;
A, if present, is N or CR';
n is 0 or 1; and
R₁ is selected from hydrogen, (C₁-C₃)-alkyl, hydroxy, or (C₁-C₃)-alkoxy.

Compounds useful as p38 MAPK inhibitors also include, but are not limited to, compounds of the following formula: 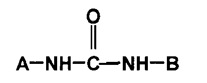 wherein A is 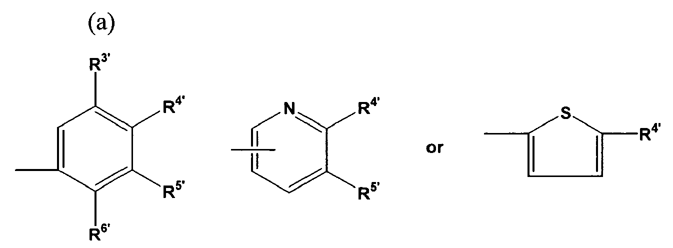 wherein
R^{3'}, R^{4'}, R^{5'} are each independently HOURS, C₁₋₁₀-alkyl, optionally substituted by halogen up to perhalo, C₁₋₁₀ alkoxy, optionally substituted by halogen, up to perhaloalkoxy, halogen; NO₂ or NH₂;
R^{6'} is HOURS, C₁₋₁₀-alkyl, C₁₋₁₀ alkoxy, -NHCOR¹; -NR¹COR¹; NO₂; 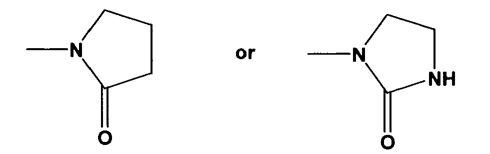 one of R^{4'}, R^{5'}, or R^{6'} can be -X-Y; or
2 adjacent R^{4'}-R^{6'} can together be an aryl or heteroaryl ring with 5-12 atoms, optionally substituted by C₁₋₁₀-alkyl, C₁₋₁₀ alkoxy, C₃₋₁₀ cycloalkyl, C₂₋₁₀ alkenyl, C₁₋₁₀ alkanoyl, C₆₋₁₂ aryl, C₅₋₁₂ heteroaryl or C₆₋₁₂ arakyl;
R¹ is C₁₋₁₀-alkyl optionally substituted by halogen, up to perhalo;
X is -CH₂-, -S-, -N(CH₃)-, -NHC(O)-, -CH₂-S-, -S-CH₂-, -C(O)-, or -O-;
X is additionally a single bond where Y is pyridyl;
Y is phenyl, pyridyl, naphthyl, pyridone, pyrazine, benzodioxane, benzopyridine, pyrimidine or benzothiazole, each optionally substituted by C₁₋₁₀-alkyl, C₁₋₁₀-alkoxy, halogen, OH, -SCH₃ or NO₂ or, where Y is phenyl, by 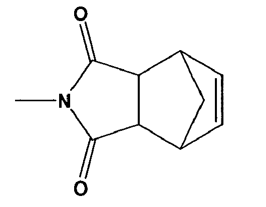 or a pharmaceutically-acceptable salt thereof;
or 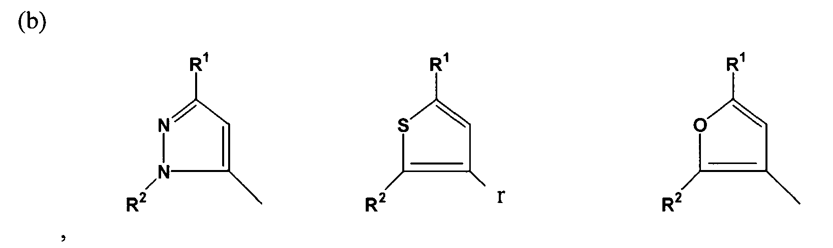 wherein
R¹ is selected from the group consisting of C₃-C₁₀ alkyl, C₃-C₁₀ cycloalkyl, up to per-halo substituted C₁-C₁₀ alkyl and up to per- halosubstituted C₃-C₁₀ cycloalkyl; and
R² is C₆-C₁₄ aryl, C₃-C₁₄ heteroaryl, substituted C₆-C₁₄ aryl or substituted C₃-C₁₄ heteroaryl;
wherein if R² is a substituted group, it is preferably substituted by one or more substituents independently selected from the group consisting of halogen, up to perhalosubstitution, and Vₙ, where n = 0-3 and each V is independently selected from the group consisting of -CN, -OC(O)NR⁵R^{5'}, -CO₂R⁵, -C(O)NR⁵R^{5'}, -OR⁵, -SR⁵, -NR⁵R^{5'}, -C(O)R⁵, -NR⁵C(O)OR^{5'}, -SO₂R⁵ -SOR⁵, -NR⁵C(O)R^{5'}, -NO₂, C₁-C₁₀ alkyl, C₃-C₁₀ cycloalkyl, C₆-C₁₄ aryl, C₃-C₁₃ heteroaryl, C₇-C₂₄ alkaryl, C₄-C₂₄ alkheteroaryl, substituted C₁-C₁₀ alkyl, substituted C₃₋C₁₀ cycloalkyl, substituted C₆-C₁₄ aryl, substituted C₃-C₁₃ heteroaryl, substituted C₇-C₂₄ alkaryl and substituted C₄-C₂₄ alkheteroaryl;
wherein if V is a substituted group, it is substituted by one or more substituents independently selected from the group consisting of halogen, up to per- halosubstitution, -CN, -CO₂R⁵, -C(O)R⁵, -C(O)NR⁵R^{5'}, -NR⁵R^{5'}, -OR⁵, -SR⁵, - NR⁵C(O)R^{5'}, -NR⁵C(O)OR^{5'} and -NO₂; and
R⁵ and R^{5'} are independently selected form the group consisting of HOURS, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per- halosubstituted C₃-C₁₀ cycloalkyl, up to per-halosubstituted C₆-C₁₄ aryl and up to per- halosubstituted C₃-C₁₃ heteroaryl; or a pharmaceutically-acceptable salt thereof; or
(c) a substituted moiety of up to 40 carbon atoms of the formula: -L-(M-L¹)_{q}, where L is a 5- or 6-membered cyclic structure bound directly to D, L¹, comprises a substituted cyclic moiety having at least 5 members, M is a bridging group having at least one atom, q is an integer of from 1-3; and each cyclic structure of L and L¹ contains 0-4 members of the group consisting of nitrogen, oxygen and sulfur;
L¹ is substituted by at least one substituent selected from the group consisting of -SO₂Rₓ, -C(O)Rₓ and -C(NR_{y})R_{z};
Ry is hydrogen or a carbon-based moiety of up to 24 carbon atoms optionally containing heteroatoms selected from N, S and O and optionally halosubstituted, up to perhalo;
R_{z} is hydrogen or a carbon-based moiety of up to 30 carbon atoms optionally containing heteroatoms selected from N, S and O and optionally substituted by halogen, hydroxy and carbon-based substituents of up to 24 carbon atoms, which optionally contain heteroatoms selected from N, S and O and are optionally substituted by halogen; and
Rₓ is R_{z} or NRₐR_{b} where Rₐ and R_{b} are
i) independently hydrogen,
   a carbon-based moiety of up to 30 carbon atoms optionally containing heteroatoms selected from N, S and O and optionally substituted by halogen, hydroxy and carbon-based substituents of up to 24 carbon atoms, which optionally contain heteroatoms selected from N, S and O and are optionally substituted by halogen, or
   -OSi(R_{f})₃ where R_{f} is hydrogen or a carbon-based moiety of up to 24 carbon atoms optionally containing heteroatoms selected from N, S and O and optionally substituted by halogen, hydroxy and carbon-based substituents of up to 24 carbon atoms, which optionally contain heteroatoms selected from N, S and O and are optionally substituted by halogen; or
ii) Rₐ and R_{b} together form a 5-7 member heterocyclic structure of 1-3 heteroatoms selected from N, S and O, or a substituted 5-7 member heterocyclic structure of 1-3 heteroatoms selected from N, S and O, substituted by halogen, hydroxy or carbon-based substituents of up to 24 carbon atoms, which optionally contain heteroatoms selected from N, S and O and are optionally substituted by halogen; or
iii) one of Rₐ or R_{b} is -C(O)-, a C₁-C₅ divalent alkylene group or a substituted C₁-C₅ divalent alkylene group bound to the moiety L to form a cyclic structure with at least 5 members, wherein the substituents of the substituted C₁-C₅ divalent alkylene group are selected from the group consisting of halogen, hydroxy, and carbon-based substituents of up to 24 carbon atoms, which optionally contain heteroatoms selected from N, S and O and are optionally substituted by halogen;
or a pharmaceutically-acceptable salt thereof; and
B is an unsubstituted or substituted, up to tricyclic, aryl or heteroaryl moiety with up to 30 carbon atoms with at least one 5- or 6-membered aromatic structure containing 0-4 members of the group consisting of nitrogen, oxygen and sulfur;
wherein if B is substituted, it is substituted by one or more substituents selected from the group consisting of halogen, up to per-halo, and Wₙ, wherein
n is 0-3 and each W is independently selected from the group consisting of
-CN, -CO₂R⁷, -C(O)NR⁷R⁷, -C(O)R⁷, -NO₂, -OR⁷, -SR⁷, -NR⁷R⁷,
-NR⁷C(O)OR⁷, -NR⁷C(O)R⁷, C₁-C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂₋₁₀-alkenyl, substituted C₁-₁₀- alkoxy, substituted C₃-C₁₀ cycloalkyl, substituted C₄-C₂₃ alkheteroaryl and -Q-Ar;
wherein if W is a substituted group, it is substituted by one or more substituents independently selected from the group consisting of -CN, -CO₂R⁷,
-C(O)NR⁷R⁷, -C(O)R⁷, -NO₂, -OR⁷, -SR⁷, -NR⁷R⁷, -NR⁷C(O)OR⁷,
-NR⁷C(O)R⁷ and halogen up to per-halo;
wherein each R⁷ is independently selected from HOURS, C₁- C₁₀ alkyl, C₂-₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per- halosubstituted C₂-₁₀-alkenyl, up to per-halosubstituted C₃-C₁₀ cycloalkyl, up to per- halosubstituted C₆-C₁₄ aryl and up to per-halosubstituted C₃-C₁₃ heteroaryl;
wherein Q is -O-, -S-, -N(R)⁷, -(CH₂)-ₘ, -C(O)-, -CH(OH)-,
-NR⁷C(O)NR⁷R⁷-, -NR⁷C(O)-, -C(O)NR⁷-, -(CH₂)ₘO-, -(CH₂)ₘS-,
-(CH₂)ₘN(R⁷)-, -O(CH₂)ₘ-, -CHX^{a}, -CX^{a}₂-, -S-(CH₂)ₘ- and -N(R⁷)(CH₂)ₘ-, where m = 1-3, and X^{a} is halogen; and
Ar is a 5-10 member aromatic structure containing 0-4 members of the group consisting of nitrogen, oxygen and sulfur, which is unsubstituted or substituted by halogen up to perhalosubstitution and optionally substituted by Zₙₗ, wherein nl is 0 to 3 and each Z substituent is independently selected from the group consisting of -CN, -CO₂R⁷, -C(O)NR⁷R⁷, -C(O)- NR⁷, -NO₂, -OR⁷, -SR⁷, -NR⁷R⁷, -NR⁷C(O)OR⁷, -C(O)R⁷, -NR⁷C(O)R⁷, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl; wherein the one or more substituents of Z are independently selected from the group consisting of -CN, -CO₂R⁷, -C(O)NR⁷R⁷, -OR⁷, -SR⁷, -N02, -NR⁷R⁷,
-NR⁷C(O)R⁷ and -NR⁷C(O)OR⁷; or a pharmaceutically-acceptable salt thereof.

Exemplary compounds of these formulas include, but are not limited to:
N-(5-*tert*-butyl-2-methoxyphenyl)-N'-(4-phenyloxyphenyl)urea;
N-(5-*tert*-butyl-2-methoxyphenyl)-N'-(4-(4-methoxyphenyloxy)phenyl)urea; *N-(5-tert-*butyl-2-methoxyphenyl)-N'-(4-(4-pyridinyloxy)phenyl)urea;
N-(5-*tert*-butyl-2-methoxyphenyl)-N'-(4-(4-pyridinylmethyl)phenyl)urea;
N-(5-tert-butyl-2-methoxyphenyl)-N'-(4-(4-pyridinylthio)phenyl)urea;
N-(5-*tert*-butyl-2-methoxyphenyl)-N'-(4-(4-(4,7-methano-1*H*-isoindole-1, 3(2*H*)-dionyl)methyl)phenyl)urea;
N-(5-*tert*-butyl-2-phenylphenyl)-N'-(2,3-dichlorophenyl)urea;
N-(5-tert-butyl-2-(3-thienyl)phenyl)-N'-(2,3-dichlorophenyl)urea;
N-(5-*tert*-butyl-2-(N-methylaminocarbonyl)methoxyphenyl)-N'-(2,3-dichlorophenyl)urea;
N-(5-*tert*-butyl-2-(N-methylaminocarbonyl)methoxyphenyl)-N'-(1- naphthyl)urea;
N-(5-tert-butyl-2-(N-morpholinocarbonyl)methoxyphenyl)-N'-(2,3- dichlorophenyl)urea;
N-(5-*tert*-butyl-2-(N-morpholinocarbonyl)methoxyphenyl)-N'-(1-naphthyl)urea;
N-(5-*tert*-butyl-2-(3-tetrahydrofuranyloxy)phenyl)-N'-(2,3- dichlorophenyl)urea;
N-(5-tert-butyl-2-methoxyphenyl)-N'-(4-(3-pyridinyl)methylphenyl)urea;
N-(5-trifluoromethyl-2-methoxyphenyl)-N'-(4-methylphenyl)urea;
N-(5-trifluoromethyl-2-methoxyphenyl)-N'-(4-methyl-2-fluorophenyl)urea;
N-(5-trifluoromethyl-2-methoxyphenyl)-N'-(4-fluoro-3-chlorophenyl)urea;
N-(5-trifluoromethyl-2-methoxyphenyl)-N'-(4-methyl-3-chlorophenyl)urea;
N-(5-trifluoromethyl-2-methoxyphenyl)-N'-(4-methyl-3-fluorophenyl)urea;
N-(5-trifluoromethyl-2-methoxyphenyl)-N'-(2,4-difluorophenyl)urea;
N-(5-trifluoromethyl-2-methoxyphenyl)-N'-(4-phenyloxy-3,5- dichlorophenyl)urea;
N-(5-trifluoromethyl-2-methoxyphenyl)-N'-(4-(4- pyridinylmethyl)phenyl)urea;
N-(5-trifluoromethyl-2-methoxyphenyl)-N'-(4-(4-pyridinylthio)phenyl)urea;
N-(5-trifluoromethyl-2-methoxyphenyl)-N'-(4-(4-pyridinyloxy)phenyl)urea;
N-(5-trifluoromethyl-2-methoxyphenyl)-N'-(3-(4-pyridinylthio)phenyl)urea;
N-(5-trifluoromethyl-2-methoxyphenyl)-N'-(4-(3-(N-methylaminocarbonyl)phenyloxy)phenyl)urea;
N-(5-fluorosulfonyl)-2-methoxyphenyl)-N'-(4-methylphenyl)urea;
N-(5-(difluromethanesulfonyl)-2-methoxyphenyl)-N'-(4-methylphenyl)urea;
N-(5-(difluoromethanesulfonyl)-2-methoxyphenyl)-N'-(4-fluorophenyl)urea;
N-(5-(difluoromethanesulfonyl)-2-methoxyphenyl)-N'-(4-methyl-2- fluorophenyl)urea;
N-(5-(difluoromethanesulfonyl)-2-methoxyphenyl)-N'-(4-methyl-3- fluorophenyl)urea;
N-(5-(difluoromethanesulfonyl)-2-methoxyphenyl)-N'-(4-methyl-3- chlorophenyl)urea;
N-(5-(difluoromethanesulfonyl)-2-methoxyphenyl)-N'-(4-fluoro-3- chlorophenyl)urea;
N-(5-(difluoromethanesulfonyl)-2-methoxyphenyl)-N'-(4-fluoro-3- methylphenyl)urea;
N-(5-(difluoromethanesulfonyl)-2-methoxyphenyl)-N'-(2,3- dimethylphenyl)urea;
N-(5-(trifluoromethanesulfonyl)-2-methoxphenyl)-N'-(4-methylphenyl)urea;
N-(3-methoxy-2-naphthyl)-N'-(2-fluorophenyl)urea;
N-(3-methoxy-2-naphthyl)-N'-(4-methylphenyl)urea;
N-(3-methoxy-2-naphthyl)-N'-(3-fluorophenyl)urea;
N-(3-methoxy-2-naphthyl)-N'-(4-methyl-3-fluorophenyl)urea;
N-(3-methoxy-2-naphthyl)-N'-(2,3-dimethylphenyl)urea;
N-(3-methoxy-2-naphthyl)-N'-(1-naphthyl)urea;
N-(3-methoxy-2-naphthyl)-N'-(4-(4-pyridinylmethyl)phenyl)urea;
N-(3-methoxy-2-naphthyl)-N'-(4-(4-pyridinylthio)phenyl)urea;
N-(3-methoxy-2-naphthyl)-N'-(4-(4-methoxyphenyloxy)phenyl)urea;
N-(3-methoxy-2-naphthyl)-N'-(4-(4-(4,7-methano-1*H*-isoindole-1,3(2*H*)-dionyl)methyl)phenyl)urea;
N-(2-hydroxy-4-nitro-5-chlorophenyl)-N'-(phenyl)urea;
N-(2-hydroxy-4-nitro-5-chlorophenyl)-N'-(4-(4-pyridinylmethyl)phenyl)urea;
and pharmaceutically acceptable salts thereof.

Such compounds are described in published PCT applications WO 96/21452, WO 96/40143, WO 97/25046, WO 97/35856, WO 98/25619, WO 98/56377, WO 98/57966, WO 99/32110, WO 99/32121, WO 99/32463, WO 99/61440, WO 99/64400, WO 00/10563, WO 00/17204, WO 00/19824, WO 00/41698, WO 00/64422, WO 00/71535, WO 01/38324, WO 01/64679, WO 01/66539, and WO 01/66540, each of which is herein incorporated by reference in its entirety.

In all instances herein where there is an alkenyl or alkynyl moiety as a substituent group, the unsaturated linkage, i.e., the vinylene or acetylene linkage, is preferably not directly attached to the nitrogen, oxygen or sulfur moieties, for instance in OR_{f}, or for certain R₂ moieties.

As used herein, "optionally substituted," unless specifically defined, shall mean such groups as halogen, such as fluorine, chlorine, bromine or iodine; hydroxy; hydroxy-substituted C₁₋₁₀alkyl; C₁₋₁₀ alkoxy, such as methoxy or ethoxy; S(O)ₘ alkyl, wherein m is 0, 1 or 2, such as methyl thio, methylsulfinyl or methyl sulfonyl; amino, mono and di-substituted amino, such as in the NR₇R₁₇ group; or where the R₇R₁₇ can together with the nitrogen to which they are attached cyclize to form a 5- to 7-membered ring which optionally includes an additional heteroatom selected from O,N, and S; C₁₋₁₀ alkyl, cycloalkyl, or cycloalkyl alkyl group, such as methyl, ethyl, propyl, isopropyl, t-butyl, etc. or cyclopropyl methyl; halo-substituted C₁₋₁₀ alkyl, such as CF₃; an optionally substituted aryl, such as phenyl, or an optionally substituted arylalkyl, such as benzyl or phenethyl, wherein these aryl moieties can also be substituted one to two times by halogen; hydroxy; hydroxy-substituted alkyl; C₁₋₁₀ alkoxy; S(O)ₘ alkyl; amino, mono- and di-substituted amino, such as in the NR₇R₁₇ group; alkyl, or CF₃.

The term "pharmaceutically acceptable salts" refers to salts prepared from pharmaceutically acceptable non-toxic bases or acids. When a compound utilized by the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Basic salts of inorganic and organic acids also include as hydrochloric acid, hydrobromic acid, sulphuric acid, phosphoric acid, methane sulphonic acid, ethane sulphonic acid, acetic acid, malic acid, tartaric acid, citric acid, lactic acid, oxalic acid, succinic acid, fumaric acid, maleic acid, benzoic acid, salicylic acid, phenylacetic acid and mandelic acid. In addition, pharmaceutically-acceptable salts of the above-described compounds can also be formed with a pharmaceutically-acceptable cation, for instance, if a substituent group comprises a carboxy moiety. Suitable pharmaceutically-acceptable cations are well known to those skilled in the art and include alkaline, alkaline earth, ammonium and quaternary ammonium cations.

Other pharmaceutically acceptable organic non-toxic bases from which salts can be formed include ion exchange resins such as, for example, arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other compounds useful as p38 MAPK inhibitors include, but are not limited to, the compounds shown in Table B, below.
TABLE B

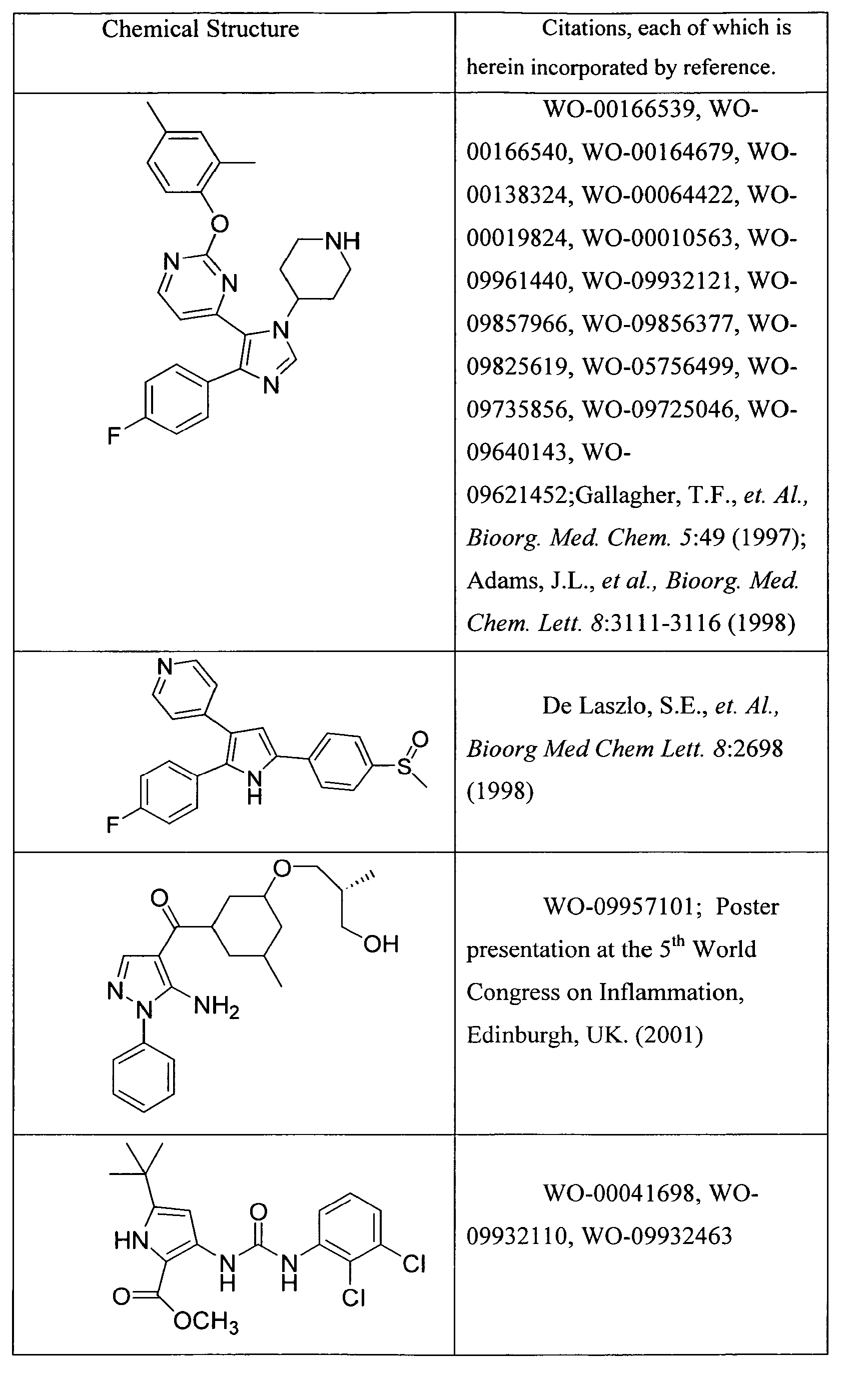

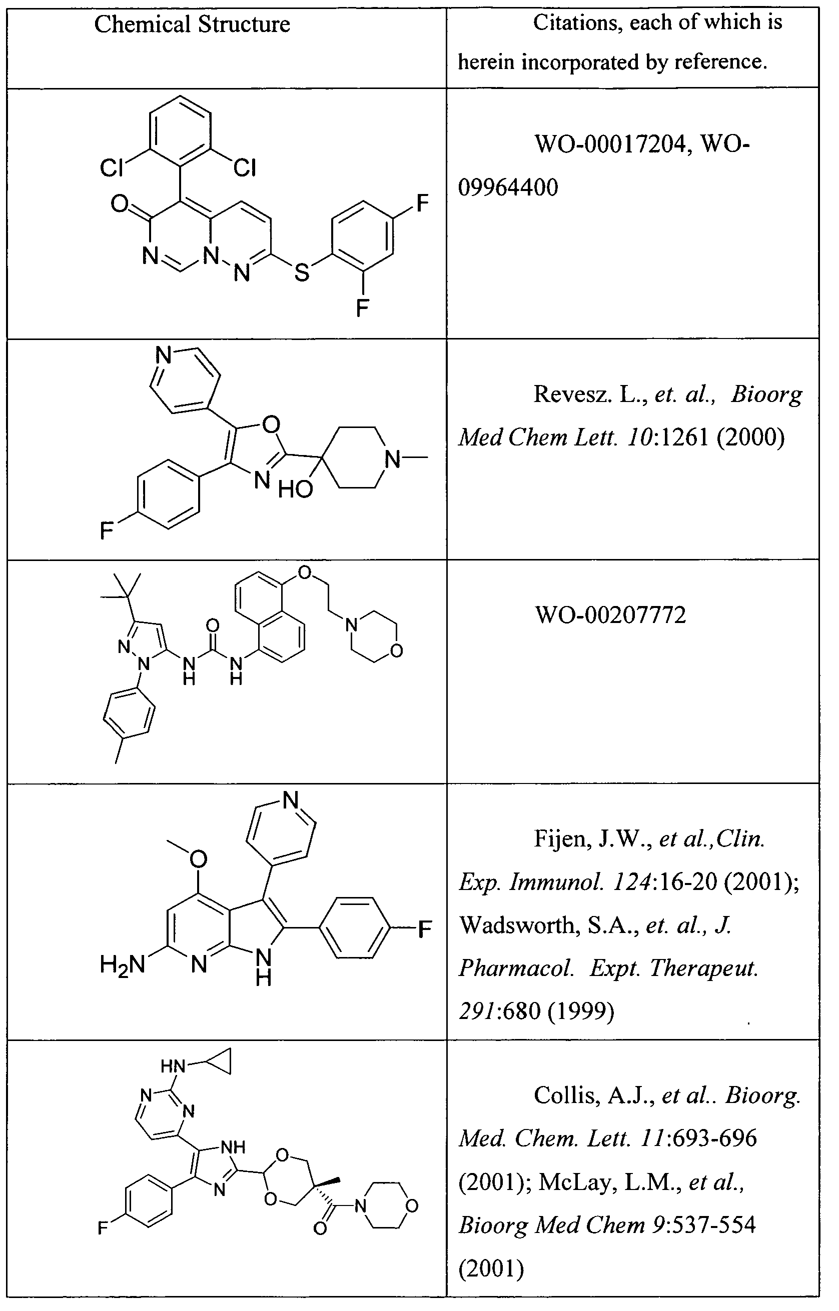

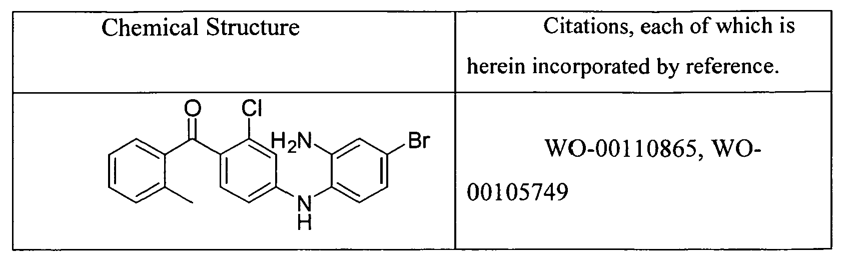

All compounds provided herein are intended for guidance and exemplary purposes only. It is understood that any inhibitor of p38 MAPK can be used in the methods of the invention.

Likewise, any JNK inhibitor, or any dual inhibitor of p38 MAPK and JNK, is suitable for use in the present invention. Suitable JNK inhibitors include, without limitation: anthrapyrazolones, such as disclosed by Bennett *et al*., 2001, *Proc. Natl. Acad. Sci. USA* 98: 13681-13686; (1,3-benzothiazol-2-yl (2-[[2-(3-pyridinyl) ethyl] amino]-4 pyrimidinyl) acetonitrile) and other (benzothiazol-2-yl) acetonitriles, disclosed by Carboni *et al.,* 2004, *J. Pharmacol. Exp. Therap.* 310(1), 25-32 and Carboni *et al.,* 2003, Abstract of Papers, 226^{th} ACS National Meeting, New York City Sept. 7-11, 2003; cell-permeable peptide inhibitors (U.S. Patent Appl. Pub. 2004/082509); compositions comprising (+)-3-(3,4-dimethoxyphenyl)-3-(1-oxo-1,3-dihydroisoindol-2-yl) propionamide (PCT Publication WO 04/045597); pyridinyl acetonitriles (PCT Publication WO 04/098607); various derivatives of 3-ethynyl or 3-cyano-1H-pyrrolo[2,3-b]pyridine (PCT Publication WO 04/101565); various derivatives of 6J-anthra[9,1-cd]isothiazol-6-one (U.S. Pat. App. Pub. 2004/092562); various benzimidazole derivatives (PCT Publications WO 04/099190 and WO 03/051277); various indazole derivatives (U.S. Pat. App. Pub. 2004/127536 and PCT Publications WO 04/040088, WO 03/068754 and WO 03072550); various isoquinolinone derivatives (PCT Publications WO 04/054979 and WO 03/068750); various pyridine derivatives (PCT Publication WO 04/042880); various sulfur heterocycle-condensed pyrimidinedione derivatives and their prodrugs (Japanese Patent 2004161716); cyanomethylidene-substituted azoles (PCT Publication WO 03/106455); various pyrazole derivatives (PCT Publication WO 03/101968), thienylmethylbenzamides (PCT Publication WO 03/010164); JNK inhibitors as disclosed in PCT Publications WO 03/075917, WO 03/108020, WO 03/099221, and others, as reviewed by Barr, R. *et al.,* 2002, *J Biol. Chem*. 277: 10987-10997 and Harper *et al.,* 2001, Drugs of the Future 26: 956-973.

Certain embodiments of the invention relate to methods of enhancing the survival of a cell, tissue, or organ graft that has been implanted into a patient that involve contacting the cell, tissue, or organ graft with a TF antagonist, in addition to a p38 MAPK and/or JNK inhibitor, as previously discussed. In preferred embodiments, the cell, tissue, or organ graft is contacted with the TF antagonist prior to implantation into the patient. In other embodiments of the invention, a TF antagonist is administered to the patient prior to implantation of the cell, tissue, or organ graft; following implantation of the cell, tissue, or organ graft; or both prior to and following implantation of the cell, tissue, or organ graft. In certain embodiments of the invention, the TF antagonist is administered to the patient during implantation of the cell, tissue, or organ graft.

A number of pathologies involving complications arising from the activation of TF on the surface of transplanted cells, tissues, or in the vasculature of transplanted organs are improved by treatment with TF antagonists according to the methods of the present invention. While the events leading to the activation of TF in the cellular components of living materials prepared for implantation into patients are largely unavoidable, blocking the functions of TF in inducing coagulation are possible using TF antagonists. Thus, while events that lead to the activation of TF, such as mechanical trauma, exposure to unnatural surfaces on the vessels and implements used, and the release of cellular contents including Ca²⁺, cannot be avoided in transplantation, the resulting coagulation can be significantly reduced or blocked with TF antagonists according to the methods of the present invention.

TF antagonists act in a variety of ways. One class of TF antagonists binds to TF protein with sufficient affinity and specificity to neutralize the function of TF. Included in this class of molecules are antibodies, antibody fragments such as, for example, F(ab) or F(ab')₂ molecules, and mimetobodies. Specific anti-TF antibodies include, for example, the murine monocolonal antibodies directed against TF described in US patents 6,001,978; 5,223,427; and 5,110,730. Further examples of anti-TF antibodies include CDR-grafted anti-TF antibodies derived from the TF8-5G9 antibody in which the complementary determining regions (CDR's) from the variable region of the mouse antibody TF8-5G9 are transplanted into the variable region of a human antibody and joined to the constant region of a human antibody, as described in W0 96/40921, incorporated herein by reference in its entirety. Additional humanized anti-TF antibodies with similar characteristics are described in Presta *et al.,* 2001, *Thromb Haemost.* 85:379-389, EP1069185 and US 6,555,319. In an exemplary embodiment of the present invention, the anti-TF antibody is a monoclonal antibody referred to as compound 3.

Another class of TF antagonists binds to TF and inhibits its activity. Included in this class of molecules are fragments of TF protein, TF muteins, and small organic molecules, such as peptidomimetics. TF antagonists also include, for example, antisense TF RNA, siRNA TF, and factor VIIa inhibitors. TF antagonists can be any of these classes of molecules as long as they are substances that decrease TF pro-coagulation activity. In certain embodiments of the invention, TF antagonists prevent the formation of the TF:factorVIIa:TF ternary complex.

Certain embodiments of the invention relate to methods for enhancing the survival of a cell, tissue, or organ graft that has been implanted into a patient that involve administering an immunosuppressive agent to the patient prior to, during, or following implantation of the graft. The most common agents that are given to patients before and after transplantation procedures act to suppress the body's ability to mount an immune response against antigens on the surface of the transplanted cells or tissues that might be recognized as foreign. Glucocorticosteroids such as prednisolone or cortisone are often a component of the pre- and post-transplantation regimen for immunosuppression. While glucocorticoids are capable of modifying the body's immune responses to diverse stimuli, they also cause profound and varied metabolic effects. In particular, they act as insulin antagonists, raising blood sugar.

The fungally derived product, cyclosporine, is also a widely used agent in pre- and post-transplant or graft management. Cyclosporine, which is sold under the names SANDIMMUNE (Novartis), NEORAL (Novartis) and GENGRAF (Abbott), exerts multiple effects on the immune system and is a potent immunosuppressive agent that prolongs the survival of allogeneic transplants in animals. Cyclosporine has been demonstrated to suppress humoral immunity to a certain extent, and to suppress cell-mediated immune reactions such as allograft rejection, delayed hypersensitivity, experimental allergic encephalomyelitis, Freund's adjuvant arthritis, and graft vs. host disease in many animal species for a variety of organs. Cyclosporine arrests the cell cycle of immunocompetent lymphocytes specifically and reversibly, and preferentially inhibits T-lymphocytes. Helper T cells are cyclosporine's main target, although T-suppressor cells may also be suppressed by cyclosporine. Cyclosporine also inhibits the production and release of lymphokines, including interleukin-2.

Another approach to preventing acute graft rejection involves the administration of an antibody that recognizes CD3 on the surface of T cells. One such product made by Ortho Biotech (Raritan, NJ) is sold under the trade name ORTHOCLONE OKT3 (muromomab-CD3).

A non-diabetogenic regimen excluding glucocorticoids has proven effective for islet cell transplantation. The non-diabetogenic regimen, referred to herein as the "Edmonton protocol" (Shapiro, A.M. et al. N. Engl. J. Med. 2000; 343: 230-238, 2000) includes sirolimus (RAPAMUNE, Wyeth-Ayest), tacrolimus (FK506, PROGRAF, Fujisawa), and an anti-IL2R Mab daclizumab (ZENAPAX, Roche), which work to suppress T-lymphocyte proliferation and activity by a variety of mechanisms. Tacrolimus inhibits T-lymphocyte activation. Sirolimus inhibits T lymphocyte activation and proliferation that occurs in response to antigenic and cytokine IL-2, IL-4, and IL-15 stimulation by a mechanism that is distinct from that of other immunosuppressants. In addition, sirolimus inhibits antibody production. Sirolimus binds to the immunophilin, FK Binding Protein-12 (FKBP-12), to generate an immunosuppressive complex that binds to and inhibits the activation of a key regulatory kinase. Inhibition of the kinase in turn suppresses cytokine-driven T-cell proliferation, inhibiting the progression from the G ₁ to the S phase of the cell cycle. Daclizumab blocks the T-cell IL-2 receptor of active T-cells, preventing T-cell responses to antigenic challenge.

The Edmonton protocol for islet transplantation has proven beneficial because it avoids the use of steroids as immunosuppressive agents in favor of agents that are more specifically directed against T cells, including sirolimus, tacrolimus, and daclizumab.

The p38 MAPK and/or JNK inhibitors, TF antagonists, and immunosuppressive agents can be applied to donor cells, tissues and organs, or otherwise administered to patients by any number of methods that result in the enhanced survival of graft material in the patients.

The duration of treatment of the donor cells or tissue will vary depending in part on the basal expression level of TF mRNA and protein. Cells or tissues with little or no basal TF expression require shorter time intervals of treatment *in vitro* prior to transplantation. In contrast, cells with a moderate to high basal expression level of TF require a longer treatment time in order to down-modulate the existing mRNA and protein load or enzymatic activity while preventing new mRNA and protein synthesis. The optimal time interval for treatment will vary with the cells or tissues of interest as well as handling manipulations, culture conditions, or exposure to any other environmental stress stimuli that may activate TF gene expression.

In one embodiment of the invention, stem cells are maintained *in vitro* for a period of time up to 30 days in culture medium supplemented with an effective amount of at least one p38 MAPK and/or JNK inhibitor and an optional TF antagonist. In specific embodiments, the agents are added at a concentration up to about 50µM. Preferably, the concentration is between 100pM and 50µM. In a further embodiment of the invention, the TF antagonist is an anti-TF antibody, preferably compound 3, at a concentration of about 0.1-100 µg/ml, preferably about 10-50 µg/ml for a period of about 0.5 - 2 hours prior to preparation of the transplantation formulation. In another embodiment of the invention, pancreatic islets are maintained *in vitro* for a period of time up to 10 days in a culture medium as described for stem cells.

In another embodiment, donor tissue is transported in medium containing an effective amount at least one p38 MAPK and/or JNK inhibitor and an optional TF antagonist. In specific embodiments, the agents are added at a concentration up to about 50µM. Preferably, the concentration is between 100pM and 50µM. The donor tissue is then treated according to the methods disclosed above or directly transplanted into the patient.

Treated donor tissue is prepared for transplantation by any appropriate method known to those skilled in the art. These methods include, for example, removing the treated cells from the culture substrate by methods such as, for example, mechanical dispersion or enzymatic treatment. Once in suspension, cells are washed to remove traces of the pharmaceutical agents employed in the present invention and placed into a suitable carrier for introduction into the recipient. Such carriers can include, for example, physiological saline or culture medium. In another embodiment, the cells can be seeded onto a biodegradable support, such as, for example, the supports disclosed in US patent application US20040062753 A1 and U.S. Pat. No. 4,557,264.

Another aspect of the invention features donor cells, tissues and organs that have been contacted with a p38 MAPK and/or JNK inhibitor, and optionally a TF antagonist, as described herein. These treated biological materials include, but are not limited to, any of the cells, tissues and organs referenced throughout the present specification.

The p38 MAPK and/or JNK inhibitors and TF antagonists need not be present locally to impart an effect; therefore, they can be administered wherever access to body compartments or fluids containing TF is achieved. The source material, the source donor, or the prepared material to be transplanted can be treated by direct application of a formulation containing the p38 MAPK and/or JNK inhibitors, and optionally TF antagonists, as described above. Alternatively, the patient or recipient of the transplant can be administered the p38 MAPK and/or JNK inhibitors, and optionally TF antagonists, prior to, during, or after the transplant is made. The latter methods include intravenous administration of a liquid composition, transdermal administration of a liquid or solid formulation, oral, topical, interstitial or inter-operative administration.

In certain embodiments, the p38 MAPK and/or JNK inhibitors, and optionally TF antagonists, are administered systemically by pre-treatment of the patient undergoing transplantation, and, optionally, combined with treatment at various intervals post-transplantation. In such methods, the p38 MAPK and/or JNK inhibitors, and optionally TF antagonists, are preferably administered within one hour, preferably 10 to 20 minutes, prior to transplantation, optionally combined with continuous treatment for up to 14 days or longer post transplantation to prevent graft rejection. Administration may also be oral or by local injection into a vein or artery supplying blood to the intended site for deposition of the transplanted cells, tissues or organ, such as, for example, to the portal vein in the case of pancreatic islet cell transplantation. The MAPK and/or JNK inhibitors, and optionally TF antagonists, are generally effectively administered intravenously, however. The general dosage range is from about 0.05 mg/kg to about 12.0 mg/kg of the p38 MAPK inhibitors, and optionally TF antagonists, which may be administered as a bolus or as a slow or continuous infusion controlled by a microprocessor and programmable pump device.

In the case of anti-TF antibody, donor material can be incubated with the antibody at a concentration of about 0.1-100 µg/ml, preferably about 10-50 µg/ml, for a period of about 0.5 to 2 hours prior to transplantation. Alternatively, DNA encoding a fragment of an anti-TF antibody can be isolated from hybridoma cells and administered to the patient undergoing the transplant. The DNA can be administered in naked form or inserted into a recombinant vector such as, for example, a vaccinia virus, in a manner that results in expression of the DNA in the cells of the patient and delivery of the antibody.

The MAPK and/or JNK inhibitors, and optionally TF antagonists, used in the methods of the present invention can be formulated by any of the established methods of formulating pharmaceutical compositions, such as, for example, those described in Remington's Pharmaceutical Sciences, 1985, incorporated herein by reference in its entirety. For ease of administration, the MAPK and/or JNK inhibitors, and optionally TF antagonists, will typically be combined with a pharmaceutically acceptable carrier. Such carriers include, for example, water, physiological saline, or oils.

Formulations suitable for parenteral administration include aqueous and non-aqueous sterile injection solutions that may contain anti-oxidants, buffers, bacteriostats and solutes that render the formulation isotonic with the blood of the intended recipient; and aqueous and non-aqueous sterile suspensions that may include suspending agents and thickening agents. Except insofar as any conventional medium is incompatible with the active ingredient and its intended use, its use in any composition is contemplated. Formulations for *in vitro* or *ex vivo* treatment of organs, tissues, or cells would include the same, but preferably an organic solvent diluted appropriately for optimal cell culture without adverse effect on cell viability. This would include, for example, alcohols and DMSO.

The formulations can be presented in unit-dose or multi-dose containers, for example, sealed ampoules and vials, and can be stored in a freeze-dried (lyophilized) condition requiring only the addition of the sterile liquid carrier, e.g., water for injections, immediately prior to use.

The following examples are illustrative of certain embodiments of the invention and should not be considered to limit the scope of the invention.

### Example 1: Anti-Coagulant Effects of an Anti-TF Antibody and a p38 MAPK Inhibitor on Peripheral Blood Mononuclear Cells (PBMC's)

Peripheral venous blood was obtained by consent from normal, healthy volunteers and mononuclear cells were isolated using Histopaque 1077 (Sigma) density gradient centrifugation. Following three washes with RPMI (Invitrogen) supplemented with 1% fetal calf serum (FCS), cells were counted and viability was assessed using a Guava PCA-96 instrument (Guava Technologies, Hayward, CA). Cells were resuspended to 1.67 x 10⁶ cells/mL in RPMI media containing 10% FCS, and 4 mL/well were added to a 6-well dish (Corning-Costar). Lipopolysaccharide (LPS) was diluted into cell cultures to a final concentration of 10 ng/ml. p38 inhibitor compound 1 was added to cell cultures at a final concentration of 10 µM. An equivalent dilution of DMSO was added to untreated cultures. The following four assay conditions were: (+) LPS (+) p38 inhibitor, (+) LPS (-) p38 inhibitor, (-) LPS (+) p38 inhibitor, and (-) LPS (-) p38 inhibitor. Cultures were incubated at 37°C in 5 % CO₂ for 18 to 24 hours.

*Plasma Clotting Assay.* Aliquots containing 100,00 PBMCs/well in 100 µL Hank's Balanced Salt Solution (HBSS) (Invitrogen) were added to a 96 well flat-bottom plate (Corning-Costar). A 4.5 mg/ml stock solution of anti-TF antibody compound 3 was diluted to 100 µg/ml and 25 µl added to each treated well. Sequential additions of CaCl₂ (25 µl to yield 15 mM final concentration) and pooled normal human plasma (100 µl; George King Biomedical, Overland Park, KS) were added to each well. Clotting was monitored at 15 second intervals for 2 hours at room temperature using a Spectra MAX 190 (Molecular Devices, Sunnyvale, CA) at a wavelength of 405 nm. Quadruplicate samples were included for each assay condition to reduce well-to-well variation between samples. The results are depicted in Figure 1A. Clotting times (time to maximal OD change) were dependent on the amount of TF on the surface of the cells; faster clotting times reflect more cell surface TF, and prolonged clotting times reflect less TF. Since there were an equal number of cells in each well, cell number was not a factor.

PBMCs (100,00 in 100 µl of HBSS) were pre-incubated with 25 µl of anti-TF antibody compound 3 (10 µg/ml) for 30 minutes at room temperature in the same 96-well plate as the untreated cells above. Control diluent (HBSS) was added to the untreated cells. CaCl₂ and pooled human plasma were added and the clotting reaction was monitored as described above. The results are presented in Figure 1B.

Figure 1C shows the time to half the maximum OD in cells treated or untreated with LPS, p38 mitogen-activated kinase inhibitor compound 1, and anti-TF antibody compound 3.

The results of these experiments demonstrate that a small molecule inhibitor of p38 MAPK can modulate coagulation times. Peripheral blood mononuclear cells have low basal levels of surface TF but can rapidly upregulate expression when exposed to an inflammatory stimulus such as LPS, and subsequently demonstrate escalated coagulation times. The p38 inhibitors used in this example interfere with this rapid LPS-induced coagulation, causing a significant delay in coagulation within a short pre-treatment phase. In combination with anti-TF antibody, there is a further delay or prolongation in coagulation.

### Example 2: Anti-Coagulant Effects of Anti-TF Antibody Compound 3 on Cells Pre-Treated with p38 inhibitors Compound 1 and Compound 2 for A Sustained Period of Time

In the previous example, cells were stimulated overnight to produce TF by LPS induction. Inhibition of this process was achieved by addition of p38 inhibitor compound 1, evaluated by plasma clotting assay. In this example, two types of cells were pre-cultured with the p38 inhibitors compound 1 and compound 2 for 10 days to evaluate the effect of p38 MAPK inhibitors on coagulation. Cells were separately treated using a final concentration of 10 µM of each p38 inhibitor. Parallel treatments of DMSO vehicle were included as a control. Media, containing the above compounds were replenished during culture at intervals of every 2 days.

Human mesenchymal stem cells (MSC) were isolated from whole bone marrow purchased from Cambrex BioScience Walkersville, Inc. Cells were obtained from 10-ml aspirates taken from the iliac crest of normal human donors (age about 20 years). Mononuclear cells were isolated by gradient centrifugation. All cells were plated at a constant density of 10,000 cells/cm² on 15-cm culture dishes in DMEM-low glucose medium containing 5% fetal bovine serum and 100 U/ml penicillin and 1000 U/ml streptomycin. After seven days, nonadherent cells were discarded. Colony-forming units were isolated by cloning rings and plated at low densities for further expansion. These cells have a low to moderate basal level of TF. J82 is a human bladder carcinoma line purchased from ATCC (Rockville, MD) and used in this example as a positive control. Previous results indicate that J82 cells have approximately 500,000 anti-TF antibody compound 3 binding sites per cell.

The plasma clotting assay was performed as described in Example 1. Cells were lightly trypsinized from culture on the day of assay, washed, counted, and viability was determined before adjusting to 10⁶ cells/ml in HBSS. The assay was performed in a flat-bottom 96-well plate using 10⁵ cells/well. Anti-TF antibody compound 3 (100 µg/ml) or control diluent was added in a volume of 25 µl/well and allowed to pre-bind for 30 minutes at room temperature. CaCl₂ was added to each well (25 µL of 150mM stock solution) along with 100 µL of pooled normal human plasma to initiate the reaction. Coagulation was monitored at room temperature in a kinetic plate reader using 15 second interval readings at 405 nm over 60 to 120 minutes. A final reading of T_{1/2} maximal clotting time was obtained for each sample condition. Controls for the assay included Simplastin (liquid thromboplastin) and HBSS buffer without cells. Cell viabilities at the time of assay were greater than 95 % for human MSC and greater than 97 % for J82. Replicate sets of eight samples for each condition were included to reduce well-to-well variation between samples with CVs of less than 13 %.

The results are expressed in terms of the time to reach half maximal OD. The time to half the maximum for human MSC was calculated as: 61.0 seconds for simplastin; 1042.0 seconds for untreated cells; 2532.0 seconds for cells treated with anti-TF antibody compound 3 alone; 2203.1 seconds for cells treated with p38 inhibitor compound 2; 1882.2 seconds for cells treated with p38 inhibitor compound 1; 3041.5 seconds for cells treated with p38 inhibitor compound 1 in combination with anti-TF antibody compound 3; 4273.3 seconds for cells treated with p38 inhibitor compound 2 in combination with anti-TF antibody compound 3; and 3469.5 seconds for wells without cells (Figure 2A). Figure 2C depicts the time to half the maximum OD for human MSC pre-cultured with compound 1 or compound 2.

For J82 cells, times to reach ½ maximal OD were: 62.3 s for simplastin; 155.01 s for untreated cells; 159.9 s for cells treated with p38 inhibitor compound 2; 160.9 s for cells treated with p38 inhibitor compound 1; 255.9 s for cells treated with p38 inhibitor compound 1 in combination with anti-TF antibody compound 3; 305.3 s for cells treated with anti-TF antibody compound 3 alone; 330.2 s for cells treated with p38 inhibitor compound 2 in combination with anti-TF antibody compound 3; and 4260.0 s for wells without cells (Figure 2B). Figure 2D depicts the time to half the maximum OD for J82 cells pre-cultured with compound 1 or compound 2.

The experiments demonstrate that a small molecule inhibitor of p38 MAPK can modulate coagulation times after long-term cell exposure. No overt signs of toxicity from these compounds were noted for cells in culture, as determined by viability or recovery of cell number. The p38 MAPK inhibitors caused a significant delay in coagulation compared to untreated control cells, which could be further enhanced with the addition of anti-TF antibody compound 3.

### Example 3: Inhibition of Coagulation in Murine Islets by p38 MAPK Inhibitors

Pancreata were harvested from Balb/c mice via cannulation of the common bile duct and perfusion with a liberase solution (Roche). Pancreata were digested at 37°C for 15 minutes followed by discontinuous ficoll gradient centrifugation to remove acinar and ductal tissue.

Islets of similar size were collected and plated in 3 ml of islet growth medium in a 6-well culture dish (300 islets per well) with or without p38 MAPK inhibitors (10 µM compound 1 or compound 2). Vehicle control was an equivalent dilution of DMSO. Islets were cultured at 37°C in 5% CO₂ for 72 hours. The three treatment conditions were: vehicle control, (+) p38 inhibitor compound 1, and (+)p38 inhibitor compound 2.

*Plasma Clotting Assay.* 50 islets/well in 125 µL Hank's Balanced Salt Solution (HBSS) (Gibco 14025-076) were added to a 96 well flat-bottom plate (Corning-Costar) followed by sequential additions of 25 µl CaCl₂ (15 mM final concentration) and 100 µl of pooled normal human plasma (George King Biomedical, Overland Park, KS). Clotting was monitored at 15 second intervals for 2 hours at room temperature in a Spectra MAX 190 (Molecular Devices, Sunnyvale, CA) at 405nm. Times to half maximum OD were calculated as 388.6 seconds for the vehicle control and 596.9 seconds for cells treated with p38 inhibitor compound 2. (Figures 3A and 3C). In a second experiment, the time to half maximum was calculated as 488.9 seconds for the vehicle control and 974.7 seconds for treated islets. (Figures 3B and 3D). Islets were approximately 88 % viable by dithizone staining at the time of the assay. Each condition was run in duplicate to reduce well-to-well variation between samples (Figures 3C and 3D).

These experiments demonstrate the ability of the p38 MAP kinase inhibitors compound 1 and compound 2 to prolong coagulation time of harvested islets. This is a beneficial therapy that will greatly reduce the number of cells lost to thrombosis during transplant and ultimately increase islet graft efficiency.

### Example 4: Effects of p38 MAPK Inhibitors and Anti-TF Antibody Compound 3 Treatment on TF mRNA in LPS stimulated PBMCs

Peripheral blood mononuclear cells (PBMCs) are isolated and cultured as described in Example 1 above using parallel wells with identical conditions: (+) LPS (+) p38 inhibitor, (+) LPS (-) p38 inhibitor, (-) LPS (+) p38 inhibitor, and (-) LPS (-) p38 inhibitor. Cultures are incubated at 37°C in 5% CO₂ for 18 to 24 hours.

*Isolation of total RNA.* Cells are resuspended to 1.67 x 10⁶ cells/mL in RPMI media containing 10 % FCS. 1 mL of cell suspension is removed to a 1.5 mL microcentrifuge tube and centrifuged at 5,000 x g for 1 minute to pellet the cells. Cell pellets are resuspended in 1 mL Trizol reagent (Invitrogen). Total RNA is isolated according to the manufacturer's protocol and quantitated by 1:100 dilutions in dH₂0 read on an Ultrospec 2100 pro spectrophotometer (Amersham Biosciences, Piscataway, NJ).

*cDNA synthesis and RT-PCR*. Total RNA is converted to cDNA using the iScript cDNA synthesis kit (Bio Rad Laboratories). Aliquots of 100-500 ng total RNA, 4 µl Superscript buffer, and 1 µl iScript reverse transcriptase are combined in a final reaction volume of 20 µl. The reaction is placed in a BioRad iCycler (BioRad Laboratories, Hercules, CA with reverse transcription conditions as follows: one cycle of 25°C for 5 minutes, 42°C for 30 minutes, 85°C for 5 minutes, and 4°C hold. Ten to 20 ng of cDNA is used in a PCR reaction to detect TF and GAPDH transcripts. The amount of cDNA is added to a reaction containing 2.5 µL 10X iTaq Buffer, 0.75 µL 50mM MgCl₂, 0.5 µL 10mM dNTP, 0.125 µL iTaq DNA Polymerase, 1 µL of forward primer, and 1 µL of reverse primer. The reaction is brought to a final volume of 25 µL with dH₂0. Primers and their sequences used are as follows:
huTF forward: 5'-CTCGGAACAGCCAACAATTCAG-3'
huTF reverse: 5'-TGTTCGGGAGGGAATCACTGCTTGAACACT-3'
huGAPDH forward: 5'-ACCCACTCCTCCACCTTTG-3'
huGAPDH reverse: 5'-CTCTTGTGCTCTTGCTGGG-3'.
Reaction conditions are as follows: huTF: 95°C for 3' initial denaturation followed by 35 cycles of 94°C for 30 seconds, 50°C for 30 seconds, and 72°C for 30 seconds. A final elongation step of 72°C for 7 minutes is followed by a 4°C hold. The reactions are electrophoresed on a 1.8 % agarose gel at 120 v for approximately 1 hour. Gels are analyzed using a Kodak Gel Logic 200 imaging system.

### Example 5: Effect of Pre-culture with p38 MAPK Inhibitors on Human Mesenchymal Stem Cells TF mRNA

Human MSC and J82 cells are isolated and cultured as described in Example 2 using parallel wells with identical conditions: (+) LPS (+) p38 inhibitor, (+) LPS (-) p38 inhibitor, (-) LPS (+) p38 inhibitor, and (-) LPS (-) p38 inhibitor. Cultures are incubated at 37°C in 5 % CO₂ for 10 days. RNA and cDNA synthesis followed by RT-PCR is carried out for MSC and J82 cells as described in Example 4.

### Example 6: Effects of p38 inhibitor and Anti-TF Antibody Compound 3 Treatment on TF Protein in LPS Stimulated PBMCs

Peripheral blood mononuclear cells (PBMCs) are isolated and cultured as described in Example 1 using parallel wells with identical conditions: (+) LPS (+) p38 inhibitor, (+) LPS (-) p38 inhibitor, (-) LPS (+) p38 inhibitor, and (-) LPS (-) p38 inhibitor. Cultures are incubated at 37°C in 5 % CO₂ for 18 to 24 hours.

*Isolation of Protein and Western Blotting.* Cells are harvested into 15 ml conical tubes and centrifuged at 5,000 x g for 3 minutes. Cells are lysed in 45 µL of western lysis buffer containing 1 % NP-40, 130 mM NaCl and 20 mM Tris pH 7.6. Complete Mini Protease Inhibitor Cocktail (Roche) is added to prevent protein degradation. Appropriate volumes of sample buffer and reducing agent are added to aliquots of each sample and heated to 70° C for 10 minutes. Equivalent volumes of cells are loaded per lane and run on 10 % NuPAGE 10 mm bistris gels (Invitrogen NP0315BOX), at 200 V for 50 minutes using 1X NuPAGE MOPS SDS running buffer (Invitrogen). Gels are transferred to nitrocellulose in a Bio-Rad mini-protean II chamber (Bio-Rad Laboratories, Hercules CA) at 30V for 1 hour using 1X NuPAGE transfer buffer with 10 % methanol. Membranes are washed once with 50 mL 1x PBS (Gibco) and blocked in solution containing 5 % milk/PBS-0.0.5 % Tween-20 (PBS-T) for 1 hour. Following one wash with 1X PBS, a 1:200 dilution of monoclonal antibody against human TF (American Diagnostica 4509) is added in 1X PBS and incubated overnight at 4°C. Membranes are washed 2 x for 15 minutes in PBS-T. Goat anti-mouse secondary antibody at a dilution of 1:100,000 in 5% milk -PBS-T is added for 30 minutes. Membranes are washed for three minutes six times PBS-T, and protein signal is detected by ECL Advance Western Detection Kit (Amersham Biosciences RPN2135).

### Example 7: Effects of Anti-TF Antibody Compound 3 on Protein Levels from Cells Pre-Treated with p38 Inhibitors Compound 1 and Compound 2 for A Sustained Period of Time

Human MSC and J82 cells are isolated and cultured as described in Example 2 using parallel wells with identical conditions: (+) LPS (+) p38 inhibitor, (+) LPS (-) p38 inhibitor, (-) LPS (+) p38 inhibitor, and (-) LPS (-) p38 inhibitor. Cultures are incubated at 37°C in 5 % CO₂ for 10 days. Cells are harvested and processed for protein detection as described in Example 6.

### Example 8: Prevention of Coagulation in Trypsinized Pancreatic Duct Cells by Anti-TF Antibody Compound 3

Ductal tissue is a common contaminant in most human islet preparations. Ductal cells express greater amounts of TF protein than islet beta cells. Furthermore, ductal cells are subject to the same environmental stresses relating to enzymatic dissociation of cellular membranes during isolation. Panc 1 cells are a human pancreatic ductal tumor line that can mimic the coagulation potential of pancreatic ductal cell tissue. Panc1 cells grow as an adherent monolayer that must be trypsinized for harvest and passage. It has been suggested that enzymatic digestion, along with physical manipulation of normal ductal tissue or Panc1 cells, may further enhance TF activation and promote more rapid coagulation.

Panc1 cells (ATCC) were cultured in DMEM (Invitrogen) plus 10 % FCS. Prior to assay, cells were trypsinized to detach, washed, plated at a density of 100,000 cells per well of a 96-well flat bottom plate (Costar 3590) and allowed to bind to the substratum overnight. In a separate experiment, Panc1 cells were trypsinized and plated 100,000 cells per well at the time of initiating the plasma clotting assay.

*Plasma Clotting Assay.* 100,00 Panc 1 cells/well in 100 µL Hank's Balanced Salt Solution (HBSS) (Gibco) were added to a 96 well flat-bottom plate (Corning-Costar). A 4.5 mg/ml stock solution of anti-TF antibody compound 3 was diluted to a final concentration of 20 µg/mL in each assay well. In wells not receiving antibody, 25 µL HBSS was added to maintain an equal volume between samples. Following a 30 minute pre-incubation to allow antibody binding, CaCl₂ was added to a final concentration of 15 mM followed by 100 µl of pooled normal human plasma (George King Biomedical, Overland Park, KS). Clotting was monitored at 15 second intervals for 2 hours at room temperature in a Spectra MAX 190 (Molecular Devices, Sunnyvale, CA) at 405 nm. At this wavelength, the turbidity of the sample represents a measure of clotting. Clotting times (time to maximal OD change) were dependent on the amount of TF on the surface of cells, where faster clotting times correlate with more surface TF, and prolonged clotting times correlate with less TF relative to a medium control. Each well contained a constant number of cells; therefore, cell number was not a contributing variable. Times to half maximum OD were calculated from the curves as follows: 1030.3 seconds for untreated cells; 1289.9 seconds for cells treated with DMSO; 1738.8 seconds for cells treated with DMSO and anti-TF antibody compound 3; and 2041.2 seconds for medium control without cells (Fig 4).

Panc1 cells (100,00 in 100 µl of HBSS) were trypsinized from culture by incubating with 0.05 % Trypsin EDTA (Invitrogen) 5 minutes at 37°C, followed by neutralization with 10 % serum and thorough washing to remove residual trypsin. Thereafter, cells were treated as previously described in Examples 1 and 2 above. The time to half maximum OD was calculated as 675.1 seconds for untreated cells, 664.9 seconds for cells treated with DMSO, 1104.7 seconds for cells treated with DMSO in combination with anti-TF antibody compound 3, and 2041.2 seconds for medium control without cells (Fig 4). Each treatment was run in quadruplicate to reduce well-to-well variation.

These experiments demonstrate that enzymatic treatment with trypsin can enhance the coagulation potential of treated cells.

The invention is not limited to the embodiments described and exemplified above, but is capable of variation and modification within the scope of the appended claims.

## Claims

1. A method for enhancing survival of a cell, tissue, or organ transplanted into a recipient comprising contacting the cell, tissue, or organ with at least one agent that decreases the rate of coagulation on the transplanted cell, tissue or organ.

2. The method of claim 1, wherein the agent reduces the amount or activity of tissue factor on or within the cell, tissue or organ.

3. The method of claim 2, wherein the agent is a p38 MAPK inhibitor, a JNK inhibitor, a p38 MAPK/JNK dual inhibitor, or any combination thereof.

4. The method of claim 3, wherein the agent is a benzyl piperidine, a piperidine, a piperazine, a pyrrolobenzimidazole, an indol or a pyridinyl-imidazol.

5. The method of claim 4, wherein the agent is 4-[4-(4-fluorophenyl)-1-(3-phenylpropyl)-5-(4-pyridinyl)-1H-imidazole-2-yl]-3-butyn-1-ol.

6. The method of claim 4, wherein the agent is an indol.

7. The method of claim 2, further comprising contacting the cell, tissue or organ with a tissue factor antagonist.

8. The method of claim 7, wherein the tissue factor antagonist is an anti-tissue factor antibody.

9. The method of claim 1, wherein the cell, tissue or organ is contacted with the agent prior to or during transplantation into the recipient.

10. The method of claim 9, wherein the cell, tissue or organ is contacted with the agent *in-vitro,* prior to transplantation into the recipient.

11. The method of claim 1, wherein the cell, tissue or organ is a pancreatic-derived cell population.

12. The method of claim 1, wherein the cell, tissue or organ is a multipotent or pluripotent progenitor cell population.

13. The method of claim 1, further comprising administering the agent to the recipient before, during, or after the transplantation, or any combination of before, during or after the transplantation.

14. The method of claim 13, wherein the agent reduces the amount or activity of tissue factor in the recipient.

15. The method of claim 14, wherein the agent is a p38 MAPK inhibitor, a JNK inhibitor, a p38 MAPK/JNK dual inhibitor, or any combination thereof.

16. The method of claim 15, wherein the agent is a benzyl piperidine, a piperidine, a piperazine, a pyrrolobenzimidazole, an indol or a pyridinyl-imidazol.

17. The method of claim 16, wherein the agent is 4-[4-(4-fluorophenyl)-1-(3-phenylpropyl)-5-(4-pyridinyl)-1H-imidazole-2-yl]-3-butyn-1-ol.

18. The method of claim 16, wherein the agent is an indol.

19. The method of claim 13, further comprising administering a tissue factor antagonist to the recipient.

20. The method of claim 19, wherein the tissue factor antagonist is an anti-tissue factor antibody.

21. The method of claim 13, further comprising administering an immunosuppressive agent to the recipient.

22. The method of claim 21, wherein the immunsuppressive agent is cyclosporine, tacrolimus, sirilimus, daclizumab or any combination thereof.

23. A cell, tissue, or organ exhibiting enhanced survival upon transplatation into a recipient, wherein the cell tissue or organ has been contacted with, and thereby comprises within or upon it, at least one agent that decreases the rate of coagulation on the cell, tissue or organ.

24. The cell, tissue or organ of claim 23, wherein the agent reduces the amount or activity of tissue factor on or within the cell, tissue or organ.

25. The cell, tissue or organ of claim 24, wherein the agent is a p38 MAPK inhibitor, a JNK inhibitor, a p3 8 MAPK/JNK dual inhibitor, or any combination thereof.

26. The cell, tissue or organ of claim 25, wherein the agent is a benzyl piperidine, a piperidine, a piperazine, a pyrrolobenzimidazole, an indol or a pyridinyl-imidazol.

27. The cell, tissue or organ of claim 26, wherein the agent is 4-[4-(4-fluorophenyl)-1-(3-phenylpropyl)-5-(4-pyridinyl)-1H-imidazole-2-yl]-3-butyn-1-ol.

28. The cell, tissue or organ of claim 26, wherein the agent is an indol.

29. The cell, tissue or organ of claim 24, further comprising a tissue factor antagonist.

30. The cell, tissue or organ of claim 29, wherein the tissue factor antagonist is an anti-tissue factor antibody.

31. The cell, tissue or organ of claim 23, which is a pancreatic-derived cell population.

32. The cell, tissue or organ of claim 23, which is a multipotent or pluripotent progenitor cell population.
